# EUROPEAN PATENT APPLICATION

(11) **EP 4 643 887 A1**
(43) Date of publication of application: **05.11.2025**
(21) Application number: 23910756.8
(22) Date of filing: 27.12.2023
(51) Int. Cl.: A61K 47/68, C07K 16/28, A61K 31/58, A61P 37/00

(54) **ANTI-BDCA2 ANTIBODY-DRUG CONJUGATE AND USE THEREOF**

(30) Priority: 28.12.2022 CN 202211697892
(71) Applicant: Duality Biologics (Shanghai) Co., Ltd., Shanghai 201204 (CN)
(72) Inventor: LI, Xi, Shanghai 201204 (CN); ZHANG, Yu, Shanghai 201204 (CN); LI, Bing, Shanghai 201204 (CN); HUA, Haiqing, Shanghai 201204 (CN); ZHU, Zhongyuan, Shanghai 201204 (CN); LI, Jian, Shanghai 201204 China (CN)
(74) Representative: De Clercq & Partners
(86) International application number: PCT/CN2023/142457
(87) International publication number: WO 2024/140838

(57) **Abstract**

An anti-BDCA2 antibody-drug conjugate and the use thereof. The anti-BDCA2 antibody-drug conjugate comprises: an antibody targeting BDCA2 or an antigen-binding fragment thereof, a linker fragment (L), and a glucocorticoid molecule. Also provided are a preparation method for and the use of the antibody-drug conjugate.

## Description

### TECHNICAL FIELD

The present application relates to an anti-BDCA2 antibody-drug conjugate and use thereof. The anti-BDCA2 antibody-drug conjugate comprises an antibody targeting BDCA2 or an antigen-binding fragment thereof, a linker fragment (L), and a glucocorticoid molecule. The present application also provides a preparation method for the antibody-drug conjugate and use thereof.

### BACKGROUND

As medical research advances, various diseases have been found to be associated with dysfunction of the immune system, and the research on the immune system enhances our further understanding of disease pathogenesis and facilitates the development of better therapeutic regimens.

The immune system is a highly complex system composed of many cell types, including but not limited to T cells, B cells, natural killer cells, antigen-presenting cells, dendritic cells, monocytes, and macrophages. These cells possess complex and subtle systems to control their interactions and responses. Cells utilize activation and inhibition mechanisms and feedback loops to maintain control of the response and to possibly prevent the negative consequences of an uncontrolled immune response (e.g., an autoimmune disease). Among the multiple signaling pathways related to immunity, there are a number of signaling molecules and their interactions involved.

Blood dendritic cell antigen 2 (BDCA2) is a C-type lectin expressed on human plasmacytoid dendritic cells (pDCs) (Dzioneketal., J. Immunol., 165: 6037-6046 (2000)). BDCA2 consists of a single extracellular carbohydrate recognition domain (CRD) at its C-terminus (which belongs to the group of type II C-type lectins), a transmembrane region from the asparagine residue at position 45 to the isoleucine residue at position 213, and a short cytoplasmic tail (which does not contain a signal motif) at its N-terminus. BDCA2 transmits intracellular signals through the related transmembrane adaptor FcεRIγ and induces a B-cell receptor (BCR)-like signaling cascade.

An antibody-drug conjugate (ADC) consists of three portions, an antibody or an antigen-binding fragment thereof (targeting), a linker, and a small molecule drug. The antibody or the antigen-binding fragment thereof is conjugated with a bioactive small molecule drug through a cleavable or non-cleavable linker. This effectively utilizes the specificity of the antibody or the antigen-binding fragment thereof for targeting cells of interest (target cells) or binding to a highly expressed antigen, as well as the high efficiency of the small molecule drug, thereby reducing or avoiding toxic side effects on non-targeted cells.

Glucocorticoids (GCs) are small molecule steroid compounds that bind to glucocorticoid receptors (GRs) and are used in anti-inflammatory and immunosuppressive therapies. However, glucocorticoid therapy is affected by toxicity in most organ systems due to the ubiquitous expression of glucocorticoid receptors in many cell types. Therefore, there is a need to develop a novel glucocorticoid and a novel therapy that minimizes the side effects caused by glucocorticoid administration, especially those caused by glucocorticoid receptor activation in non-targeted cells.

No antibody-drug conjugate targeting BDCA2 has been developed to date, and thus the development of related clinical drugs has great space and clinical demands.

### SUMMARY

The present application provides an antibody-drug conjugate targeting BDCA2, which may have one or more effects selected from the group consisting of: (1) having the ability to affect the activity of immune cells; (2) the ligand thereof having a targeting effect; (3) having plasma stability; (4) having biological safety; (5) having the ability to affect cytokine release from immune cells; (6) having the ability to affect transcription of responsive genes of IFN signaling pathway; (7) having the ability to affect the degree of skin fibrosis; (8) having the ability to affect the number and/or proportion of dendritic cells; (9) having the ability to affect the collagen content of the skin; (10) having the ability to affect the GRE expression level; (11) having the ability to affect cytokine release from peripheral blood mononuclear cells; (12) having the ability to affect skin swelling; (13) having the ability to affect arthritic symptoms; and (14) having the ability to inhibit inflammation.

In one aspect, the present application provides an antibody-drug conjugate, comprising an antibody targeting BDCA2 or an antigen-binding fragment thereof,
a linker fragment (L), and a glucocorticoid molecule, wherein the antibody targeting BDCA2 or the antigen-binding fragment thereof comprises:
   (1) HCDR1 with the amino acid sequence set forth in SEQ ID NO: 1, HCDR2 with the amino acid sequence set forth in SEQ ID NO: 2,
      HCDR3 with the amino acid sequence set forth in SEQ ID NO: 3, LCDR1 with the amino acid sequence set forth in SEQ ID NO: 4, LCDR2 with the amino acid sequence set forth in SEQ ID NO: 5, and LCDR3 with the amino acid sequence set forth in SEQ ID NO: 6; or
   (2) HCDR1 with the amino acid sequence set forth in SEQ ID NO: 7, HCDR2 with the amino acid sequence set forth in SEQ ID NO: 8,
HCDR3 with the amino acid sequence set forth in SEQ ID NO: 9, LCDR1 with the amino acid sequence set forth in SEQ ID NO: 10, LCDR2 with the amino acid sequence set forth in SEQ ID NO: 11, and LCDR3 with the amino acid sequence set forth in SEQ ID NO: 12.

In some embodiments, in the antibody-drug conjugate described herein, the antibody or the antigen-binding fragment thereof comprises:
(1) a heavy chain variable region with the amino acid sequence set forth in SEQ ID NO: 13, and a light chain variable region with the amino acid sequence set forth in SEQ ID NO: 14; or
(2) a heavy chain variable region with the amino acid sequence set forth in SEQ ID NO: 15, and a light chain variable region with the amino acid sequence set forth in SEQ ID NO: 16; or
(3) a heavy chain variable region with the amino acid sequence set forth in SEQ ID NO: 17, and a light chain variable region with the amino acid sequence set forth in SEQ ID NO: 18.

In some embodiments, in the antibody-drug conjugate described herein, the antibody is selected from a human antibody, a humanized antibody, a chimeric antibody, a multispecific antibody, a monoclonal antibody, and a polyclonal antibody.

In some embodiments, in the antibody-drug conjugate described herein, the antibody or the antigen-binding fragment thereof is selected from a humanized antibody or an antigen-binding fragment thereof.

In some embodiments, in the antibody-drug conjugate described herein, the antibody described herein is a monoclonal antibody.

In some embodiments, in the antibody-drug conjugate described herein, the antibody is a multispecific antibody comprising a light chain variable region and a heavy chain variable region of the antibody or the antigen-binding fragment thereof described herein.

In some embodiments, in the antibody-drug conjugate described herein, the antigen-binding fragment is selected from a Fab, a Fab', a F(ab')₂, an Fv, an scFv, a Fab'-SH, an sdAb, a VHH, a bispecific antibody, and a linear antibody.

In some embodiments, in the antibody-drug conjugate described herein, the antibody or the antigen-binding fragment thereof comprises an immunoglobulin constant region, the immunoglobulin constant region being a human IgG constant region, e.g., a human IgG1 constant region.

In some embodiments, in the antibody-drug conjugate described herein, the antibody or the antigen-binding fragment thereof comprises or consists of the following sequences:
(1) a heavy chain with the amino acid sequence set forth in SEQ ID NO: 19 or having at least 95%, 96%, 97%, 98%, or 99% sequence identity thereto, and a light chain with the amino acid sequence set forth in SEQ ID NO: 20 or having at least 95%, 96%, 97%, 98%, or 99% sequence identity thereto; or
(2) a heavy chain with the amino acid sequence set forth in SEQ ID NO: 21 or having at least 95%, 96%, 97%, 98%, or 99% sequence identity thereto, and a light chain with the amino acid sequence set forth in SEQ ID NO: 22 or having at least 95%, 96%, 97%, 98%, or 99% sequence identity thereto; or
(3) a heavy chain with the amino acid sequence set forth in SEQ ID NO: 23 or having at least 95%, 96%, 97%, 98%, or 99% sequence identity thereto, and a light chain with the amino acid sequence set forth in SEQ ID NO: 24 or having at least 95%, 96%, 97%, 98%, or 99% sequence identity thereto.

In some embodiments, in the antibody-drug conjugate described herein, the antibody or the antigen-binding fragment thereof comprises or consists of the following sequences:
(1) a heavy chain with the amino acid sequence set forth in SEQ ID NO: 19, and a light chain with the amino acid sequence set forth in SEQ ID NO: 20; or
(2) a heavy chain with the amino acid sequence set forth in SEQ ID NO: 21, and a light chain with the amino acid sequence set forth in SEQ ID NO: 22; or
(3) a heavy chain with the amino acid sequence set forth in SEQ ID NO: 23, and a light chain with the amino acid sequence set forth in SEQ ID NO: 24.

In some embodiments, the amino acid sequences of the CDRs may have at least 80%, 90%, 91%, 92%, 93%, 94%, 95%, 96%, 97%, 98%, or 99% identity to the sequences described above. In some embodiments, the amino acid sequences of the variable regions may have least 90%, 91%, 92%, 93%, 94%, 95%, 96%, 97%, 98%, or 99% identity to the sequences described above.

As described above, at least one amino acid addition, deletion and/or substitution in the VH or VL region may not be in any CDR sequence, but in framework (FRW) sequences. For example, the isolated antibody or antigen-binding moiety thereof may comprise one or more amino acid substitutions in framework sequences, such as FRW1, FRW2, FRW3, and/or FRW4 of the VH or VL region.

In some embodiments, the 6 CDRs may have a total of 0, 1, 2, 3, 4, or 5 amino acid modifications (complete amino acid substitutions), as well as changes in the framework regions of the heavy chain variable region and the light chain variable region, so long as the framework (excluding the CDRs) maintains at least about 80%, 85%, or 90% identity to the parent antibody. Thus, the same CDR described herein can be combined with different framework sequences from human germline sequences, so long as the framework regions maintain at least 80%, 85%, or 90% identity to the human germline sequences.

In some embodiments, in the antibody-drug conjugate described herein, the antibody is an IgG antibody, e.g., IgG1, IgG2, IgG3, or IgG4 antibody or a modified form thereof, as described below.

In some embodiments, one or more amino acid modifications may be introduced into an Fc region of the antibody provided herein, thus producing an Fc region variant. The Fc region variant may comprise a human Fc region sequence (such as the human IgG1, IgG2, IgG3, or IgG4 Fc region) comprising an amino acid modification (such as substitution) at one or more amino acid positions.

In some embodiments, antibodies modified by cysteine engineering may need to be produced, such as "sulfo-MAb", wherein one or more residues of the antibodies are substituted with cysteine residues.

In some embodiments, the antibodies provided herein can be further modified to contain other non-protein moieties known in the art and readily available. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), glucan or poly(n-vinylpyrrolidone)polyethylene glycol, propylene glycol homopolymer, polypropylene oxide/ethylene oxide copolymer, polyoxyethylated polyol (such as glycerol), polyvinyl alcohol, and mixtures thereof.

In some embodiments, in the antibody-drug conjugate described herein, the antibody is selected from anti-BDCA2 antibodies Hu033-03, Hu033-20, and Hu005-04.

In some embodiments, the antibodies Hu033-03, Hu033-20, and Hu005-04 used in the drug conjugate, composition, use, or method of the present disclosure are derived from the antibodies Hu033-03, Hu033-20, and Hu005-04 described in CN202210599888.X.

The anti-BDCA2 antibody Hu033-03 or the antigen-binding fragment thereof described herein is prepared as described in CN202210599888.X. In some embodiments, the CDR sequences of the antibody used in the drug conjugate, composition, use, or method of the present disclosure comprise HCDR1, HCDR2, and HCDR3 with the amino acid sequences set forth in SEQ ID NO: 1, SEQ ID NO: 2, and SEQ ID NO: 3, respectively, and LCDR1, LCDR2, and LCDR3 with the amino acid sequences set forth in SEQ ID NO: 4, SEQ ID NO: 5, and SEQ ID NO: 6, respectively. In some embodiments, the variable region sequences of the antibody used in the drug conjugate, composition, or use of the present disclosure comprises a heavy chain variable region with the amino acid sequence set forth in SEQ ID NO: 13, and a light chain variable region with the amino acid sequence set forth in SEQ ID NO: 14. In some embodiments, the antibody used in the drug conjugate, composition, use, or method of the present disclosure comprises a heavy chain with the amino acid sequence set forth in SEQ ID NO: 19, and a light chain with the amino acid sequence set forth in SEQ ID NO: 20.

The anti-BDCA2 antibody Hu033-20 or the antigen-binding fragment thereof described herein is prepared as described in CN202210599888.X. In some embodiments, the CDR sequences of the antibody used in the drug conjugate, composition, use, or method of the present disclosure comprise HCDR1, HCDR2, and HCDR3 with the amino acid sequences set forth in SEQ ID NO: 1, SEQ ID NO: 2, and SEQ ID NO: 3, respectively, and LCDR1, LCDR2, and LCDR3 with the amino acid sequences set forth in SEQ ID NO: 4, SEQ ID NO: 5, and SEQ ID NO: 6, respectively. In some embodiments, the variable region sequences of the antibody used in the drug conjugate, composition, or use of the present disclosure comprises a heavy chain variable region with the amino acid sequence set forth in SEQ ID NO: 15, and a light chain variable region with the amino acid sequence set forth in SEQ ID NO: 16. In some embodiments, the antibody used in the drug conjugate, composition, use, or method of the present disclosure comprises a heavy chain with the amino acid sequence set forth in SEQ ID NO: 21, and a light chain with the amino acid sequence set forth in SEQ ID NO: 22.

The anti-BDCA2 antibody Hu005-04 or the antigen-binding fragment thereof described herein is prepared as described in CN202210599888.X. In some embodiments, the CDR sequences of the antibody used in the drug conjugate, composition, use, or method of the present disclosure comprise HCDR1, HCDR2, and HCDR3 with the amino acid sequences set forth in SEQ ID NO: 7, SEQ ID NO: 8, and SEQ ID NO: 9, respectively, and LCDR1, LCDR2, and LCDR3 with the amino acid sequences set forth in SEQ ID NO: 10, SEQ ID NO: 11, and SEQ ID NO: 12, respectively. In some embodiments, the variable region sequences of the antibody used in the drug conjugate, composition, or use of the present disclosure comprises a heavy chain variable region with the amino acid sequence set forth in SEQ ID NO: 17, and a light chain variable region with the amino acid sequence set forth in SEQ ID NO: 18. In some embodiments, the antibody used in the drug conjugate, composition, use, or method of the present disclosure comprises a heavy chain with the amino acid sequence set forth in SEQ ID NO: 23, and a light chain with the amino acid sequence set forth in SEQ ID NO: 24.

In certain preferred embodiments of the present disclosure, certain groups in the structures represented by formulas (I), (II), (III-A), and (III-A1) to (III-A4) are defined as follows, and the groups not mentioned are as described in any one of the embodiments of the present application ("in some embodiments" for short).

In some embodiments, in the antibody-drug conjugate described herein, the glucocorticoid molecule comprises or is selected from the following structures: a structure represented by formula (I), and a tautomer, a mesomer, a racemate, an enantiomer, a diastereoisomer or a mixture thereof, or a pharmaceutically acceptable salt or a solvate thereof, 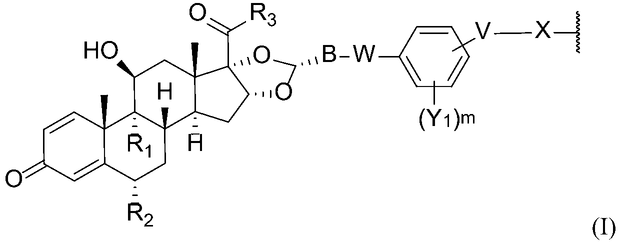 wherein
X is selected from: -O-, -S-, and -N(R^{1a})-, wherein R^{1a} is selected from H, C₁-C₆ alkyl, and -C₁-C₆ alkylhydroxy;
R₁ and R₂ are each independently selected from H, protium, deuterium, tritium, halogen, C₁-C₆ alkyl, and halogenated C₁-C₆ alkyl;
R₃ is selected from C₁-C₆ alkyl, halogenated C₁-C₆ alkyl, C₁-C₆ alkoxy, halogenated C₁-C₆ alkoxy, C₁-C₆ alkylthio, halogenated C₁-C₆ alkylthio, -C₁-C₆ alkyl-O-P(=O)(OC₁-C₆ alkyl)₂, and -C₁-C₆ alkyl-O-P(=O)(OH)₂, wherein the C₁-C₆ alkyl, C₁-C₆ alkoxy, and C₁-C₆ alkylthio are each independently optionally substituted with one or more substituents selected from halogen, CN, OH, or SH;
ring B is selected from phenyl or 5- to 6-membered heteroaryl, and ring B is optionally substituted with one or more substituents selected from halogen, OH, CN, NH₂, C₁-C₆ alkyl, C₁-C₆ alkoxy, and halogenated C₁-C₆ alkyl;
W is selected from a single bond, -O-, -S-, -C(O)-, -NH-, -C(O)NH-, -NHC(O)-, -C(O)-N(C₁-C₆ alkyl)-, -N(C₁-C₆ alkyl)-C(O)-, -C₁-C₆ alkylene-, -N(C₁-C₆ alkyl)-, -C₁₋₆ alkylene-NH-, -O-C₁-C₆ alkylene-, and -C₁-C₆ alkylene-O-; the C₁-C₆ alkyl and C₁-C₆ alkylene are optionally substituted with one or more substituents selected from halogen, C₁-C₆ alkyl, C₁-C₆ alkoxy, halogenated C₁-C₆ alkyl, C₃-C₆ cycloalkyl, oxo, OH, CN, or NH₂;
V is selected from a single bond or -(C(R^{2a})(R^{2b}))ₙ-, wherein R^{2a} and R^{2b} are each independently selected from H, halogen, OH, NH₂, -CN, C₁-C₆ alkyl, and halogenated C₁-C₆ alkyl, or R^{2a} and R^{2b}, together with the carbon atom to which they are linked, form carbonyl or C₃₋₆ cycloalkyl, and n is selected from 1, 2, 3, 4, 5, or 6;
Y₁ is selected from H, halogen, OH, nitro, NH₂, -NH(C₁-C₆ alkyl), -N(C₁-C₆ alkyl)₂, C₁-C₆ alkyl, -CN, C₁-C₆ alkoxy, -C₁-C₆ alkylhydroxy, and halogenated C₁-C₆ alkyl;
m is selected from 1, 2, or 3.

In some embodiments, in the antibody-drug conjugate described herein, X is selected from -O-, -S-,
and -N(R^{1a})-, wherein R^{1a} is selected from H, -CH₃, and -CH₂CH₃.

In some embodiments, in the antibody-drug conjugate described herein, X is selected from -O-. In some embodiments, in the antibody-drug conjugate described herein, X is selected from - NH-.

In some embodiments, in the antibody-drug conjugate described herein, X is selected from -S-. In some embodiments, in the antibody-drug conjugate described herein, R₁ and R₂ are each independently
selected from H, F, Cl, and -CH₃.

In some embodiments, in the antibody-drug conjugate described herein, R₁ and R₂ are each independently
selected from H or F.

In some embodiments, in the antibody-drug conjugate described herein, R₃ is selected from - CH₂Cl, - CH₂SH, -CH₂OH 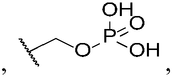 -OCH₃, -OCH₂F, -OCH₂Cl, -OCH₂CN, -OCH₂CH₃, -SCH₂F, -SCH₂Cl, -SCH₂CF₃, and -SCH₂CN.

In some embodiments, in the antibody-drug conjugate described herein, R₃ is selected from - CH₂OH, - SCH₂F and

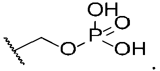

In some embodiments, in the antibody-drug conjugate described herein, R₃ is selected from - CH₂OH and

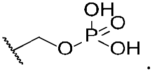

In some embodiments, in the antibody-drug conjugate described herein, ring B is selected from phenyl, pyridinyl,
thienyl, pyrrolyl, furanyl, pyrazolyl, imidazolyl, and thiazolyl, and ring B is optionally substituted with one or more substituents selected from halogen, OH, CN, NH₂, C₁-C₆ alkyl, C₁-C₆ alkoxy, and halogenated C₁-C₆ alkyl.

In some embodiments, in the antibody-drug conjugate described herein, ring B is selected from phenyl and thienyl, and ring B is optionally substituted with one or more substituents selected from halogen, OH, CN, NH₂, -CH₃, -CH₂CH₃, or -OCH₃.

In some embodiments, in the antibody-drug conjugate described herein, ring B is selected from

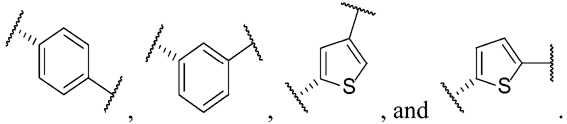

In some embodiments, in the antibody-drug conjugate described herein, ring B is

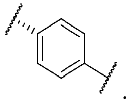

In some embodiments, in the antibody-drug conjugate described herein, ring B is

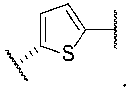

In some embodiments, in the antibody-drug conjugate described herein, W is selected from -C₁-C₆ alkylene-, wherein the C₁-C₆ alkylene is optionally substituted with one or more substituents selected from halogen, C₁-C₃ alkyl, C₁-C₃ alkoxy, halogenated C₁-C₃ alkyl, C₃-C₆ cycloalkyl, oxo, OH, CN, or NH₂.

In some embodiments, in the antibody-drug conjugate described herein, W is selected from 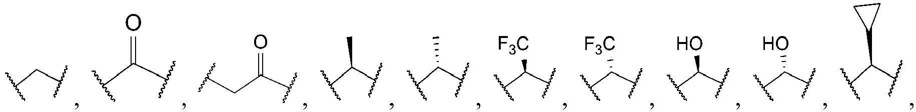 and

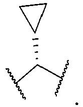

In some embodiments, in the antibody-drug conjugate described herein, W is selected from

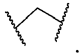

In some embodiments, in the antibody-drug conjugate described herein, V is selected from a single bond or -(C(R^{2a})(R^{2b}))ₙ-, wherein R^{2a} and R^{2b} are each independently selected from H, halogen, OH, NH₂, -CN, C₁-C₃ alkyl, and halogenated C₁-C₃ alkyl, and n is selected from 1, 2, or 3.

In some embodiments, in the antibody-drug conjugate described herein, V is selected from a single bond, -CH₂-,
or -CH₂CH₂-.

In some embodiments, in the antibody-drug conjugate described herein, Y₁ is selected from H, halogen, OH, NH₂, -CN, C₁-C₃ alkyl, C₁-C₃ alkoxy, -C₁-C₃ alkylhydroxy, and halogenated C₁-C₃ alkyl.

In some embodiments, in the antibody-drug conjugate described herein, Y₁ is selected from H, halogen, OH, NH₂, -CN, -CH₃, -CH₂CH₃, -OCH₃, and -CH₂OH.

In some embodiments, in the antibody-drug conjugate described herein, m is 1.

In some embodiments, in the antibody-drug conjugate described herein, the structure of the glucocorticoid molecule
is selected from:

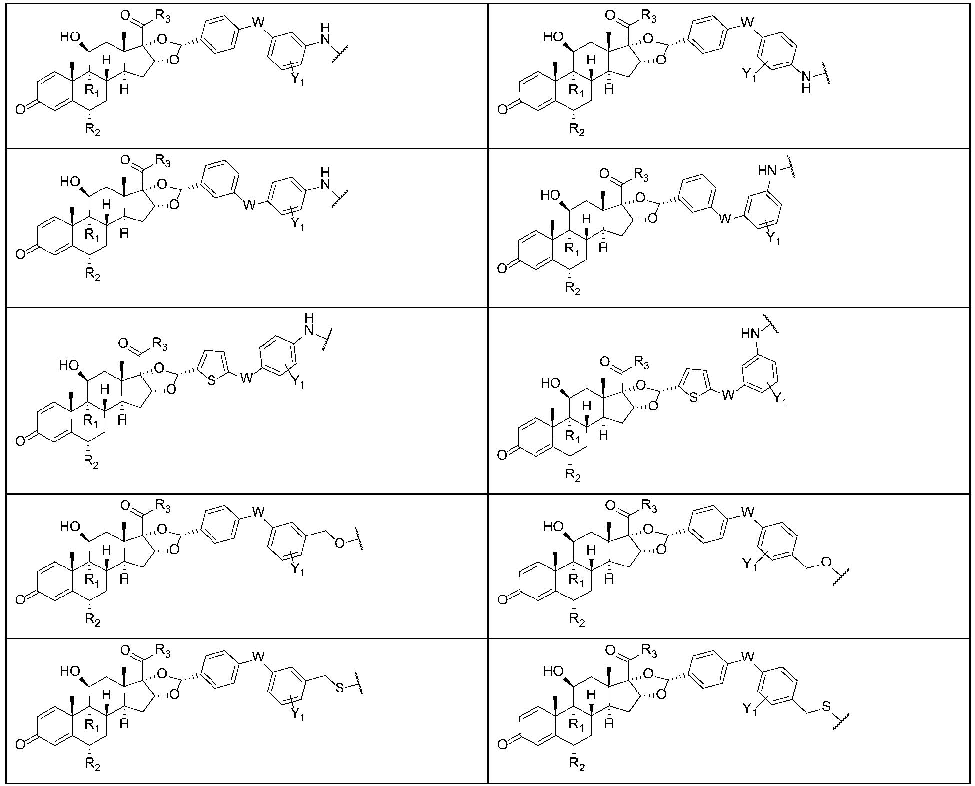

wherein
R₁ and R₂ are as defined in any one of the embodiments herein;
R₃ is as defined in any one of the embodiments herein;
W is as defined in any one of the embodiments herein;
Y₁ is as defined in any one of the embodiments herein.

In some embodiments, in the antibody-drug conjugate described herein, the glucocorticoid molecule is selected
from:

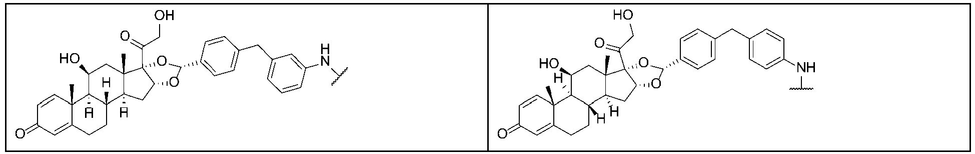

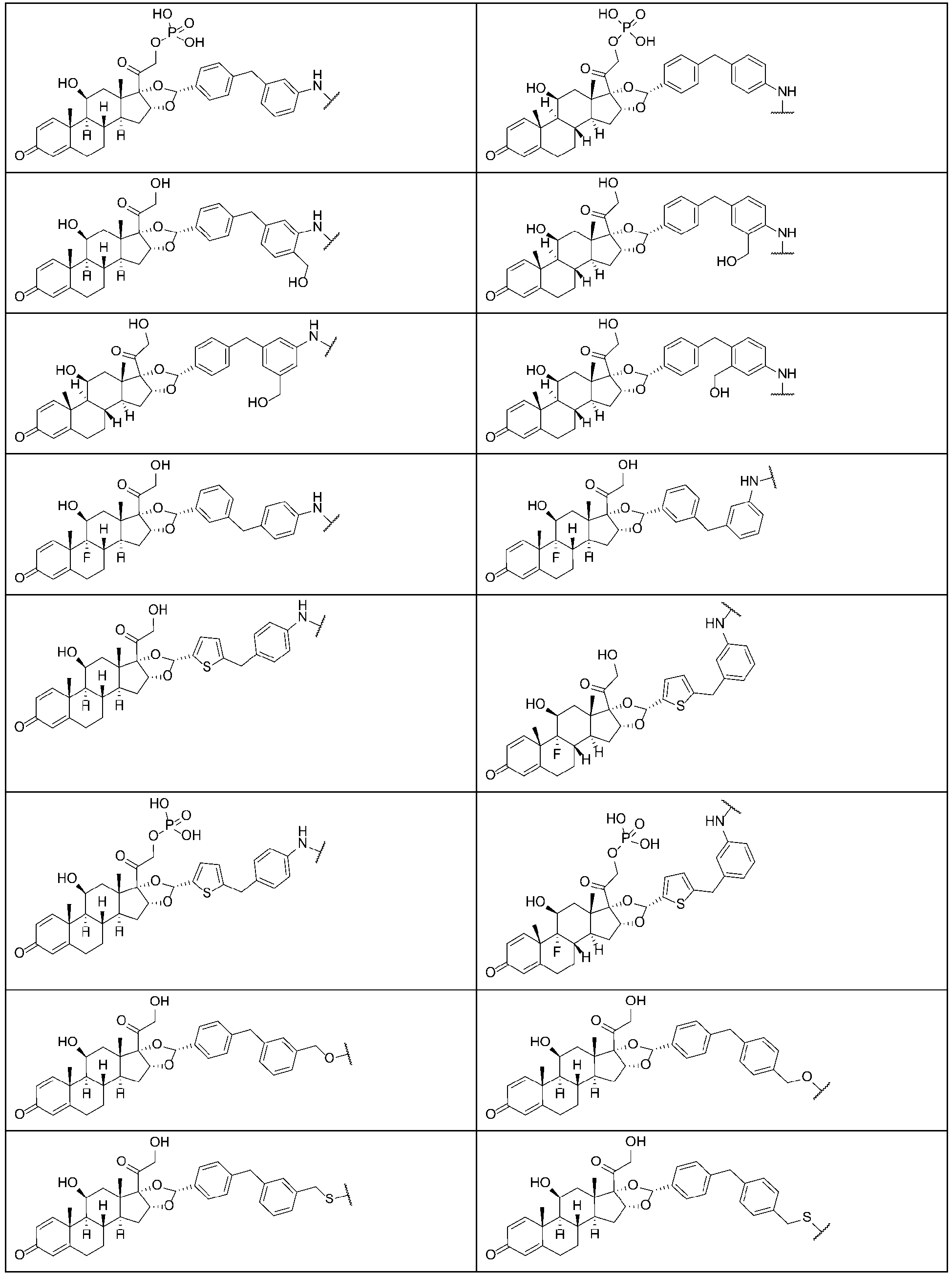

In some embodiments, in the antibody-drug conjugate described herein, the linker fragment (L) comprises Tr, L₁, L₂, and L₃, and the antibody-drug conjugate comprises a structure represented by formula (II): 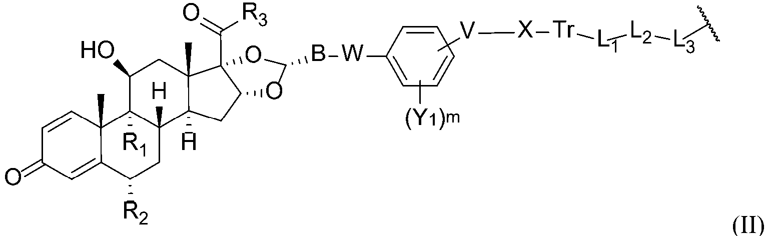 wherein Tr is absent or Tr is any group;
L₃ is selected from a polypeptide fragment;
L₂ is absent or selected from a linker fragment;
L₁ is selected from a coupling unit.
X, R₁, R₂, R₃, B, W, V, Y₁, and m are as defined in any one of the embodiments of the present application.

In some embodiments, in the antibody-drug conjugate described herein,
Tr is absent or selected from: 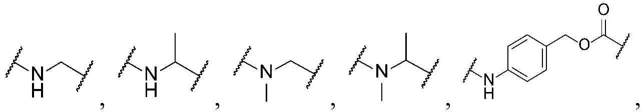 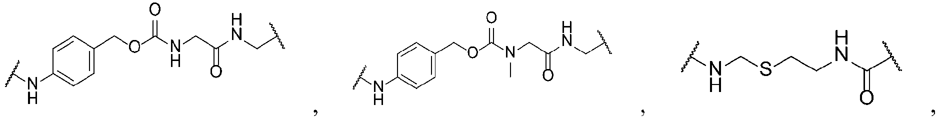 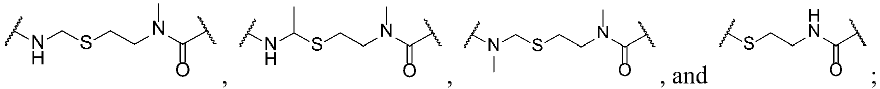
L₃ is selected from: glycine-glycine-phenylalanine-glycine (GGFG), alanine-alanine-alanine-glycine (AAAG), glycine-glycine-glycine-glycine (GGGG), valine-alanine-glycine (VAG), valine-citrulline-glycine (VCG), alanine-alanine-glycine (AAG), alanine-alanine-alanine (AAA), valine-alanine (VA), valine-citrulline (VC), alanine-alanine (AA), glutamic acid-alanine-glycine-glycine (EAGG), glycine-glutamic acid-alanine-glycine (GEAG), glycine-glutamic acid-glycine-glycine (GEGG), glutamic acid-glycine-glycine (EGG), glutamic acid-alanine-glycine (EAG), valine-lysine-glycine (VKG), glycine-glutamic acid-glycine (GEG), glutamic acid-alanine (EA), glutamic acid-glycine (EG), and glycine-glutamic acid (GE);
L₂ is absent or selected from: 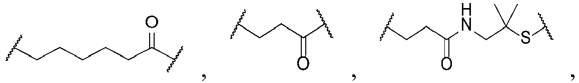 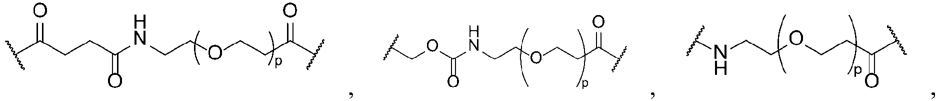 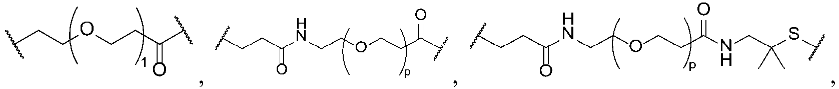 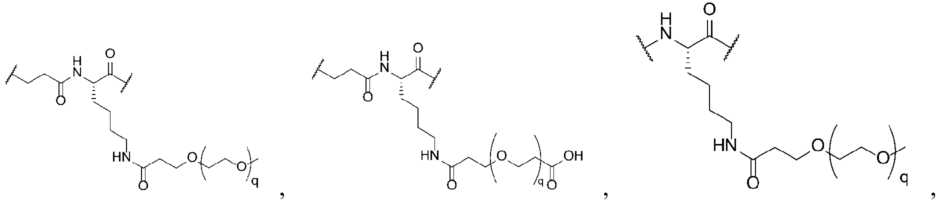 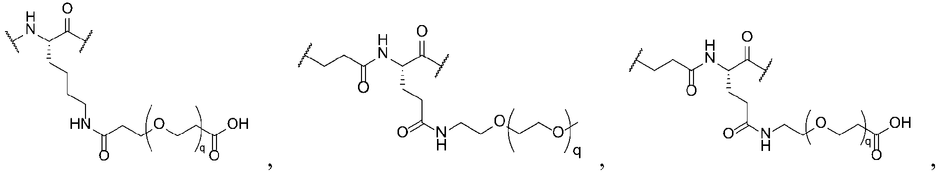 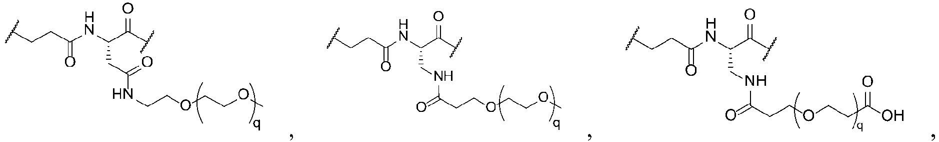 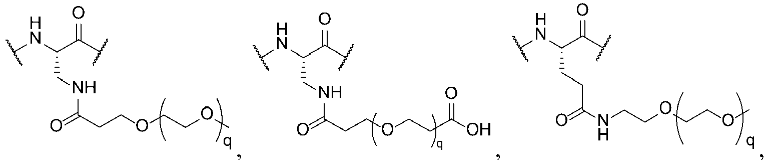 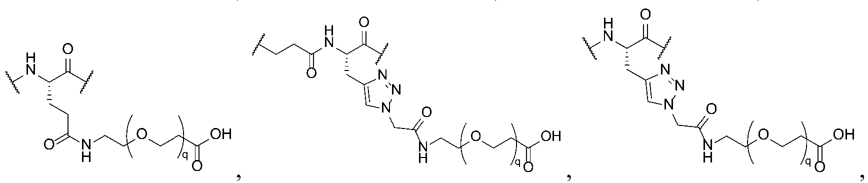 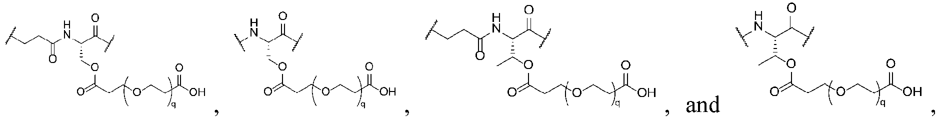
wherein q is selected from any integer from 1 to 30, and p is any integer from 1 to 20;
L₁ is selected from:
   (1) when L₁ is coupled to Ab via sulfhydryl, L₁ is selected from the following structures: 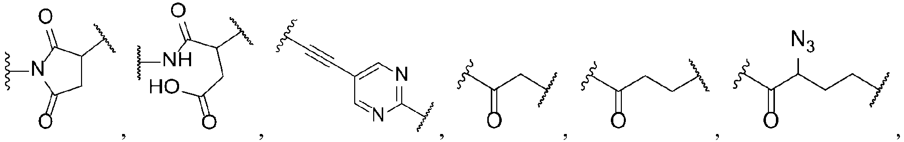 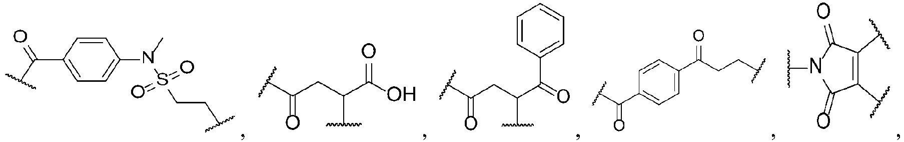 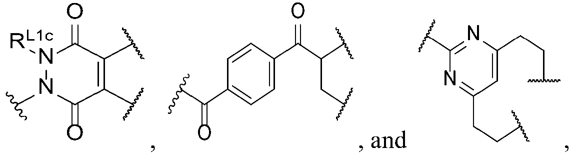 wherein R^{L1c} is selected from: hydrogen, optionally substituted alkyl, and optionally substituted aryl;
   (2) when L₁ is coupled to Ab via amino, L₁ is selected from the following structures: 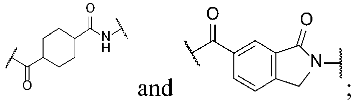
   (3) when L₁ is coupled to Ab via click chemistry, L₁ is selected from the following structures:

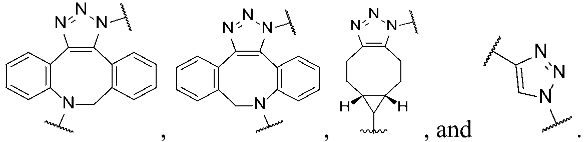

In some embodiments, in the antibody-drug conjugate described herein, the structural unit -Tr-L₃-L₂-L₁- is selected from:

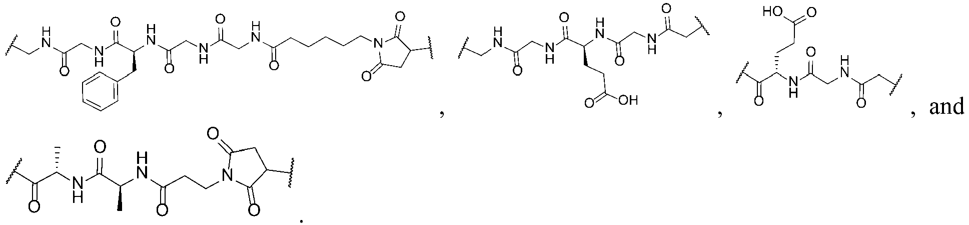

In some embodiments, the antibody-drug conjugate described herein is of the structure represented by formula (III-A): 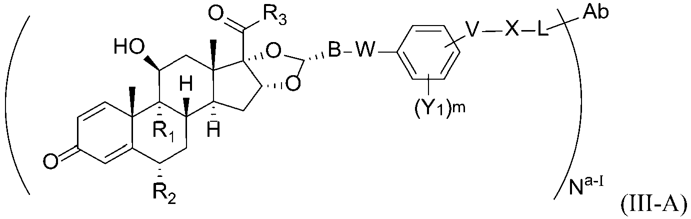 wherein
Ab is as defined in any one of the embodiments herein, and N^{a-I} is any number from 1 to 10;
L is as defined in any one of the embodiments herein;
X is selected from: -O-, -S-, and -N(R^{1a})-, wherein R^{1a} is selected from H, C₁-C₆ alkyl, and C₁-C₆ alkylhydroxy;
R₁ and R₂ are each independently selected from H, protium, deuterium, tritium, halogen, C₁-C₆ alkyl, and halogenated C₁-C₆ alkyl;
R₃ is selected from halogenated C₁-C₆ alkyl, C₁-C₆ alkoxy, C₁-C₆ alkylthio, and -C₁-C₆ alkyl-OP(=O)(OH)₂, wherein the C₁-C₆ alkyl, C₁-C₆ alkoxy, and C₁-C₆ alkylthio are each independently optionally substituted with one or more substituents selected from halogen, CN, OH, or SH;
ring B is selected from phenyl or 5- to 6-membered heteroaryl, and ring B is optionally substituted with one or more substituents selected from halogen, OH, CN, NH₂, C₁-C₆ alkyl, C₁-C₆ alkoxy, and halogenated C₁-C₆ alkyl;
W is selected from a single bond, -O-, -S-, -C(O)-, -NH-, -C(O)NH-, -NHC(O)-, -C(O)-N(C₁-C₆ alkyl)-, -N(C₁-C₆ alkyl)-C(O)-, -C₁-C₆ alkylene-, -N(C₁-C₆ alkyl)-, -C₁₋₆ alkylene-NH-, -O-C₁-C₆ alkylene-, and -C₁-C₆ alkylene-O-; the C₁-C₆ alkyl and C₁-C₆ alkylene are optionally substituted with one or more substituents selected from halogen, C₁-C₆ alkyl, C₁-C₆ alkoxy, halogenated C₁-C₆ alkyl, C₃-C₆ cycloalkyl, oxo, OH, CN, or NH₂;
V is selected from a single bond or -(C(R^{2a})(R^{2b}))ₙ-, wherein R^{2a} and R^{2b} are each independently selected from H, halogen, OH, NH₂, -CN, C₁-C₆ alkyl, and halogenated C₁-C₆ alkyl, or R^{2a} and R^{2b}, together with the carbon atom to which they are linked, form carbonyl or C₃₋₆ cycloalkyl, and n is selected from 1, 2, 3, 4, 5, or 6;
Y₁ is selected from H, halogen, OH, NH₂, C₁-C₆ alkyl, -CN, C₁-C₆ alkoxy, -C₁-C₆ alkylhydroxy, and halogenated C₁-C₆ alkyl;
m is selected from 1, 2, or 3.

In some embodiments, the antibody-drug conjugate described herein is of the structures represented by formulas (III-A1) to (III-A4): 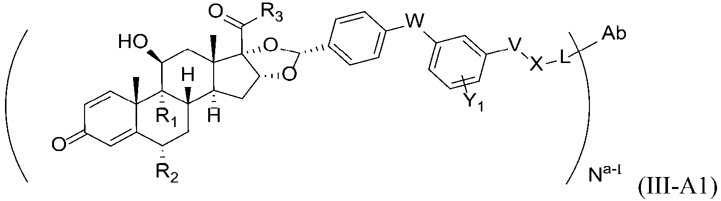 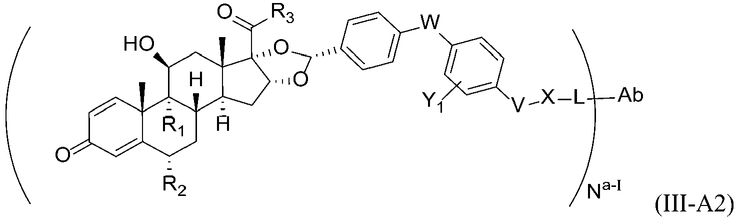 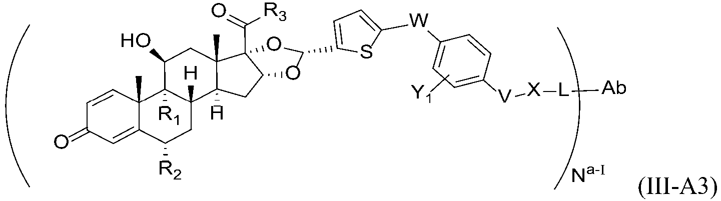 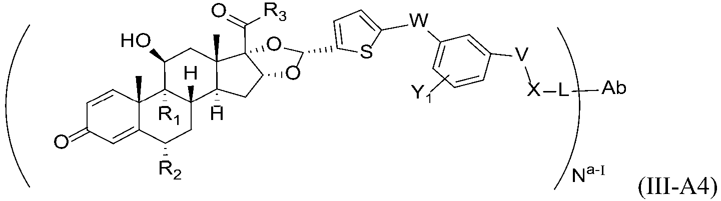 wherein
Ab is as defined in any one of the embodiments herein, and N^{a-I} is any number from 1 to 10;
L is selected from 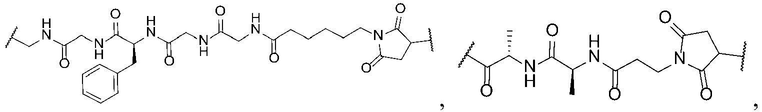 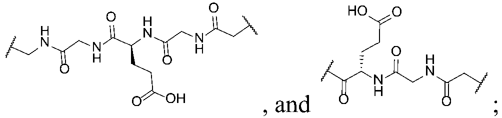
X is selected from: -O-, -S-, and -N(R^{1a})-, wherein R^{1a} is selected from H, C₁-C₆ alkyl, and C₁-C₆ alkylhydroxy;
R₁ and R₂ are each independently selected from H, protium, deuterium, tritium, halogen, C₁-C₆ alkyl, and halogenated C₁-C₆ alkyl;
R₃ is selected from halogenated C₁-C₆ alkyl, C₁-C₆ alkoxy, C₁-C₆ alkylthio, and -C₁-C₆ alkyl-OP(=O)(OH)₂, wherein the C₁-C₆ alkyl, C₁-C₆ alkoxy, and C₁-C₆ alkylthio are each independently optionally substituted with one or more substituents selected from halogen, CN, OH, or SH;
ring B is selected from phenyl or 5- to 6-membered heteroaryl, and ring B is optionally substituted with one or more substituents selected from halogen, OH, CN, NH₂, C₁-C₆ alkyl, C₁-C₆ alkoxy, and halogenated C₁-C₆ alkyl;
W is selected from a single bond, -O-, -S-, -C(O)-, -NH-, -C(O)NH-, -NHC(O)-, -C(O)-N(C₁-C₆ alkyl)-, -N(C₁-C₆ alkyl)-C(O)-, -C₁-C₆ alkylene-, -N(C₁-C₆ alkyl)-, -C₁₋₆ alkylene-NH-, -O-C₁-C₆ alkylene-, and -C₁-C₆ alkylene-O-; the C₁-C₆ alkyl and C₁-C₆ alkylene are optionally substituted with one or more substituents selected from halogen, C₁-C₆ alkyl, C₁-C₆ alkoxy, halogenated C₁-C₆ alkyl, C₃-C₆ cycloalkyl, oxo, OH, CN, or NH₂;
V is selected from a single bond or -(C(R^{2a})(R^{2b}))ₙ-, wherein R^{2a} and R^{2b} are each independently selected from H, halogen, OH, NH₂, -CN, C₁-C₆ alkyl, and halogenated C₁-C₆ alkyl, or R^{2a} and R^{2b}, together with the carbon atom to which they are linked, form carbonyl or C₃₋₆ cycloalkyl, and n is selected from 1, 2, 3, 4, 5, or 6;
Y₁ is selected from H, halogen, OH, NH₂, C₁-C₆ alkyl, -CN, C₁-C₆ alkoxy, -C₁-C₆ alkylhydroxy, and halogenated C₁-C₆ alkyl;
m is selected from 1, 2, or 3.

In some embodiments, in formulas (III-A) and (III-A1) to (III-A4), Ab, N^{a-I}, L, X, R₁, R₂, R₃, ring B, W, V, and Y₁ are as described in any one of the embodiments of the present application. In some embodiments, the antibody-drug conjugate described herein is selected from the following structures: 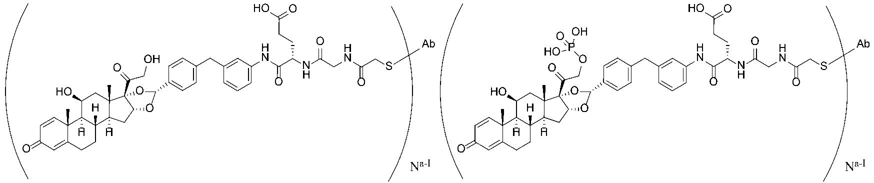 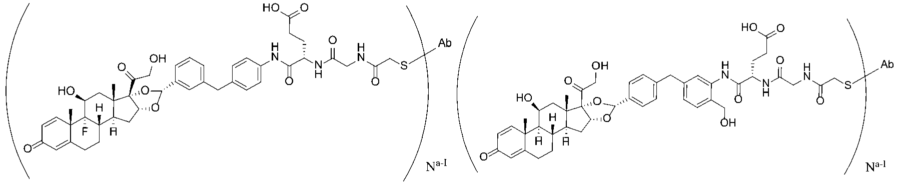 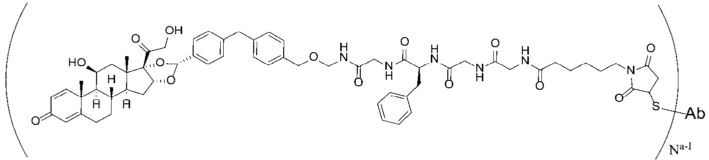 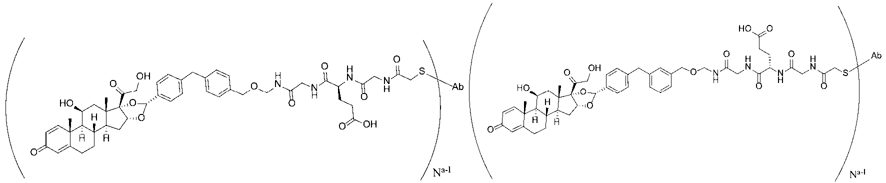 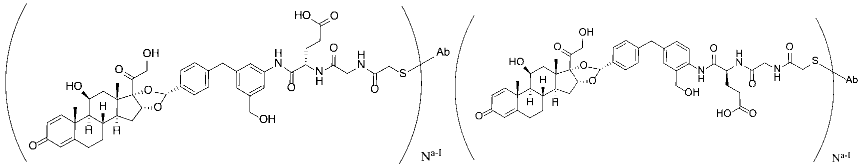 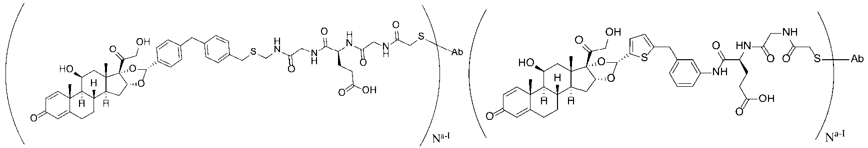 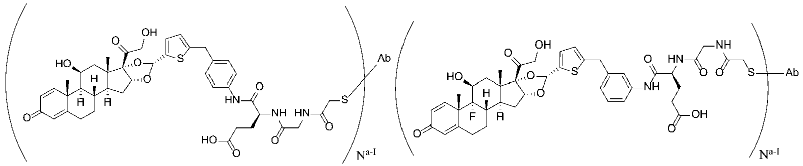 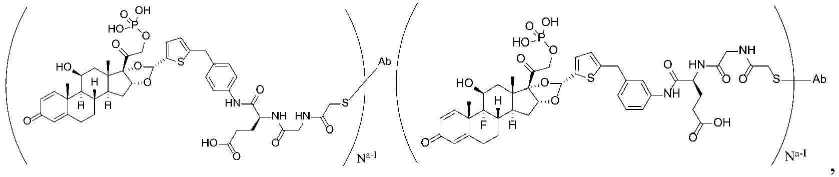 wherein each Ab is as defined in any one of the embodiments herein, and N^{a-I} is any number from 1 to 10.

In some embodiments, the antibody-drug conjugate described herein is selected from the following structures: 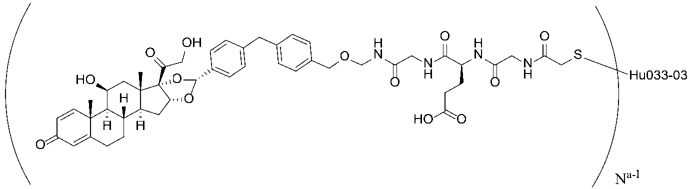 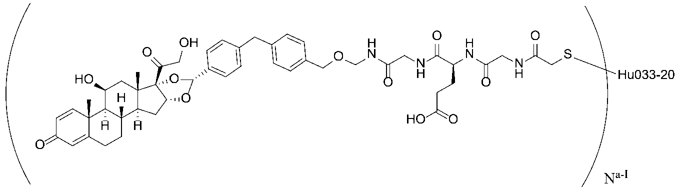 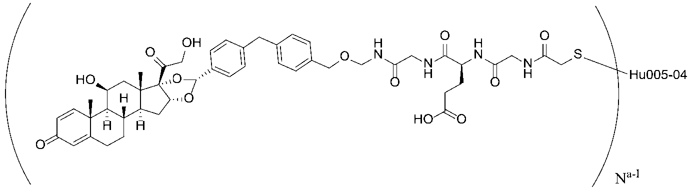 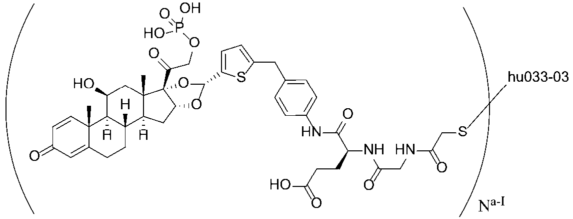 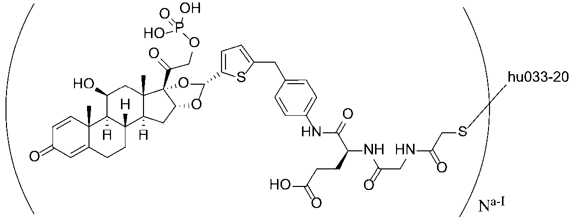 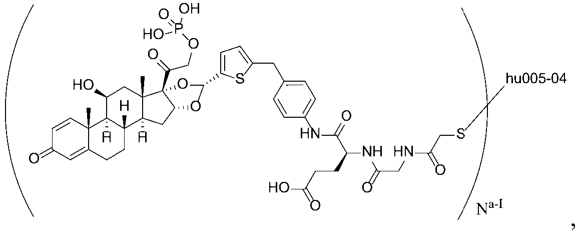 wherein N^{a-I} is any number from 1 to 10; preferably, N^{a-I} is an integer or a decimal from 2 to 8; preferably, N^{a-I} is an integer or a decimal from 3 to 8; preferably, N^{a-I} is an integer or a decimal from 3 to 4 or from 4 to 5, e.g., 4.01, 4.34, and 4.46; Hu033-03, Hu033-20, and Hu005-04 are anti-BDCA2 antibodies.

In some embodiments, the antibody-drug conjugate described herein is selected from the following structures: 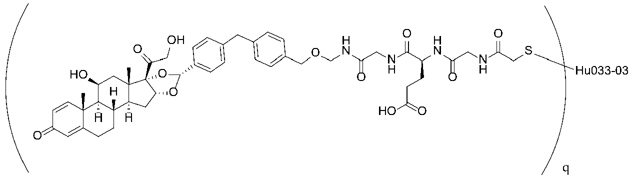 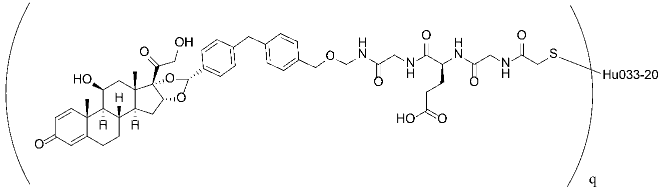 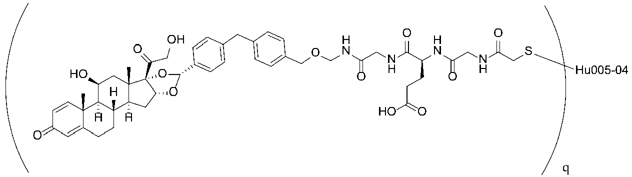 wherein the connection number q is an integer from 1 to 10; preferably, the connection number q is an integer from 2 to 8; preferably, the connection number q is an integer from 4 to 6; preferably, the connection number q is 4 or 6, preferably 4; Hu033-03, Hu033-20, and Hu005-04 are anti-BDCA2 antibodies.

The amino acid sequences of the light chain and the heavy chain of the anti-BDCA2 antibodies Hu033-03, Hu033-20, and Hu005-04 of the present disclosure are shown below. The antibody CDRs of the present disclosure are numbered using the Kabat numbering method.

Amino acid sequence of Hu033-03 (the underlined parts are CDRs)
Heavy chain variable region VH SEQ ID NO: 13 
EVQLVESGGGLVKPGGSLRLSCAASGFTFSSYGMSWVRQAPGKGLEWVSTISSGDSYT
YYPDSVKGRFTISRDNAKNSLYLQMNSLRAEDTAVYYCARQIYYDYAYYFDFWGQGT
TVTVSS
HCDR1: SYGMS (SEQ ID NO: 1)
HCDR2: TISSGDSYTYYPDSVKG (SEQ ID NO: 2)
HCDR3: QIYYDYAYYFDF (SEQ ID NO: 3)
Light chain variable region VL SEQ ID NO: 14 
DIVLTQSPASLAVSPGQRATITCRASESVSFRTSHLMHWYQQKPGQPPKLLIYGASNLES
GVPARFSGSGSETDFTLTINPVEAEDTANYFCQQSIEDPPTFGGGTKVEIK
LCDR1: RASESVSFRTSHLMH (SEQ ID NO: 4)
LCDR2: GASNLES (SEQ ID NO: 5)
LCDR3: QQSIEDPPT (SEQ ID NO: 6)

Amino acid sequence of Hu033-20 (the underlined parts are CDRs)
Heavy chain variable region VH SEQ ID NO: 15 
EVQLVESGGGLVKPGGSLRLSCAASGFTFRSYGMSWVRQAPGKRLEWVSTISSGDSYT
YYPDSVKGRFTISRDNAKNSLYLQMNSLKAEDTAVYYCARQIYYDYAYYFDFWGQGT
TVTVSS
HCDR1: SYGMS (SEQ ID NO: 1)
HCDR2: TISSGDSYTYYPDSVKG (SEQ ID NO: 2)
HCDR3: QIYYDYAYYFDF (SEQ ID NO: 3)
Light chain variable region VL SEQ ID NO: 16 
DIVMTQSPDSLAVSLGERATINCRASESVSFRTSHLMHWYQQKPGQPPKLLIYGASNLE
SGVPDRFSGSGSGTDFTLTISSLQAEDVAVYYCQQSIEDPPTFGGGTKVEIK
LCDR1: RASESVSFRTSHLMH (SEQ ID NO: 4)
LCDR2: GASNLES (SEQ ID NO: 5)
LCDR3: QQSIEDPPT (SEQ ID NO: 6)

Amino acid sequence of Hu005-04 (the underlined parts are CDRs)
Heavy chain variable region VH SEQ ID NO: 17 
QVQLQQSGPGLVKPSETLSLTCTVSGGSLTNYGVHWIRQPPGKGLEWIGVIWSGESTD
YDAAFISRVTISKDNSKNQVSLKLSSVTAADTAVYYCARRRSHYYGYVMDYWGQGTT
VTVSS
HCDR1: NYGVH (SEQ ID NO: 7)
HCDR2: VIWSGESTDYDAAFIS (SEQ ID NO: 8)
HCDR3: RRSHYYGYVMDY (SEQ ID NO: 9)
Light chain variable region VL SEQ ID NO: 18 
DIVLTQSPASLAVSPGQRATITCKASQSIDYdaIGYLNWYQQKPGQPPKLLIYAASNLES
GVPARFSGSGSGTDFTLTINPVEANDTANYYCQQSNEDPPTFGQGTKLEIK
LCDR1: KASQSIDYDAIGYLN (SEQ ID NO: 10)
LCDR2: AASNLES (SEQ ID NO: 11)
LCDR3: QQSNEDPPT (SEQ ID NO: 12)
Hu033-03-HC-IgG1: SEQ ID NO: 19 
EVQLVESGGGLVKPGGSLRLSCAASGFTFSSYGMSWVRQAPGKGLEWVSTISSGDSYT
YYPDSVKGRFTISRDNAKNSLYLQMNSLRAEDTAVYYCARQIYYDYAYYFDFWGQGT
TVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPC
PAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK
Hu033-03-LC: SEQ ID NO: 20 
DIVLTQSPASLAVSPGQRATITCRASESVSFRTSHLMHWYQQKPGQPPKLLIYGASNLES
GVPΛRFSGSGSETDFTLTINPVEΛEDTΛNYFCQQSIEDPPTFGGGTKVEIKRTVΛΛPSVFI
FPPSDEQLKSGTASVVCLLNNFYPREAKVQWKVDNALQSGNSQESVTEQDSKDSTYSL
 SSTLTLSKADYEKHKVYACEVTHQGLSSPVTKSFNRGEC
Hu033-20-HC-IgG1: SEQ ID NO: 21 
EVQLVESGGGLVKPGGSLRLSCAASGFTFRSYGMSWVRQAPGKRLEWVSTISSGDSYT
YYPDSVKGRFTISRDNAKNSLYLQMNSLKAEDTAVYYCARQIYYDYAYYFDFWGQGT
TVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPC
PAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK
Hu033-20-LC: SEQ ID NO: 22 
DIVMTQSPDSLAVSLGERATINCRASESVSFRTSHLMHWYQQKPGQPPKLLIYGASNLE
SGVPDRFSGSGSGTDFTLTISSLQAEDVAVYYCQQSIEDPPTFGGGTKVEIKRTVAAPSV
FIFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC
Hu005-04-HC-IgG1: SEQ ID NO: 23 
QVQLQQSGPGLVKPSETLSLTCTVSGGSLTNYGVHWIRQPPGKGLEWIGVIWSGESTD
YDAAFISRVTISKDNSKNQVSLKLSSVTAADTAVYYCARRSHYYGYVMDYWGQGTT
VTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFF
LYSKLTVDKSRWQQGNVFSCSVMHEALHNHYTQKSLSLSPGK
Hu005-04-LC: SEQ ID NO: 24

DIVLTQSPASLAVSPGQRATITCKASQSIDYdaIGYLNWYQQKPGQPPKLLIYAASNLES
GVPARFSGSGSGTDFTLTINPVEANDTANYYCQQSNEDPPTFGQGTKLEIKRTVAAPSV
FIFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC

In some embodiments, the average connection number N^{a-I} described herein may be an integer or a decimal from 1 to 10. For example, the average connection number N^{a-I} may be an integer or a decimal from 2 to 8. For example, the average connection number N^{a-1} may be an integer or a decimal from 3 to 8. For example, the average connection number N^{a-1} may be an integer or a decimal from 1 to 2, 2 to 3, 3 to 4, 4 to 5, 5 to 6, 6 to 7, 7 to 8, 8 to 9, or 9 to 10.

In some embodiments, the connection number q described herein may be an integer from 1 to 10. For example, the connection number q may be an integer from 2 to 8. For example, the connection number q may be an integer from 3 to 8. For example, the connection number q may be any integer of 1, 2, 3, 4, 5, 6, 7, 8, 9, or 10.

In yet another aspect, the present disclosure provides a pharmaceutical composition, which comprises the antibody-drug conjugate described herein or a pharmaceutically acceptable salt thereof, and a pharmaceutically acceptable carrier or excipient.

A further object of the present disclosure is to provide a method for preparing the pharmaceutical composition of the present disclosure, the method comprising combining the compound described herein, or a pharmaceutically acceptable form thereof, or a mixture thereof, with one or more pharmaceutically acceptable carriers.

The pharmaceutically acceptable carrier that can be used in the pharmaceutical composition of the present disclosure is a pharmaceutically acceptable carrier, and suitable examples of the pharmaceutically acceptable carrier are as described in Remington's Pharmaceutical Sciences (2005).

The pharmaceutical composition may be administered in any form as long as it achieves prevention, alleviation, inhibition, or cure of a symptom in a human or animal patient. For example, it may be formulated into various suitable dosage forms according to the administration route.

In other embodiments, the administration of the compound or the pharmaceutical composition of the present disclosure may be combined with an additional treatment method. The additional treatment method may be selected from, but is not limited to: radiation therapy, chemotherapy, immunotherapy, or a combination thereof.

The present disclosure also relates to a pharmaceutical formulation, which comprises the compound of the present disclosure, or a pharmaceutically acceptable form thereof, or a mixture thereof as an active ingredient, or the pharmaceutical composition of the present disclosure. In some embodiments, the formulation is in the form of a solid formulation, a semisolid formulation, a liquid formulation, or a gaseous formulation.

In yet another aspect, the present disclosure provides use of the antibody-drug conjugate described herein or the pharmaceutical composition described herein in the preparation of a medicament for treating and/or preventing a disease or disorder mediated by BDCA2 or a plasmacytoid dendritic cell.

In yet another aspect, the present disclosure provides a method for treating and/or preventing a disease or disorder mediated by BDCA2 or a plasmacytoid dendritic cell, which comprises administering to a subject in need thereof the antibody-drug conjugate described herein or the pharmaceutical composition described herein.

In yet another aspect, the present disclosure provides the antibody-drug conjugate or the pharmaceutical composition described herein for use in treating and/or preventing a disease or disorder mediated by BDCA2 or a plasmacytoid dendritic cell.

In some embodiments, the disease and/or disorder may comprise a disease and/or disorder associated with glucocorticoid receptor signal transduction.

In some embodiments, the disease and/or disorder is selected from the group consisting of: proliferative diseases and/or symptoms, metabolic diseases and/or symptoms, inflammatory diseases and/or symptoms, and neurodegenerative diseases and/or symptoms.

In some embodiments, the disease and/or disorder is selected from an autoimmune disease and/or disorder.

In some embodiments, the disease and/or disorder is selected from an inflammatory disease and/or disorder.

In some embodiments, the disease and/or disorder is selected from systemic lupus erythematosus, discoid lupus, lupus nephritis, epidermal lupus erythematosus, rheumatoid arthritis, inflammatory bowel disease, systemic sclerosis (scleroderma), psoriasis, type I diabetes, dermatomyositis, and polymyositis.

In yet another aspect, the present disclosure provides a pharmaceutical combination, which comprises the antibody-drug conjugate described herein or the pharmaceutical composition described herein, and one or more additional therapeutic agents. The additional treatment method includes, but is not limited to: radiation therapy, chemotherapy, immunotherapy, or a combination thereof.

In yet another aspect, the present disclosure provides a kit, which comprises the antibody-drug conjugate described herein or the pharmaceutical composition described herein.

The administration regimen may be adjusted to provide the optimum desired response. For example, when the administration is performed in the form of an injection, a single bolus injection, bolus injection, and/or continuous infusion, etc. may be performed. For example, several separate doses may be administered over time, or the dose may be proportionally decreased or increased as indicated by the exigencies of the treatment situation. It is noted that dose values may vary with the type and severity of the condition to be alleviated, and may include single or multiple doses. Generally, the therapeutic dose will vary, depending on considerations such as the age, sex, and general health of the patient to be treated; the frequency of treatment and the nature of the desired effect; the extent of tissue damage; the duration of symptoms; and other variables that can be adjusted by physicians. It is further understood that for any particular individual, the specific administration regimen should be adjusted over time according to the individual need and the professional judgment of the person administering the composition or supervising the administration of the composition. The amount and regimen of administration of the pharmaceutical composition can be readily determined by those of ordinary skill in the clinical art. For example, the composition or compound of the present disclosure may be administered 4 times daily to once every 3 days in separate doses, and the administration dose may be, for example, 0.01-1000 mg/time. The desired dose may be administered once or more times to achieve the desired result. The pharmaceutical composition according to the present disclosure may also be provided in a unit dosage form.

### Definitions of terms

Unless otherwise stated, the present disclosure will be implemented with conventional techniques of molecular biology (including recombinant techniques), microbiology, cytobiology, biochemistry, and immunology, which are all within the skill of the art.

In order to facilitate the understanding of the present disclosure, some technical and scientific terms are specifically defined as follows. Unless otherwise specifically defined herein, all technical and scientific terms or expressions used herein have the meanings as commonly understood by those of ordinary skill in the art to which the present disclosure relates. For definitions and terminology in the art, those skilled can specifically refer, at least in part, to *Current Protocols in Molecular Biology* (Ausubel). Abbreviations for amino acid residues follow standard 3-letter and/or 1-letter codes used in the art to denote one of the 20 commonly used L-amino acids. The singular forms used herein (including claims) include their plural forms, unless otherwise specified in the context explicitly.

The term "about", when used in combination with a numerical value, is intended to encompass the numerical values in a range from a lower limit less than the specified numerical value by 5% to an upper limit greater than the specified numerical value by 5%, including but not limited to, ±5%, ±2%, ±1%, and ±0.1%, as these variations are suitable for implementing the disclosed methods.

The term "and/or" should be understood to refer to any one of the options or a combination of any two or more of the options.

The term "or" as used herein should be understood as having the same meaning as "and/or" defined above. For example, when items in a list are separated, "or" or "and/or" should be interpreted as being inclusive, that is, including at least one number or one of a list of elements, but also including more than one, and, optionally, additional unlisted items. Only in cases where contrary terms such as "only one", "exactly one", or "consisting of ..." as used in the claims are explicitly indicated will it refer to the one number solely listed or one element of the list.

Unless the contrary is explicitly indicated in the context, the words "a/an" and "one" as used herein should be interpreted as "at least one".

The term "BDCA2" herein is a type II C-type lectin BDCA2 specifically expressed on plasmacytoid dendritic cells (pDCs), which consists of a single extracellular carbohydrate recognition domain (CRD) at the C-terminus, a transmembrane region from the asparagine residue at position 45 to the isoleucine residue at position 213, and a short cytoplasmic tail (without a signal motif) at the N-terminus. BDCA2 sends intracellular signals through the related transmembrane adaptor FcεRIγ. Antibody-mediated binding of BDCA2 results in the recruitment of spleen tyrosine kinase (SYK) to the phosphorylated immunoreceptor tyrosinebased activation motif (ITAM) of FcεRIγ. The activation of Syk results in the activation of the B-cell linker (BLNK), Bruton's tyrosine kinase (BTK), and phospholipase Cγ2 (PLCγ2), thereby leading to the flux of Ca²⁺.

Unless otherwise stated, the term "BDCA2" includes variants, subtypes, and species homologs of human BDCA2 or BDCA2 of other species, and analogs having at least one common epitope of BDCA2. The term includes untreated full-length BDCA2 and BDCA2 in any form resulting from intracellular treatment. The term encompasses "full-length" unprocessed BDCA2 and BDCA2 in any form resulting from intracellular processing or any fragment thereof, such as a splice variant or an allelic variant. In one embodiment, BDCA2 refers to full-length BDCA2 from humans or cynomolgus monkeys, or fragments thereof (such as mature fragments thereof lacking a signal peptide). The term "anti-BDCA2 antibody", "anti-BDCA2", "BDCA2 antibody", or "antibody targeting BDCA2" refers to an antibody that is capable of binding to a BDCA2 protein or a fragment thereof with sufficient affinity such that the antibody can be used as a diagnostic and/or therapeutic agent targeting BDCA2.

The term "immune response" refers to the action of, for example, lymphocytes, antigen-presenting cells, phagocytes, granulocytes, and soluble macromolecules (including antibodies, cytokines, and complements) produced by the above cells or the liver, which results in the selective damage, destruction, or elimination of invading pathogens, pathogen-infected cells or tissues, cancer cells, or normal human cells or tissues in the case of autoimmunity or pathological inflammation from the human body.

The term "plasmacytoid dendritic cells (pDCs)" are a specialized population of bone marrowderived cells that secrete type I interferons (IFNs) in response to toll-like receptor (TLR) ligands. The pDCs are a key link of cellular immunity, involving induction and initiation of immune responses and antigen tolerance. The pDCs differ from normal DCs in that, for example, they acquire antigens through receptor-mediated internalization, etc. Type I interferons, produced by pDCs activated by pattern recognition receptors, can also present antigens, thereby correlating innate immune responses and adaptive immune responses. Meanwhile, excessive activation of pDCs may also have a negative impact on the process of immune responses, which may lead to, for example, autoimmune diseases.

In the present application, the terms "antibody-drug conjugate" and "ADC" are used interchangeably and generally refer to a binding protein (e.g., an antibody or an antigen-binding fragment thereof) that is chemically linked to one or more chemical drugs, which may optionally be a therapeutic agent or a cytotoxic agent. In the present application, the antibody may comprise an antibody or an antigen-binding fragment thereof. In the present application, the payload moiety may comprise a glucocorticoid molecule. In certain embodiments, the ligand may be linked to the glucocorticoid molecule via a linker fragment.

In the present application, the term "antibody" generally refers to an immunoglobulin that is reactive to a specified protein or peptide or a fragment thereof. The antibody may be an antibody of any class, including but not limited to, IgG, IgA, IgM, IgD, and IgE, and of any subclass (e.g., IgG1, IgG2, IgG3, and IgG4). The antibody may have a heavy chain constant region selected from, for example, IgG1, IgG2, IgG3, or IgG4. The antibody may also have a light chain selected from, for example, kappa (κ) or lambda (λ). The antibodies of the present application may be derived from any species. The term "antibody" may comprise intact polyclonal antibodies, intact monoclonal antibodies, chimeric antibodies, humanized antibodies, human antibodies, fusion proteins comprising an antibody and any other modified immunoglobulin molecules so long as the antibodies exhibit the desired biological activity.

In the present application, the term "antigen-binding fragment" generally refers to a portion of an antibody molecule that comprises amino acids responsible for specific binding of an antibody to an antigen. The portion of the antigen specifically recognized and bound by the antibody is referred to as the "epitope" described above. As described above, the antigen-binding domain may typically comprise an antibody light chain variable region (VL) and an antibody heavy chain variable region (VH); however, the antigen-binding domain does not necessarily comprise both. An Fd fragment, for example, has two VH regions and typically retains some of the antigen-binding functions of the intact antigen-binding domain. Examples of antigen-binding fragments of antibodies include: (1) a Fab fragment, a monovalent fragment having a VL, a VH, a constant light chain (CL), and a CH1 domain; (2) a F(ab')2 fragment, a bivalent fragment having two Fab fragments linked by a disulfide bridge at the hinge region; (3) an Fd fragment, having two VH and CH1 domains; (4) an Fv fragment, having VL and VH domains of a single arm of an antibody; (5) a dAb fragment (Ward et al., "Binding Activities of a Repertoire of Single Immunoglobulin Variable Domains Secreted From Escherichia coli", Nature 341: 544-546 (1989), which is incorporated herein by reference in its entirety), having a VH domain; (6) an isolated complementarity-determining region (CDR); (7) a single-chain Fv (scFv), e.g., derived from an scFV-library, although the two domains of the Fv fragment, VL and VH, are encoded by separate genes, they may be joined by a recombinant method using a synthetic linker that allows them to be prepared as a single protein chain in which the VL and VH regions pair to form a monovalent molecule (known as single-chain Fv (scFv)) (see, e.g., Huston et al., "Protein Engineering of Antibody Binding Sites: Recovery of Specific Activity in an Anti-Digoxin Single-Chain Fv Analogue Produced in Escherichia coli", Proc. Natl. Acad. Sci. USA 85: 5879-5883 (1988)); and (8) "VHH", which relates to a variable antigen-binding domain of a heavy chain antibody from Camelidae (camel, dromedary, llama, alpaca, etc.) (see Nguyen V.K. et al., 2000, The EMBO Journal, 19, 921-930; Muyldermans S., 2001, J Biotechnol., 74, 277-302; and a review of Vanlandschoot P. et al., 2011, Antiviral Research 92, 389-407). VHH may also be referred to as nanobody (Nb).

In the present application, the term "variable region" or "variable domain" generally refers to a domain of a heavy chain or light chain of an antibody involved in the binding of the antibody to an antigen. In the present application, the term "variable" generally means that certain portions of the sequences of the variable domains of antibodies vary considerably, resulting in the binding and specificity of various particular antibodies to their particular antigens. Variability is not evenly distributed throughout the variable regions of the antibody. It is concentrated in three segments in each of the light and heavy chain variable regions known as complementarity-determining regions (CDRs) or hypervariable regions (HVRs), namely LCDR1, LCDR2, LCDR3, HCDR1, HCDR2, and HCDR3. The more highly conserved portions of the variable domains are called framework regions (FRs). The variable domains of native heavy and light chains each comprise four FRs (H-FR1, H-FR2, H-FR3, H-FR4, L-FR1, L-FR2, L-FR3, and L-FR4) largely in a β-sheet configuration. The FRs are connected by three CDR structural loop regions. The CDRs in each chain are held together in close proximity by the FRs and, together with the CDRs from the other chain, form the antigen-binding sites of the antibody.

In the art, the variable regions of an antibody can be encoded or the CDRs of an antibody can be divided by various methods, such as the Kabat numbering scheme and definition rule based on sequence variability (see Kabat et al., Sequences of Proteins of Immunological Interest, fifth edition, National Institutes of Health, Bethesda, Md. (1991)), the Chothia numbering scheme and definition rule based on the positions of structural loop regions (see A1-Lazikani et al., J Mol Biol 273: 927-48, 1997), the IMGT numbering scheme and definition rule based on alignment of amino acid sequences of germline V genes by efranc et al., as well as the Honneger's numbering scheme (AHo's), the Martin numbering scheme, the Gelfand numbering scheme, etc., see Mathieu Dondelinger et al., Understanding the Significance and Implications of Antibody Numbering and Antigen-Binding Surface/Residue Definition, Front. Immunol., October 16, 2018.

A "monoclonal antibody" refers to an antibody obtained from a substantially homogeneous population of antibodies, i.e., the antibodies composing the population are identical except for possible naturally occurring mutations that may be present in minor amounts. A monoclonal antibody is highly specific and targets a single antigen epitope. In contrast, conventional (polyclonal) antibody preparations typically include a large number of antibodies targeting (or specific for) different epitopes. The modifier "monoclonal" indicates the characteristic of an antibody obtained from a substantially homogeneous population of antibodies, and is not to be construed as producing the antibody by any particular method.

The term "full-length antibody" refers to an immunoglobulin molecule comprising at least four peptide chains when present naturally, including two heavy (H) chains and two light (L) chains linked to each other by disulfide bonds. Each heavy chain consists of a heavy chain variable region (abbreviated herein as VH) and a heavy chain constant region (abbreviated herein as CH). The heavy chain constant region consists of 3 domains (CH1, CH2 and CH3). Each light chain consists of a light chain variable region (abbreviated herein as VL) and a light chain constant region. The light chain constant region consists of one domain CL. The VH and VL regions can be further divided into complementarity-determining regions (CDRs) with high variability and more conservative regions called framework regions (FRs) that are spaced apart by the CDRs. Each VH or VL region consists of 3 CDRs and 4 FRs arranged in the following order from the amino terminus to the carboxyl terminus: FR1, CDR1, FR2, CDR2, FR3, CDR3, FR4. The variable regions of the heavy and light chains comprise binding domains that interact with antigens. The constant regions of an antibody can mediate the binding of immunoglobulins to host tissues or factors, including the binding of various cells of the immune system (e.g., effector cells) to the first component (Clq) of a classical complement system.

The term "single-chain Fv" or "scFv" antibody refers to an antibody fragment comprising the VH and VL domains of an antibody, where these domains are present in a single polypeptide chain. In general, an Fv polypeptide also comprises a polypeptide linker between the VH and VL domains that enables the scFv to form the desired structure for antigen binding.

The term "domain antibody" is an immunofunctional immunoglobulin fragment that contains only the heavy chain variable region or the light chain variable region. In certain cases, two or more VH regions are covalently linked to a peptide linker to form a bivalent domain antibody. The 2 VH regions of the bivalent domain antibody may target the same or different antigens.

The term "bivalent antibody" comprises 2 antigen binding sites. In certain cases, the 2 binding sites have the same antigen specificity. However, the bivalent antibody may be bispecific.

The term "bispecific antibody" refers to a small antibody fragment having two antigen-binding sites and comprising a heavy chain variable domain (VH) linked to a light chain variable domain (VL) in the same polypeptide chain (VH-VL or VL-VH). By using a linker that is too short to allow pairing between two domains in one chain, the domains are forced to pair with the complementary domains of another chain to form two antigen-binding sites.

The term "humanized antibody" refers to an antibody form containing sequences from both human and non-human (such as mouse and rat) antibodies. In general, a humanized antibody comprises substantially all of at least one, and typically two, variable domains, in which all or substantially all of the hypervariable loops correspond to those of a non-human immunoglobulin and all or substantially all of the framework regions (FRs) are those of a human immunoglobulin sequence. The humanized antibody may optionally comprise at least a portion of a human immunoglobulin constant region (Fc).

The "isotype" of an antibody refers to the type of antibody (e.g., IgM, IgE, and IgG (such as IgG1, IgG2, or IgG4)) provided by the heavy chain constant region gene. Isotypes also include modified forms of one of these types in which modifications have been made to alter Fc function, for example, to enhance or attenuate effector function or binding to Fc receptors.

The term "Fc region" is used herein to define the C-terminal region of an immunoglobulin heavy chain comprising at least a part of constant regions. The term includes Fc regions of native sequences and variant Fc regions. In some embodiments, the Fc region of a human IgG heavy chain extends from Cys226 or Pro230 to the carboxyl-terminus of the heavy chain. However, the C-terminal lysine (Lys447) of the Fc region may or may not exist (numbering in this paragraph is according to the EU numbering system, also known as the EU index, Kabat et al., Sequences of Proteins of Immunological Interest, 5th Ed. Public Health Service, National Institutes of Health, Bethesda, MD, 1991).

The term "cross-reaction" refers to binding to antigenic fragments of the same target molecule of human, monkey and/or murine (mouse or rat) origin. Thus, "cross-reaction" should be understood as an interspecies reaction of an antigen-binding molecule (e.g., an antibody) with a similar molecule (e.g., BDCA2) expressed in a different species. The cross-reaction specificity of monoclonal antibodies recognizing human BDCA2, and monkey and/or murine (mouse or rat) BDCA2 can be determined by FACS analysis.

"Affinity" or "binding affinity" refers to the inherent binding affinity that reflects the interaction between members of a binding pair. The affinity of molecule X for its partner Y can be generally represented by the equilibrium dissociation constant (K_{D}), which is a ratio of the dissociation rate constant (k_{dis}) to the association rate constant (kₒₙ). Affinity can be measured by common methods known in the art. In some embodiments of the present disclosure, the affinity, e.g., between the antibody of the present disclosure and an antigen, is measured using the surface plasmon resonance (SPR) technology. In some preferred embodiments of the present disclosure, one specific method for measuring affinity is the BIAcore method herein.

The term "not bind to" a protein or cell means not binding to the protein or cell, or not binding to it with high affinity, that is, binding to the protein or cell with a K_{D} of 1.0 × 10⁻⁶ M or higher, more preferably 1.0 × 10⁻⁵ M or higher, more preferably 1.0 × 10⁻⁴ M or higher and 1.0 × 10⁻³ M or higher, and more preferably 1.0 × 10⁻² M or higher.

The term "high affinity" of IgG antibodies refers to a K_{D} for the antigen of 1.0 × 10⁻⁶ M or less, preferably 5.0 × 10⁻⁸ M or less, more preferably 1.0 × 10⁻⁸ M or less and 5.0 × 10⁻⁹ M or less, and more preferably 1.0 × 10⁻⁹ M or less. For other antibody subtypes, "high-affinity" binding may vary. For example, "high-affinity" binding of the IgM subtype refers to a K_{D} of 10⁻⁶ M or lower, preferably 10⁻⁷ M or lower, and more preferably 10⁻⁸ M or lower.

The term "percent (%) amino acid sequence identity" or simply "identity" is defined as the percentage of amino acid residues in a candidate amino acid sequence that are identical to those in a reference amino acid sequence after aligning the amino acid sequences (with gaps introduced if necessary) to achieve the maximum percent sequence identity without considering any conservative substitution as part of the sequence identity. Sequence alignment for the purpose of determining percent amino acid sequence identity can be performed using various methods in the art, for example, using computer software available to the public, such as the BLAST, BLAST-2, ALIGN or MEGALIGN (DNASTAR) software. Those skilled in the art can determine suitable parameters for measuring alignment, including any algorithm required to achieve the maximum alignment over the full length of the aligned sequences.

In the present application, the term "glucocorticoid" generally refers to naturally occurring steroid hormones or synthetic steroid hormones that interact with the glucocorticoid receptor.

In the present application, the term "halogen" generally refers to fluorine, chlorine, bromine, or iodine, and it may be, for example, fluorine or chlorine.

In the present application, the term "alkyl" generally refers to a residue derived from an alkane by the removal of a hydrogen atom. The alkyl may be substituted or unsubstituted, or replaced or unreplaced. The term "alkyl" generally refers to a saturated linear or branched aliphatic hydrocarbon group having a residue derived from a parent alkane by the removal of hydrogen atoms from the same carbon atom or two different carbon atoms, and it may be a linear or branched group containing 1 to 20 carbon atoms, e.g., 1 to 12 carbon atoms, such as alkyl containing 1 to 6 carbon atoms. Non-limiting examples of alkyl include but are not limited to methyl, ethyl, propyl, propyl, butyl, etc. The alkyl may be substituted or unsubstituted, or replaced or unreplaced. For example, when substituted, the alkyl may be substituted at any available connection site with a substituent that may be independently optionally selected from one or more of alkyl, alkenyl, alkynyl, alkoxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo, and the substituent may, e.g., be hydrogen, protium, deuterium, tritium, halogen, -NO₂, -CN, -OH, -SH, -NH₂, -C(O)H, - CO₂H, -C(O)C(O)H, -C(O)CH2C(O)H, -S(O)H, -S(O)₂H, -C(O)NH₂, -SO₂NH₂, -OC(O)H, - N(H)SO₂H, or a C₁₋₆ aliphatic group.

In the present application, the term "alkylene" generally refers to a saturated linear or branched aliphatic hydrocarbon group having 2 residues derived from a parent alkane by the removal of two hydrogen atoms from the same carbon atom or two different carbon atoms, and it may be a linear or branched group containing 1 to 20 carbon atoms; for example, the term "methylene" may refer to a residue derived from a one-carbon atom group by removal of two hydrogen atoms. The methylene may be substituted or unsubstituted, or replaced or unreplaced; for example, the alkylene contains 1 to 12 carbon atoms, e.g., alkylene containing 1 to 6 carbon atoms. Non-limiting examples of alkylene include but are not limited to methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), 1,5-butylene (-CH₂CH₂CH₂CH₂CH₂-), etc. The alkylene may be substituted or unsubstituted, or replaced or unreplaced. For example, when substituted, the alkylene may be substituted at any available connection site with a substituent that may be independently optionally selected from one or more of alkyl, alkenyl, alkynyl, alkoxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo, and, for example, may be hydrogen, protium, deuterium, tritium, halogen, -NO₂, -CN, -OH, -SH, -NH₂, -C(O)H, -CO₂H, - C(O)C(O)H, -C(O)CH₂C(O)H, -S(O)H, -S(O)₂H, -C(O)NH₂, -SO₂NH₂, -OC(O)H, -N(H)SO₂H, or C₁₋₆ aliphatic group. The methylene or the alkylene may be substituted or unsubstituted.

The term "alkoxy" refers to -O-(alkyl) and -O-(unsubstituted cycloalkyl), wherein the alkyl or cycloalkyl is as defined herein. Non-limiting examples of alkoxy include: methoxy, ethoxy, propoxy, butoxy, cyclopropyloxy, cyclobutoxy, cyclopentyloxy and cyclohexyloxy. The alkoxy may be optionally substituted or unsubstituted, and when it is substituted, the substituent is preferably one or more of the following groups independently selected from: alkyl, alkenyl, alkynyl, alkoxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, and heterocycloalkylthio.

The term "alkylthio" refers to -S-(alkyl) and -S-(unsubstituted cycloalkyl), wherein the alkyl or cycloalkyl is as defined herein. Non-limiting examples of alkylthio include: methylthio, ethylthio, propylthio, butylthio, cyclopropylthio, cyclobutylthio, cyclopentylthio, and cyclohexylthio. The alkylthio may be optionally substituted or unsubstituted, and when it is substituted, the substituent is preferably one or more of the following groups independently selected from: alkyl, alkenyl, alkynyl, alkoxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, and heterocycloalkylthio.

In the present application, the term "aryl" generally refers to a group having a residue derived from an aromatic ring by removal of a hydrogen atom. The term "aromatic ring" may refer to a 6- to 14-membered all-carbon monocyclic ring or fused polycyclic ring (i.e., rings that share adjacent pairs of carbon atoms) having a conjugated π-electron system, and it may be 6- to 10-membered, such as benzene and naphthalene. The aromatic ring can be fused to a heteroaryl, heterocyclyl or cycloalkyl ring, wherein the ring connected to the parent structure is the aryl ring. The aryl may be substituted or unsubstituted, and when it is substituted, the substituent may be one or more of the following groups independently selected from the group consisting of: alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio. The aryl may be substituted or unsubstituted.

In the present application, the term "heteroaryl" generally refers to a group having a residue derived from a heteroaromatic ring by removal of a hydrogen atom from a carbon atom. The term "heteroaromatic ring" refers to a heteroaromatic system containing 1 to 4 heteroatoms and 5 to 14 ring atoms, wherein the heteroatoms may be selected from the group consisting of: oxygen, sulfur, and nitrogen. The heteroaryl may be 5- to 10-membered and may be 5- or 6-membered, such as furanyl, thienyl, pyridinyl, pyrrolyl, N-alkylpyrrolyl, pyrimidinyl, pyrazinyl, imidazolyl, tetrazolyl, etc. The heteroaryl ring can be fused to an aryl, heterocyclyl or cycloalkyl ring, wherein the ring connected to the parent structure is the heteroaryl ring. The heteroaryl may be optionally substituted or unsubstituted, and when it is substituted, the substituent may be one or more of the following groups independently selected from the group consisting of: alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio. The heteroaryl may be substituted or unsubstituted.

In the present application, the term "cycloalkyl" refers to a saturated or partially unsaturated monocyclic or polycyclic hydrocarbon substituent, and the cycloalkyl ring contains 3 to 20 carbon atoms, preferably 3 to 12 carbon atoms, preferably 3 to 10 carbon atoms, preferably 3 to 8 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl. The cycloalkyl may be substituted or unsubstituted. When it is substituted, substitution with a substituent may be performed at any available linking site, and the substituent is preferably independently selected from one or more of hydrogen, halogen, alkyl, alkoxy, haloalkyl, hydroxy, hydroxyalkyl, cyano, amino, nitro, cycloalkyl, heterocyclyl, aryl, and heteroaryl.

In the present application, the term "ring-forming atom" generally refers to an atom contained in a cyclic structure. For example, a ring-forming atom may be a carbon atom in a benzene ring or may be a nitrogen atom in a pyridine ring. When a hydrogen atom is connected to a ring-forming atom, the ring-forming atom may be substituted or unsubstituted.

In the present application, the term "each independently" or "each ... is independently" generally means that a variable applies in any case irrespective of the presence or absence of variables having the same or different definitions in the same compound. For example, the variable may refer to the type or number of substituents in the compound, the type of atoms in the compound, and so on. For example, where R occurs twice in a compound and R is defined as "independently carbon or nitrogen", both R may be carbon, both R may be nitrogen, or one R may be carbon while the other R is nitrogen.

In the present application, the term "optional" or "optionally" generally means that the event or circumstance subsequently described may, but not necessarily occur, and that the description includes instances where the event or circumstance occurs or does not occur. For example, "heterocyclyl group optionally substituted with alkyl" means that alkyl may be, but not necessarily present, and that the description may include instances where the heterocyclyl group is or is not substituted with alkyl.

In the present application, the term "substituted" generally means that one or more hydrogen atoms in the group, for example, up to 5 (e.g., 1-3) hydrogen atoms, are each independently substituted with a corresponding number of substituents. A substituent is only in its possible chemical position, and those skilled in the art will be able to determine (by experiments or theories) possible or impossible substitution without undue efforts. For example, it may be unstable when amino or hydroxy having a free hydrogen is bound to a carbon atom having an unsaturated (such as olefin) bond.

In the present application, the terms such as "alkyl", "alkenyl" and "cycloalkyl" may be preceded by a notation to indicate the number of atoms present in the group under particular circumstances as in C₁-C₄ alkyl, C₃-C₇ cycloalkoxy, and C₁-C₄ alkylcarbonylamino and the like, as known to those skilled in the art, and the subscript numeral following "C" indicates the number of carbon atoms present in the group. For example, C₃ alkyl refers to an alkyl group having three carbon atoms (e.g., n-propyl or isopropyl); in C₁₋₁₀, members of the group may have any number of carbon atoms within the range of 1-10.

In the present application, the "linking" of group X to group Y may generally be in any orientation, which generally means that when group X is used for linker Y and group Z, two or more linking sites of group X may be linked arbitrarily to either group Y or group Z.

In the present application, the compound or antibody-drug conjugate of the present application includes tautomers, mesomers, racemates, enantiomers, and/or diastereoisomers thereof. In the present application, the term "diastereoisomer" generally refers to a stereoisomer that has two or more chiral centers and whose molecules are not mirror images of each other. Diastereoisomers may have different physical properties, e.g., melting points, boiling points, spectral properties, and reactivities. In the present application, the terms "tautomer" and "tautomeric form" are used interchangeably and generally refer to structural isomers of different energies that can be converted into each other by crossing a low energy barrier. For example, proton tautomers (also known as prototropic tautomers) include interconversions by proton migration, such as keto-enol isomerization and imine-enamine isomerization. Valence tautomers include interconversions by recombination of some bonding electrons. In the present application, the term "mesomer" generally means that the molecule contains asymmetric atoms but the total optical rotation is zero due to the presence of symmetric factors. The term "racemate" or "racemic mixture" refers to a composition of two enantiomeric substances in equimolar amounts.

In the present application, certain atoms of the compound of the present application may be present in one or more isotopic forms. For example, hydrogen may occur as protium (¹H), deuterium (²H), and tritium (³H), and carbon may naturally occur as three different isotopes (¹²C, ¹³C, and ¹⁴C). Examples of isotopes that can be incorporated into the compound of the present application also include but are not limited to ¹⁵N, ¹⁸O, ¹⁷O, ¹⁸F, ³²P, ³³P, ¹²⁹I, ¹³¹I, ¹²³I, ¹²⁴I, ¹²⁵I, or similar isotopes. Thus, one or more of such isotopes may be enriched in the compounds of the present application relative to the natural abundance of such isotopes. Such isotopically enriched compounds can be used for a variety of purposes, as known to those skilled in the art. For example, replacement with heavy isotopes such as deuterium (²H) may offer certain therapeutic advantages, possibly due to higher metabolic stability. For example, the natural abundance of deuterium (²H) is about 0.015%. Accordingly, one out of about 6500 hydrogen atoms is a deuterium atom. Accordingly, the deuterium abundance of one or more sites (as the case may be) in the deuterium-containing compound of the present disclosure is greater than 0.015%. Unless otherwise indicated, the structures described herein may also include compounds that differ only in the presence or absence of one or more isotopically enriched atoms. For example, compounds having a structure identical to the structure disclosed herein except for the substitution of the hydrogen atom by deuterium or tritium or the substitution of the carbon atom by carbon 13 or carbon 14 shall fall within the scope of the present application.

In the present application, the term "linker" generally refers to any chemical moiety capable of linking a protein (e.g., an antibody, an antibody fragment (e.g., an antigen-binding fragment), or a functional equivalent) to a glucocorticoid. The moiety may be a chemical moiety. In certain embodiments, the linker may be susceptible to cleavage, thereby facilitating release of the glucocorticoid molecule. For example, the linker may be susceptible to acid-induced cleavage, light-induced cleavage, peptidase-induced cleavage, esterase-induced cleavage, and disulfide bond cleavage under conditions that the glucocorticoid and/or antibody retains activity. In certain embodiments, the linker may be substantially resistant to cleavage.

The term "pharmaceutical composition" generally refers to a mixture containing one or more of the compounds described herein or a physiologically/pharmaceutically acceptable salt or prodrug thereof, and other chemical components, for example physiologically/pharmaceutically acceptable carriers and excipients. The pharmaceutical composition may promote the administration to an organism and facilitate the absorption of the active ingredient, thereby exerting biological activities. The preparation of conventional pharmaceutical compositions can refer to Chinese Pharmacopoeia. The pharmaceutical composition may be in the form of a sterile aqueous injection or oily suspension for intramuscular and subcutaneous administration. The suspension may be prepared according to a known technique using those suitable dispersing agents or wetting agents and suspending agents described above. The sterile formulation for injection may also be a sterile solution for injection or suspension prepared in a parenterally acceptable non-toxic diluent or solvent, e.g., a solution prepared in 1,3-butanediol. In addition, a sterile fixed oil may be conveniently used as a solvent or a suspending medium. For example, any blended fixed oil including synthetic mono- or di-glycerides can be used. In addition, fatty acids such as oleic acid may also be used in the preparation of injections.

The term "pharmaceutically acceptable salt" generally refers to a salt of the compound of the present application or a ligand-drug conjugate, or a salt of the compound described herein. Such salts may possess safety and/or efficacy when used in mammals and may possess the required biological activity, and the antibody-antibody-drug conjugate compound of the present application may form a salt with an acid. Non-limiting examples of pharmaceutically acceptable salts include: hydrochloride, hydrobromide, hydroiodide, sulfate, bisulfate, citrate, acetate, succinate, ascorbate, oxalate, nitrate, sorbate, hydrogen phosphate, dihydrogen phosphate, salicylate, hydrogen citrate, tartrate, maleate, fumarate, formate, benzoate, mesylate, ethanesulfonate, benzene sulphonate, and p-toluenesulfonate.

The term "pharmaceutically acceptable carrier" generally refers to a carrier or carrier agent that provides therapeutic agents, such as antibodies or polypeptides, genes, and other therapeutic agents. The term refers to any pharmaceutical carrier that does not itself induce the production of antibodies harmful to the individual receiving the composition and can be given without producing undue toxicity. Suitable carriers may be macromolecules that are large and metabolized slowly, such as proteins, polysaccharides, polylactic acids, polyglycolic acids, poly(amino acid)s, amino acid copolymers, lipid aggregates, and inactivated virus particles. Such carriers are well known to those skilled in the art. Pharmaceutically acceptable carriers in therapeutic compositions may include liquids such as water, saline, glycerol, and ethanol. Auxiliary substances, such as wetting or emulsifying agents, or pH buffering substances, may also be present in these carriers.

The terms "treatment" and "treating" generally refer to a method of achieving beneficial or desired results, including but not limited to therapeutic benefits. Therapeutic benefits include but are not limited to eradication, inhibition, reduction, or amelioration of the underlying disorder being treated. In addition, therapeutic benefits are achieved by eradicating, inhibiting, reducing, or ameliorating one or more physiological symptoms associated with the underlying disorder, and thus improvements are observed in the patient, but the patient may still be afflicted with the underlying disorder.

The terms "prevention" and "preventing" generally refer to a method of achieving beneficial or desired results, including but not limited to prophylactic benefits. For the purpose of prophylactic benefits, a pharmaceutical composition can be administered to a patient at risk of developing a particular disease, or to a patient who reports he/she has one or more physiological symptoms of the disease, even if the patient has not yet been diagnosed with the disease.

The term "subject" or "patient" generally refers to a human (i.e., a male or female in any age group, e.g., a pediatric subject (e.g., an infant, a child, or an adolescent) or an adult subject (e.g., a young person, a middle-aged person, or an elderly person)) and/or other primate (e.g., a cynomolgus monkey, or a rhesus monkey); a mammal, including commercially relevant mammals, such as cows, pigs, horses, sheep, goats, cats, and/or dogs; and/or a bird, including commercially relevant birds such as chickens, ducks, geese, quail and/or turkeys.

The terms "therapeutically effective amount", "therapeutically effective dose", and "effective amount" refer to an amount of the ligand-drug conjugate of the present disclosure that is effective in preventing or ameliorating one or more symptoms of a disease or condition or the progression of the disease or condition when administered alone or in combination with other therapeutic drugs to a cell, tissue or subject. The therapeutically effective dose also refers to a dose sufficient to cause amelioration of symptoms, e.g., an amount for treating, curing, preventing or ameliorating a related condition or promoting the treatment, cure, prevention or amelioration of such condition. When an active ingredient is administered to an individual alone, a therapeutically effective dose only refers to the amount of the ingredient. In the case of administration in combination, a therapeutically effective dose refers to the combined amount of active ingredients that produces a therapeutic effect, regardless of whether these active ingredients are administered in combination, sequentially or simultaneously. An effective amount of a therapeutic agent will result in an increase in a diagnostic index or parameter by at least 10%, generally at least 20%, preferably at least about 30%, more preferably at least 40%, and most preferably at least 50%.

As used herein, the term "disease mediated by BDCA2 or a plasmacytoid dendritic cell" refers to a disease in which plasmacytoid dendritic cells are involved in the development, progression, delay of disease, or the like, e.g., involving activation (e.g., abnormal activation or excessive activation) of plasmacytoid dendritic cells, and the involvement of BDCA2 or plasmacytoid dendritic cells directly or indirectly produces at least one of the following effects: the onset/development of the disease; increased/deepened pathological changes in the disease; an enlarged site/extent of disease impact; an aggravated body damage caused by the disease; an increased pain from the disease; disease insensitivity/resistance to treatment measures; a reduced tendency to self-heal and a poorer prognosis of the disease. In some embodiments of the present disclosure, the disease mediated by BDCA2 or a plasmacytoid dendritic cell is an inflammatory disease; in some specific embodiments of the present disclosure, the disease mediated by BDCA2 or a plasmacytoid dendritic cell is systemic lupus erythematosus, discoid lupus, lupus nephritis, epidermal lupus erythematosus, rheumatoid arthritis, inflammatory bowel disease, systemic sclerosis (scleroderma), psoriasis, type I diabetes, dermatomyositis, and/or polymyositis.

As used herein, the term "treat", "treating", or "treatment" for any disease or disorder refers in one embodiment to ameliorating the disease or disorder (i.e., slowing or arresting or reducing the progression of the disease or at least one of its clinical symptoms). In another embodiment, the "treat", "treating", or "treatment" refers to alleviating or ameliorating at least one physical parameter, including those physical parameters that may not be discernible by the patient. In another embodiment, the "treat", "treating", or "treatment" refers to modulating the disease or disorder, physically (e.g., stabilization of discernible symptoms), physiologically (e.g., stabilization of physical parameters), or both. Unless explicitly described herein, methods for assessing the treatment and/or prevention of a disease are generally known in the art.

### BRIEF DESCRIPTION OF THE DRAWINGS

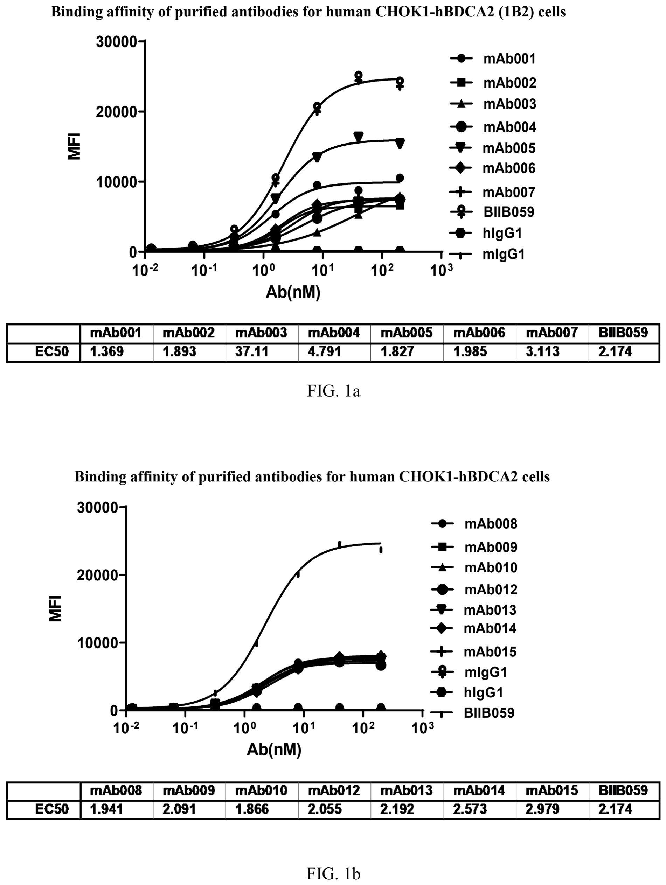

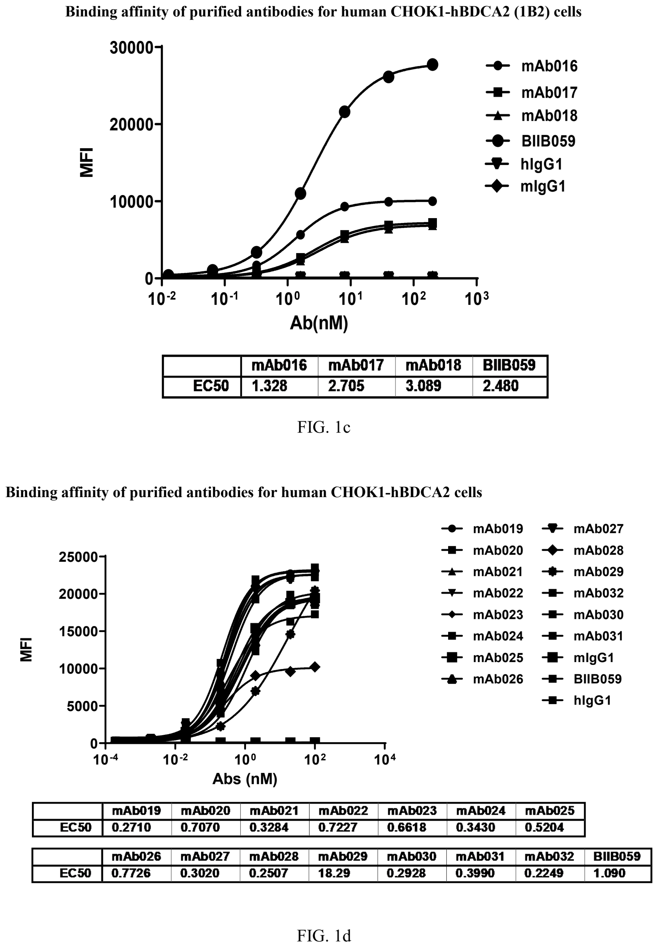

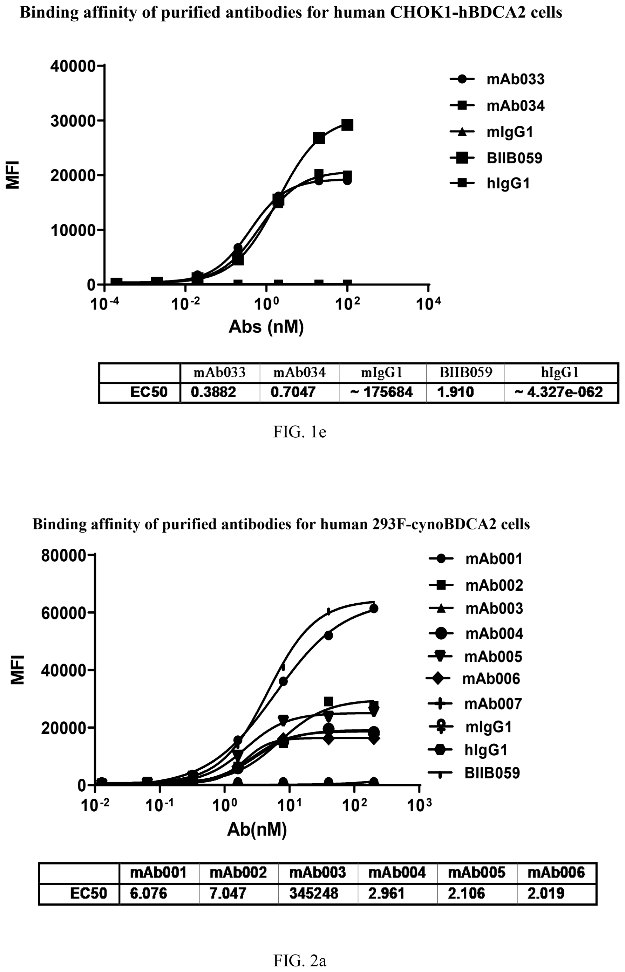

FIGs. 1a-1e show the results of binding assays for hybridoma antibodies to stably transfected human CHOK1-hBDCA2 cells expressing the human BDCA2 molecule by the FACS method, where the antibodies relate to antibodies from different hybridomas, as indicated by the legend.

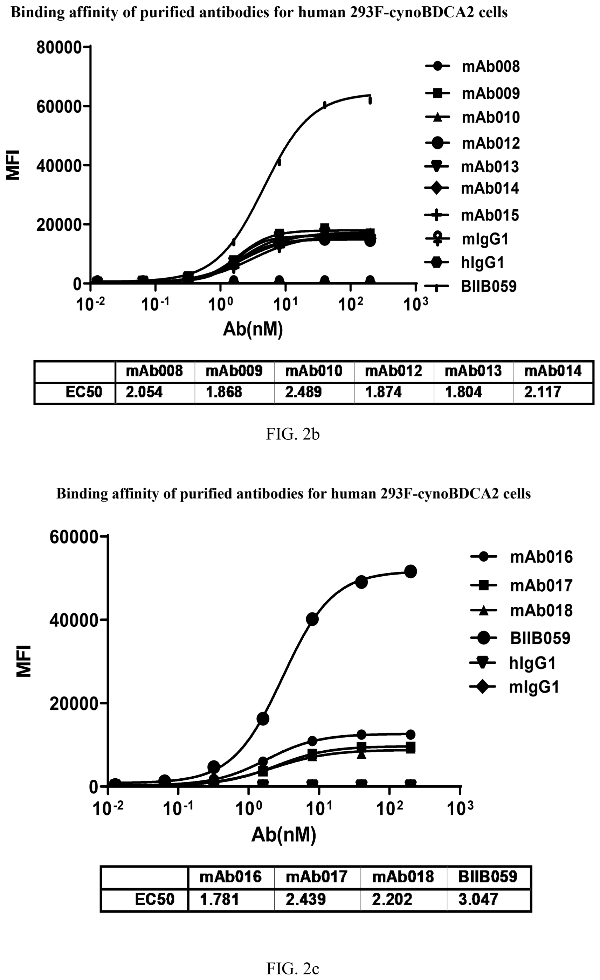

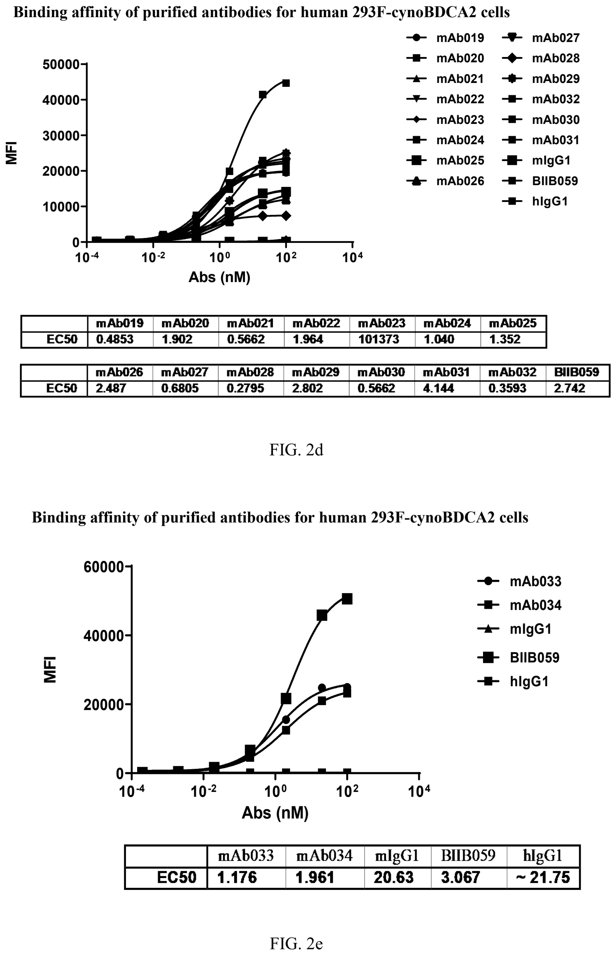

FIGs. 2a-2e show the results of binding assays for hybridoma antibodies to stably transfected human 293F-cynoBDCA2 cells expressing the cynomolgus monkey BDCA2 molecule by the FACS method, where the antibodies relate to antibodies from different hybridomas, as indicated by the legend.

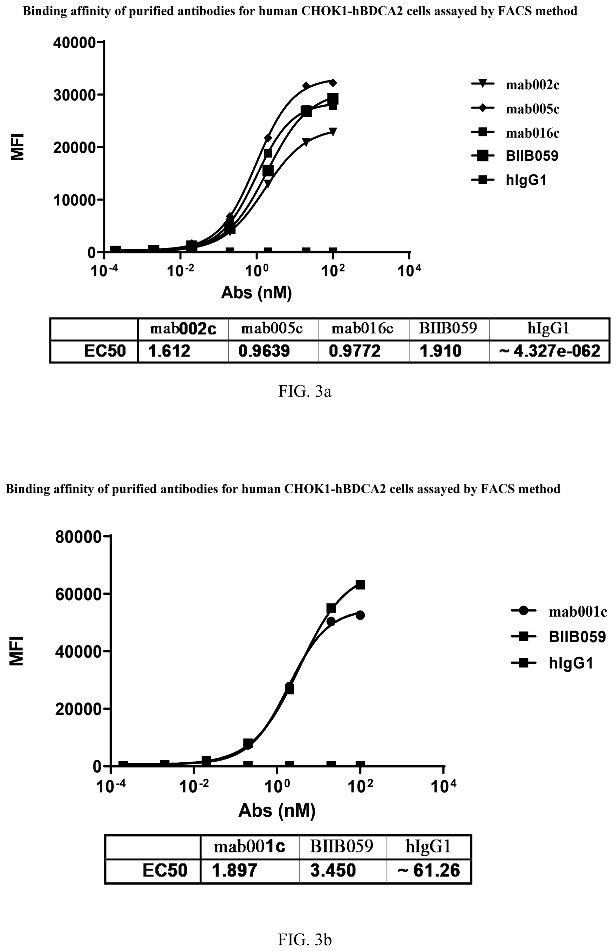

FIGs. 3a-3c show the results of binding assays for anti-BDCA2 chimeric antibodies to human CHOK1-hBDCA2 cells by the FACS method, where the antibodies relate to different chimeric antibodies, as indicated by the legend.

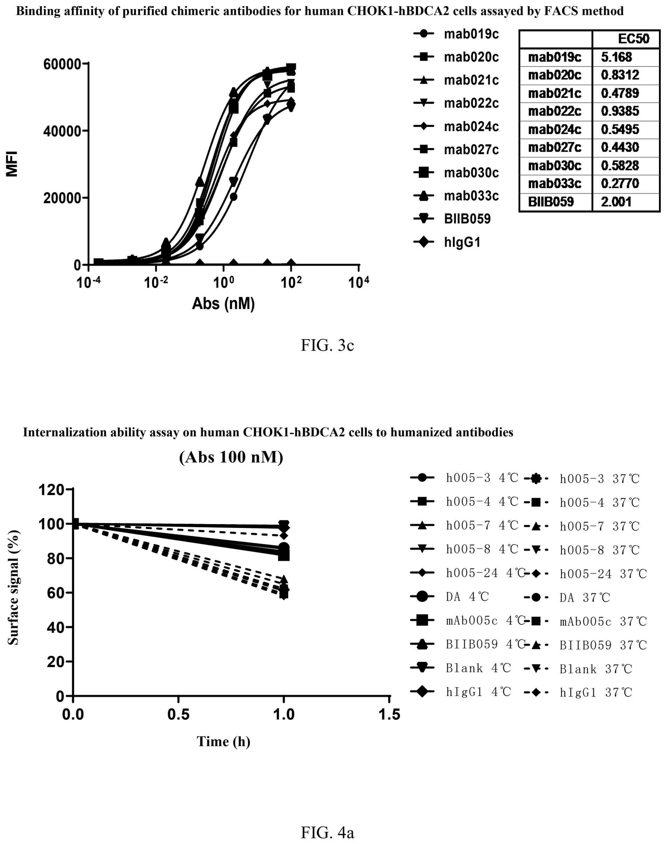

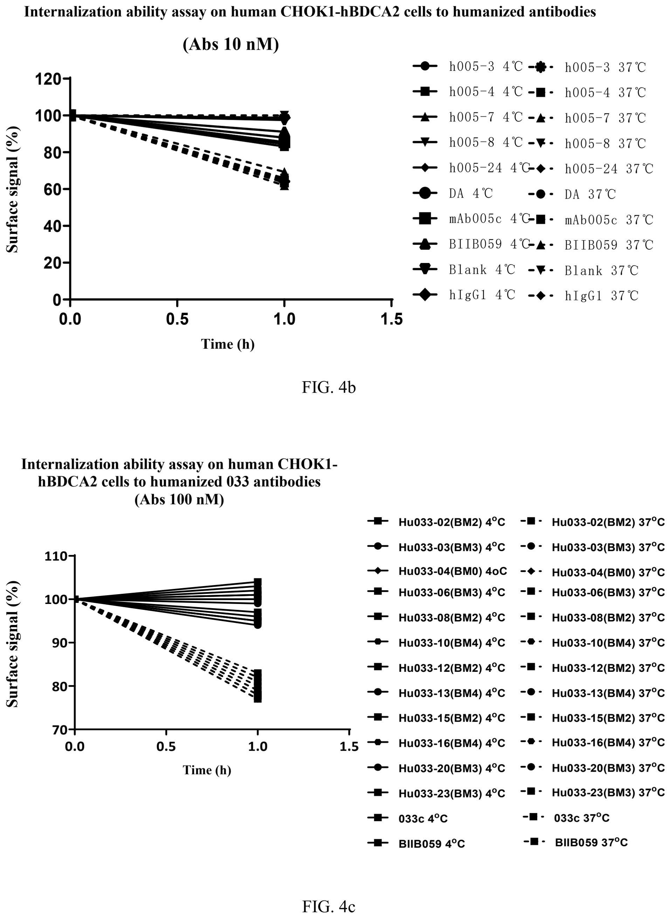

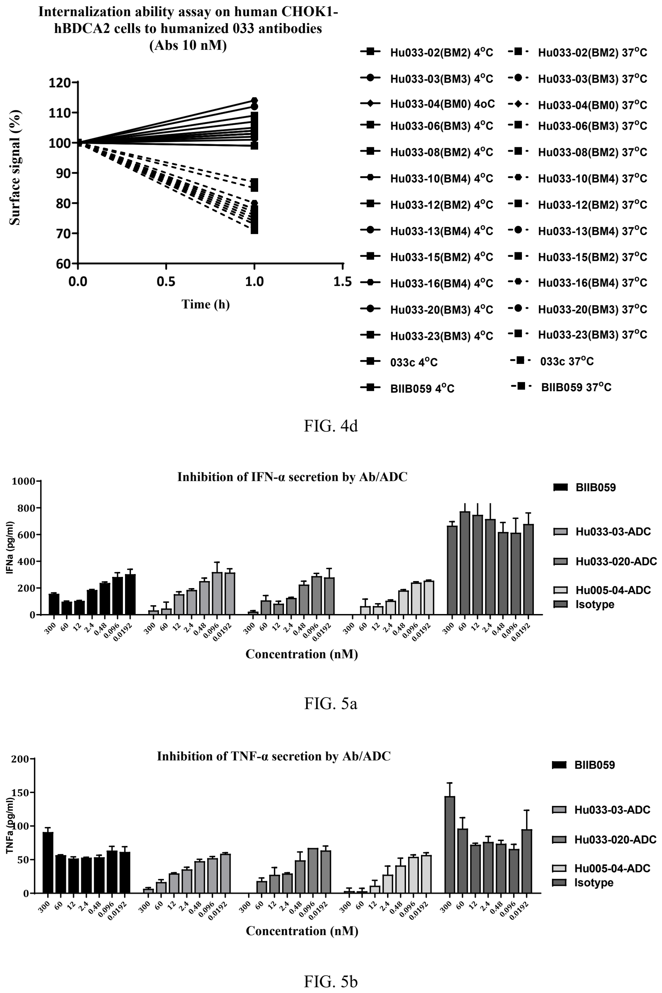

FIGs. 4a-4d show the internalization ability assays for humanized antibodies. FIG. 4a shows the results of humanized antibodies in the hu005 group assayed at a concentration of 100 nM. FIG. 4b shows the results of humanized antibodies in the hu005 group assayed at a concentration of 10 nM. FIG. 4c shows the results of humanized antibodies in the hu033 group assayed at a concentration of 100 nM. FIG. 4d shows the results of humanized antibodies in the hu033 group assayed at a concentration of 10 nM.
FIGs. 5a-5b show the inhibition of CpG-A-induced production of IFN-α and TNF-α from human peripheral blood mononuclear cells by BDCA2-ADC. FIG. 5a shows the inhibition of CpG-A-induced IFN-α from human peripheral blood mononuclear cells by BDCA2-ADC. FIG. 5b shows the inhibition of CpG-A-induced TNF-α from human peripheral blood mononuclear cells by BDCA2-ADC.

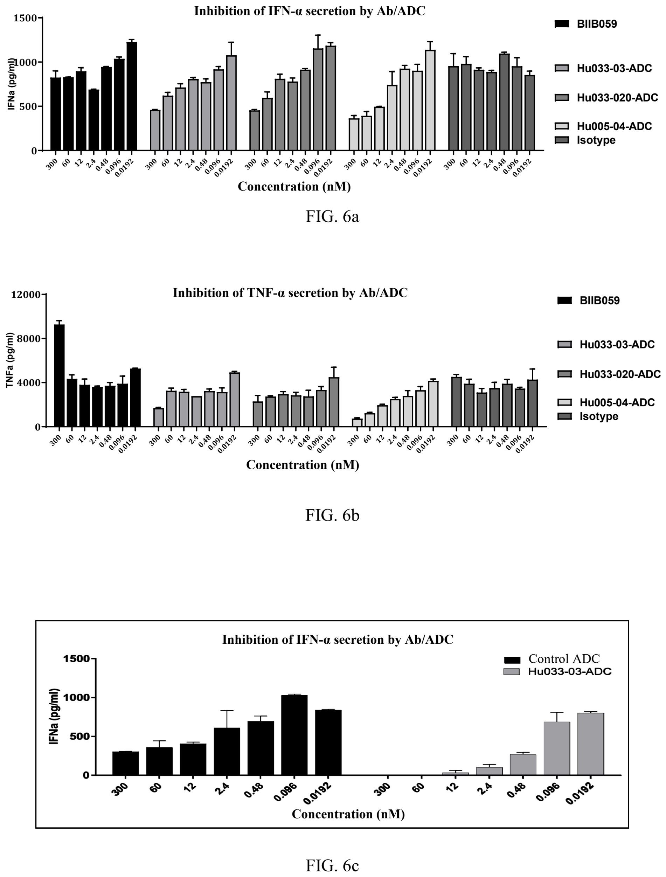

FIGs. 6a-6b show the inhibition of R848-induced production of IFN-α and TNF-α from human peripheral blood mononuclear cells by BDCA2-ADC. FIG. 6a shows the inhibition of R848-induced IFN-α from human peripheral blood mononuclear cells by BDCA2-ADC. FIG. 6b shows the inhibition of R848-induced TNF-α from human peripheral blood mononuclear cells by BDCA2-ADC.

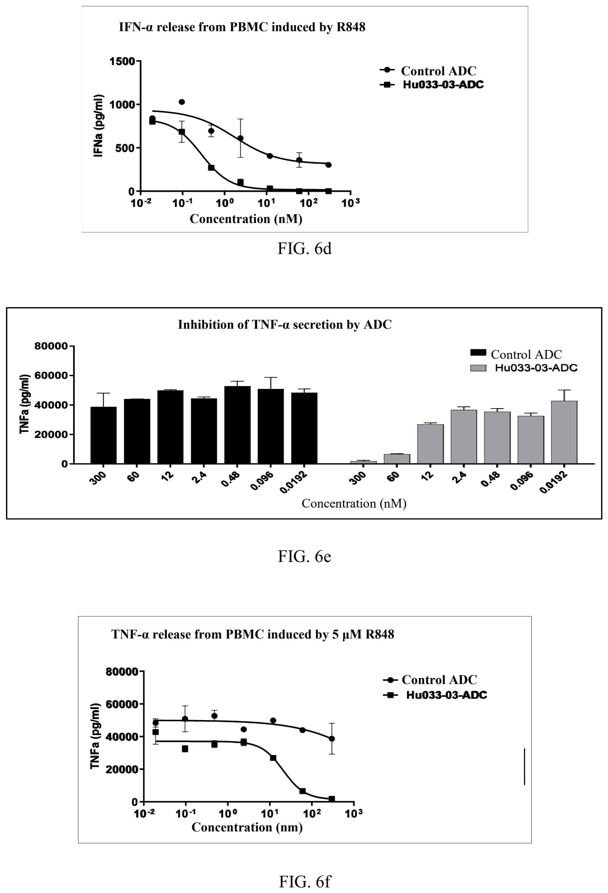

FIGs. 6c-6f show the inhibition of R848-induced production of IFN-α and TNF-α from human peripheral blood mononuclear cells by BDCA2-ADC. FIGs. 6c and 6d show the inhibition of R848-induced IFN-α from human peripheral blood mononuclear cells by BDCA2-ADC. FIGs. 6e and 6f show the inhibition of R848-induced TNF-α from human peripheral blood mononuclear cells by BDCA2-ADC.

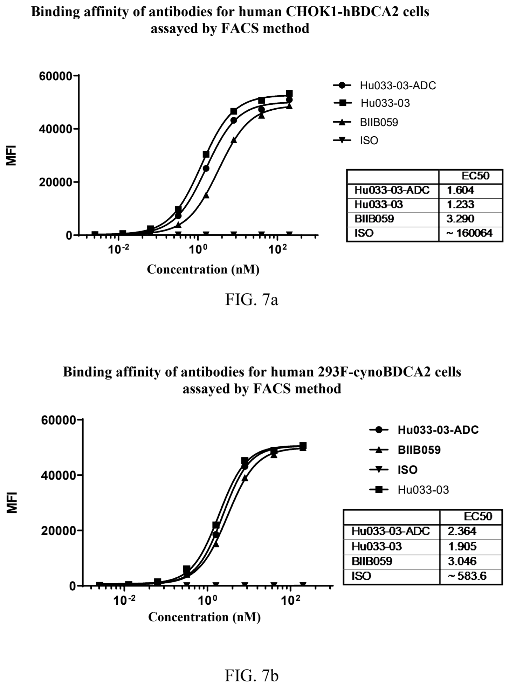

FIGs. 7a-7b show the results of binding assays for Hu003-03-ADC to cells by the FACS method. FIG. 7a shows the result of the binding assay for Hu003-03-ADC to CHOK1-human BDCA2 cells by the FACS method. FIG. 7b shows the result of the binding assay for Hu003-03-ADC to 293F-cynomolgus monkey BDCA2 cells by the FACS method.

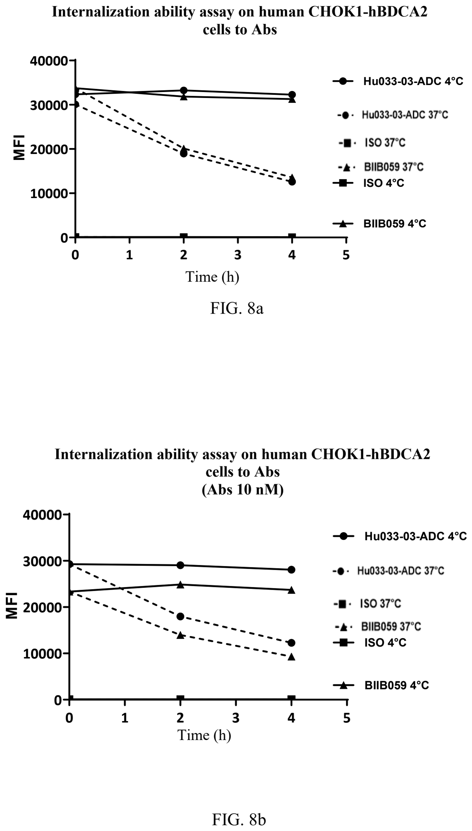

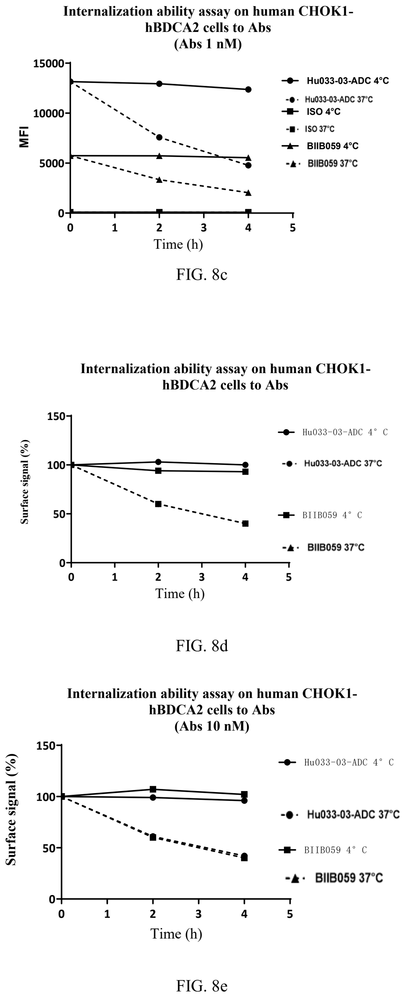

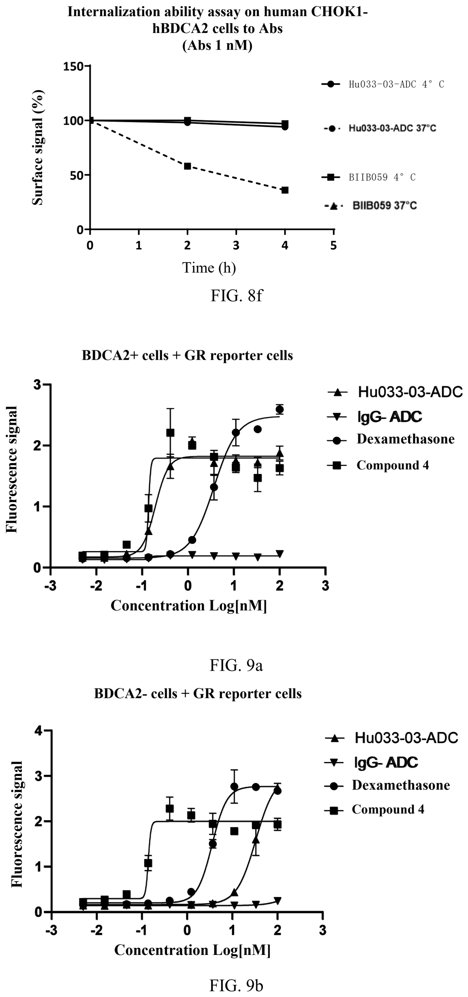

FIGs. 8a-8f show the internalization ability assays for antibody-drug conjugates. FIG. 8a shows the results of Hu003-03-ADC assayed at a concentration of 100 nM. FIG. 8b shows the results of Hu003-03-ADC assayed at a concentration of 10 nM. FIG. 8c shows the results of Hu003-03-ADC assayed at a concentration of 1 nM. FIG. 8d shows the results of Hu003-03-ADC assayed at a concentration of 100 nM. FIG. 8e shows the results of Hu003-03-ADC assayed at a concentration of 10 nM. FIG. 8f shows the results of Hu003-03-ADC assayed at a concentration of 10 nM.
FIGs. 9a-9b show assays for the bystander killing effects of antibody-drug conjugates. FIG. 9a shows the fluorescence signal detection results in HEK293-humanBDCA2 cells and GR reporter gene cells. FIG. 9b shows the fluorescence signal detection results in HEK293 cells not expressing BDCA2 and GR reporter gene cells.

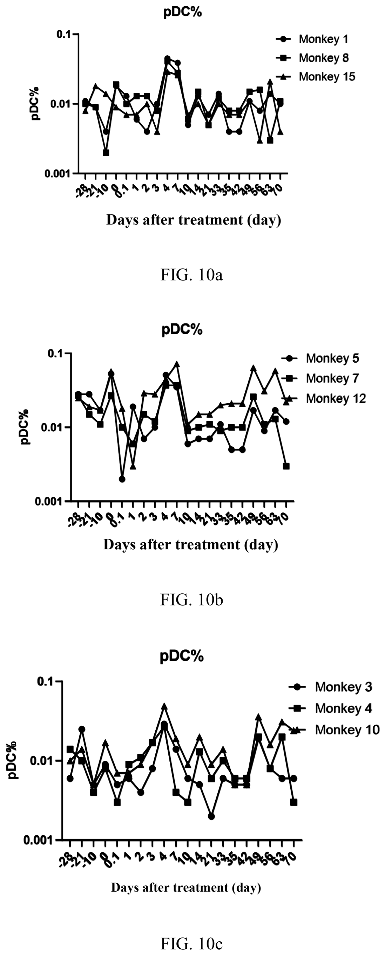

FIGs. 10a-10c show the pDC percentage concentrations in the peripheral blood of cynomolgus monkeys assayed by FACS. FIG. 10a shows the pDC percentage concentrations in the peripheral blood of cynomolgus monkeys in the blank control group. FIG. 10b shows the pDC percentage concentrations in the BDCA2-ADC group. FIG. 10c shows the pDC percentage concentrations in the peripheral blood of cynomolgus monkeys in the BIIB059 group.

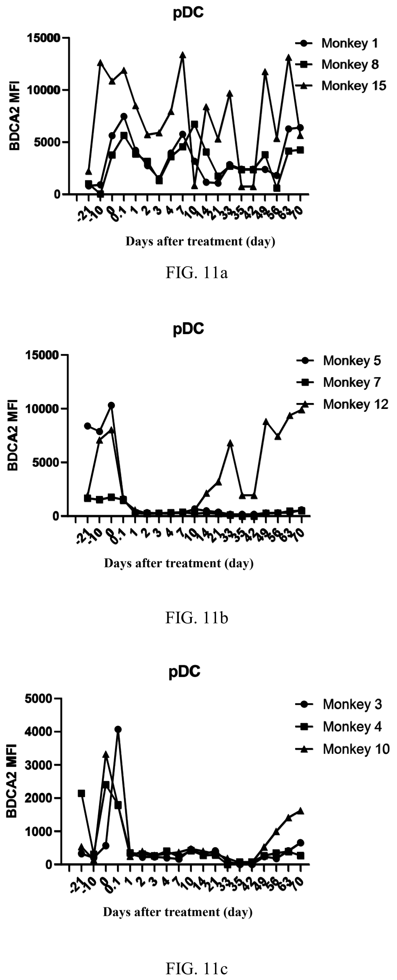

FIGs. 11a-11c show BDCA2 expression levels on the surface of pDCs in the peripheral blood of cynomolgus monkeys assayed by FACS. FIG. 11a shows BDCA2 expression levels on the surface of pDCs in the blank control. FIG. 11b shows BDCA2 expression levels on the surface of pDCs in the BDCA2-ADC group. FIG. 11c shows BDCA2 expression levels on the surface of pDCs in the BIIB059 group.

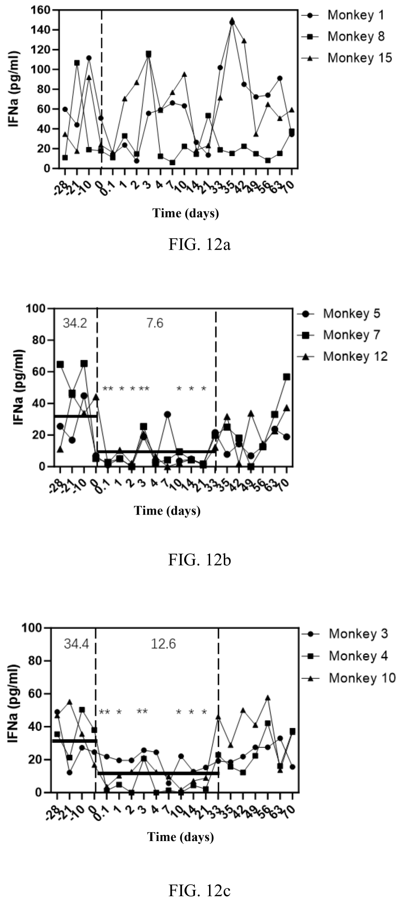

FIGs. 12a-12c the inhibition of CpG-A-induced production of IFN-α from cynomolgus monkey peripheral blood mononuclear cells by BDCA2-ADC or BIIB059. FIG. 12a shows CpG-A-induced IFN-α from cynomolgus monkey peripheral blood mononuclear cells in the blank control group. FIG. 12b shows the inhibition of CpG-A-induced IFN-α from cynomolgus monkey peripheral blood mononuclear cells by BDCA2-ADC. FIG. 12c shows the inhibition of CpG-A-induced IFN-α from cynomolgus monkey peripheral blood mononuclear cells by BIIB059, wherein *: p < 0.05, and **: p < 0.01.

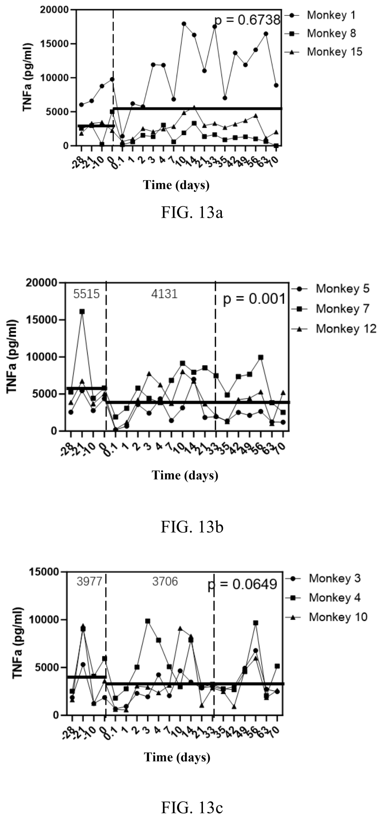

FIGs. 13a-13c the inhibition of CpG-A-induced production of TNF-α from cynomolgus monkey peripheral blood mononuclear cells by BDCA2-ADC or BIIB059. FIG. 13a shows CpG-A-induced TNF-α from cynomolgus monkey peripheral blood mononuclear cells in the blank control group. FIG. 13b shows the inhibition of CpG-A-induced TNF-α from cynomolgus monkey peripheral blood mononuclear cells by BDCA2-ADC. FIG. 13c shows the inhibition of CpG-A-induced TNF-α from cynomolgus monkey peripheral blood mononuclear cells by BIIB059.

### DETAILED DESCRIPTION

The present disclosure includes all combinations of the specific embodiments described. Further embodiments of the present disclosure and the full scope of applicability will become apparent from the detailed description provided below. However, it should be understood that the detailed description and the specific examples, while indicating preferred embodiments of the present disclosure, are provided by way of illustration only, as various changes and modifications within the spirit and scope of the present disclosure will become apparent to those skilled in the art from the detailed description. All publications, patents and patent applications cited herein, including the citations, are hereby incorporated by reference in their entirety for all purposes.

Experimental procedures without specified conditions in the following examples were performed in accordance with conventional procedures and conditions, or in accordance with product instructions.

### Examples

The present disclosure will be described in more detail below by referring to examples and test examples, which are not intended to limit the scope of the present disclosure and may be modified without departing from the scope of the present disclosure.

### Example 1. Anti-BDCA2 Antibodies

In the present application, mice were immunized with BDCA2 antigens to obtain hybridoma cells, and single cell clones meeting the conditions were screened to produce a murine antibody with high affinity for BDCA2. After the obtained murine antibody was sequenced, the immunoglobulin heavy chain and light chain variable regions of hybridoma origin were operably linked to human IgG constant regions to obtain chimeric antibodies, which were further humanized and subjected to removal of post-translational modifications to obtain anti-BDCA2 antibodies Hu033-03, Hu033-20, and Hu005-04.

### Example 1.1. Preparation of Recombinant Proteins for Assay on Activity of Anti-BDCA2 Antibodies

The cDNA sequence encoding the extracellular domain (Asn45-Ile213) of human BDCA2 (hBDCA2) was synthesized by Genscript according to the reference sequence in the Genbank. The amplified fragment was cloned into an eukaryotic expression plasmid system (pCP-EF1a-CMV-FcεRIγ) containing a human Fc fragment (hIgGl) constructed in-house by using a conventional cloning technique, thereby producing a recombinant fusion protein expression plasmid Human BDCA2 Fc (pCP-EF1a-BDCA2-CMV-FcεRIγ). The expression plasmid was expressed in an expression cell 293E and purified by using a size exclusion chromatography column to obtain a Human BDCA2 Fc recombinant protein (with an Fc region located in the N-terminal direction of the BDCA2 extracellular domain, fused via a linker), and this recombinant protein was identified by polyacrylamide gel electrophoresis.

The cDNA sequence encoding the extracellular domain (Asn45-Ile212) of cynomolgus monkey BDCA2 (Cyno BDCA2) was synthesized by Genscript according to the reference sequence in the Genbank, and 6 His expression sequences were added at the N-terminus of the cDNA. The fragment was cloned into an eukaryotic expression plasmid system (pCP-EF1a-CMV-FcεRIγ) constructed in-house by using a conventional cloning technique, thereby producing a recombinant fusion protein expression plasmid Cyno BDCA2 His (pCP-EF1a-Cyno BDCA2-CMV-FcεRIγ). The expression plasmid was expressed in an expression cell 293E and purified by using a size exclusion chromatography column to obtain a Cyno BDCA2 His recombinant protein, and this recombinant protein was identified by polyacrylamide gel electrophoresis.

After the identification, the concentration, total amount, and endotoxin content of the two obtained proteins were suitable for later use.

The amino acid sequence of Human BDCA2 Fc is set forth in SEQ ID NO: 27, and the protein sequence of Human BDCA2 is shown under the NCBI accession No. Q8WTT0; the amino acid sequence of Cyno BDCA2 His is set forth in SEQ ID NO: 28, and the protein sequence of Cyno BDCA2 is shown under the NCBI accession No. A0A2K5UWP4-1. All the sequences were used for the assay on the activity of the antibodies of the present disclosure.

The sequence of the reference antibody BIIB059 specifically binding to BDCA2 was derived from the patent US9902775B2, in which the heavy chain sequence corresponded to the patent sequence No.4 and the light chain sequence corresponded to the patent sequence No.3, and was produced by using a conventional recombinant antibody production process.

### Example 1.2. Construction of BDCA2-Expressing Cell Strain for Assay on Activity of Anti-BDCA2 Antibodies

The cDNA sequence encoding the human BDCA2 was synthesized by Genscript according to the reference sequence in the Genbank. The amplified fragment was cloned into a lentiviral packaging plasmid system (PLvx-EF1a-CMV-hFcεRIγ-IRES-puro) constructed in-house by using a conventional cloning technique, thereby producing a lentiviral packaging plasmid Human BDCA2 (PLvx-EF1a-hBDCA2-CMV-hFcεRIγ-IRES-puro).

The cDNA sequence encoding the cynomolgus monkey Cyno BDCA2 was synthesized by Genscript according to the reference sequence in the Genbank. The amplified fragment was cloned into a lentiviral packaging plasmid system (PLvx-EF1a-CMV-hFcεRIγ-IRES-puro) constructed in-house by using a conventional cloning technique, thereby producing a lentiviral packaging plasmid Cyno BDCA2 (PLvx-EF1a-cynoBDCA2-CMV-cynoFcεRIγ-IRES-puro).

293T cells were used as host cells, and the PLvx-EF1a-hBDCA2-CMV-hFcεRIγ-IRES-puro plasmid or PLvx-EF1a-cynoBDCA2-CMV-cynoFcεRIγ-IRES-puro was transiently transfected into the 293T cells. Cell culture supernatants containing human BDCA2- or Cyno BDCA2-expressing viruses were collected 48 h and 72 h after the transfection and concentrated to 1E8 EU/mL.

CHOK1 cells cultured in advance to the logarithmic growth phase were digested, plated in a 6-well cell culture plate at 1E5 cells/well, and cultured overnight to allow the cells to adhere to the wall. Subsequently, the concentrated human BDCA2 viruses were added to the cells adhered in advance to the wall of the 6-well plate at progressively increasing amounts of infection (100 µL and 200 µL), and the infected cells were screened with a culture medium containing puromycin. The antigen expression level was assayed by flow cytometry, and the cells were subcloned. Flow cytometry was performed using BIIB059 as the reference antibody for assay, and the results show that significant binding to BDCA2 was assayed relative to the control hIgG. Monoclones with high antigen expression levels were selected and subjected to expansion culture to serve as a cell strain for later experiments.

293F cells cultured in advance to the logarithmic growth phase were digested, plated in a 6-well cell culture plate at 1E5 cells/well, and cultured overnight to allow the cells to adhere to the wall. Subsequently, the concentrated Cyno BDCA2 viruses were added to the cells adhered in advance to the wall of the 6-well plate at progressively increasing amounts of infection (100 µL and 200 µL), and the infected cells were screened with a culture medium containing puromycin. The antigen expression level was assayed by flow cytometry, and the cells were subcloned. Flow cytometry was performed using BIIB059 as the reference antibody for assay, and the results show that significant binding to BDCA2 was assayed relative to the control hIgG. Monoclones with high antigen expression levels were selected and subjected to expansion culture to serve as a cell strain for later experiments.

### Example 1.3. Preparation of Mouse Hybridoma Cells

### 1.3.1. Mouse immunization and serum titer assay

### 1.3.1.1. Mouse immunization regimen

A total of 3 groups of mice (5 mice per group) were immunized by 2 immunization routes, with an interval of 14 days between each immunization. In group 1, there were 5 Balb/c mice (purchased from SLAC, female BALB/c, 8 weeks old), and in group 2, there were 5 SJL mice (purchased from SLAC, female BALB/c, 8 weeks old). The mice were immunized 5 times using a combined regimen of a gene gun (pCP-EF1a-BDCA2-CMV-FcεRIγ) with a protein (humanBDCA2 Fc), with the first 4 gene gun immunizations at a dose of 4 µg/time, and the 5th protein immunization at a dose of 25 µg/mouse. In group 3, 5 SJL mice (purchased from SLAC, female, 8 weeks old) were immunized with the pure protein (humanBDCA2 Fc) for a total of 3 immunizations, with a first immunization dose of 50 µg/mouse and the next two immunization doses of 25 µg/mouse. See Table 1.

**Table 1. Mouse immunization regimen**

| **Immunogen** | **Animal grouping** | **Strain** | **Route** | **Dose (µg/mouse)** | **Adjuvant** |
|---|---|---|---|---|---|
| Plasmid + protein mixed | G1 (9731,9732,9733,9734,9735) | Balb/c | Gene gun | 4/4/4/4; 25 | GM-CSF & FLT3L |
| | G2 (9736,9737,9738,9739,9740) | SJL | | | |
| BDCA2-Fc protein | G3 (9946,9947,9948,9949,9950) | SJL | I.P. | 50/25/25 | CFA/IFA /IFA |

### 1.3.1.2. Mouse immune titer assay

Serum samples from the immunized mice were collected at various time points and assayed by ELISA and FACS. According to the results of several assays (taking the last mouse serum assay (TB) result as the main reference factor, the detailed result is not shown), 4 mice were selected, and 2 fusions were performed: for the fusion #F0223, two mice, Balb/c#9735 with the highest fluorescence intensity in group G1 and SJL#9740 with the highest fluorescence intensity in group G2, were selected; for the fusion #F0511, two mice, SJL#9947 and SJL#9948 with the highest fluorescence intensity in group G3, were selected.

### 1.3.2 Cell fusion

On day four after the last booster immunization, the mice were sacrificed, and then spleen cells were collected and ground in normal saline. A suspension rich in lymphocytes was collected and mixed with mouse myeloma cells Sp2/0 using the conventional electrotransfection method. The cells were fused by efficient electrofusion.

The fused cells were diluted in a DMEM culture medium containing HT (hypoxanthine and thymidine), plated in a 96-well plate in proportion, and cultured overnight in an incubator at 37 °C with 5% CO₂. After 24 h, a DMEM culture medium containing 2× HAT (hypoxanthine, amethopterin, and thymidine) was added to screen successfully fused cells (hybridoma cells). The formula of the DMEM complete culture medium was as follows: 15% FBS (fetal bovine serum) + 1:50 L-glutamine + 100 U/mL penicillin-streptomycin + 1:100 OPI (oxaloacetic acid, pyruvic acid, and insulin). The incubator conditions were 8% CO₂ and 37 °C.

### Example 1.4. Screening of Mouse Hybridoma Cells and Purification of Murine Anti-BDCA2 Antibodies

After the fused cells were cultured and screened for about 10 days, the expression of the BDCA2 antibody in the supernatant was assayed by using an Acumen laser cytometer. For the assay, CHOK1-humanBDCA2 was used as a positive cell, CHOK1-blank was used as a negative cell, and the reference antibody BIIB059 was used as a positive control antibody. According to the Acumen results, positive clones were selected and transferred to a 24-well plate for expansion culture. After 3 days of culture, the expanded culture supernatants in the 24-well plate were collected and assayed by the following method: 1) the binding activity to CHOK1-humanBDCA2 cells and 293F-cynoBDCA2 cells was assayed by FACS; 2) the binding activity to humanBDCA2 Fc protein and cynoBDCA2 His protein was assayed by ELISA; and 3) the inhibitory ability (inhibition of IFNα production) was assayed in an experiment in which CpG-A stimulated PBMCs to produce the cytokine IFNα.

Among the polyclonal hybridoma cells obtained by the F0223 fusion, 189 hybridoma cell strains were screened based on Acumen, and the antibodies secreted therefrom could specifically bind to hBDCA2. The 189 binding-positive hybridoma cell strains were further transferred to a 24-well plate for expansion and rescreened. The antibodies expressed by 28 hybridoma cell strains could bind to hBDCA2 and cyno BDCA2 in the ELISA assay and could bind to hBDCA2 and cyno BDCA2 expressed on the cell surface in the FACS, and in the IFNα blocking experiment, the above 28 polyclonal cell strains could inhibit the induction of CpG on the secretion of IFNα from PBMC cells. The 28 hybridoma cell strains were subcloned. Eighteen blocking monoclonal cell strains were obtained by screening subclones and numbered as mAb001-mAb018.

Among the polyclonal hybridoma cells obtained by the F0511 fusion, 168 hybridoma cell strains were screened based on Acumen, and the antibodies secreted therefrom could specifically bind to hBDCA2. The 168 binding-positive hybridoma cell strains were further transferred to a 24-well plate for expansion and rescreened. The antibodies expressed by 35 hybridoma cell strains could bind to hBDCA2 and cyno BDCA2 in the ELISA assay and could bind to hBDCA2 and cyno BDCA2 expressed on the cell surface in the FACS, and in the IFNα blocking experiment, the above 35 polyclonal cell strains could inhibit the induction of CpG on the secretion of IFNα from PBMC cells. The 35 hybridoma cell strains were subcloned. Sixteen blocking monoclonal cell strains were obtained by screening subclones and numbered as mAb019-mAb034.

A total of 34 positive monoclones were finally obtained by 2 fusions and screening. The antibodies secreted therefrom were purified and analyzed, and the concentration, quality, purity, and endotoxin content of the antibodies met the requirements of the subsequent experiments.

### Example 1.5. Performance Determination of Anti-BDCA2 Murine Antibodies

The obtained murine antibodies had good binding activity to humanBDCA2 Fc protein, and the binding capacity EC₅₀ assayed by ELISA was < 0.3 nM. The murine antibodies had relatively good binding ability to cynoBDCA2 His protein, and most antibodies had binding capacity EC₅₀ < 0.5 nM assayed by ELISA.

All murine antibodies bound to CHOK1-humanBDCA2 cells (see FIGs. 1a-1e for results), with EC₅₀ superior or comparable to the control antibody BIIB059. Most murine antibodies bound to 293F-cynoBDCA2 cells (see FIGs. 2a-2e for results), with EC₅₀ superior or comparable to the control antibody BIIB059.

Thirty-two antibodies binding to both CHOK1-humanBDCA2 and 293F-cynoBDCA2 cells were assayed for their ability to inhibit the release of the cytokine IFNα in the experiment in which CpG-A stimulated PBMCs. These antibodies all had certain inhibitory activity. Among them, the IC₅₀ values of mAb005, mAb019, mAb020, mAb021, mAb022, mAb024, mAb027, mAb030, and mAb033 for inhibiting cytokine release were 0.08667 nM, 0.003323 nM, 0.006146 nM, 0.004047 nM, 0.006232 nM, 0.002744 nM, 0.07004 nM, 0.004762 nM, and 0.004083 nM, respectively.

The above experimental procedure is shown below. Among them, mAb001-mAb034 were the murine antibodies of the present disclosure, BIIB059 was the reference antibody described above, hIgG1 was the human IgG1 negative control, and mIgG1 was the mouse IgG1 negative control.

### 1.5.1 Assay on binding activity of murine antibodies to proteins by ELISA

The HumanBDCA2 Fc protein or cynoBDCA2 His protein was diluted to 1 µg/mL with PBS and added to an ELISA plate at 100 µL/well, and the plate was incubated at 4 °C overnight. The plate was blocked with an ELISA blocking solution (PBS phosphate buffer containing 1% BSA, pH 7.4, the percentage being mass percentage) at 37 °C for 2 h. An antibody diluted in a 10-fold gradient was added for a total of 8 concentration points (0.0001 nM-100 nM, including 0 concentration point as a control). The antibody was added at 100 µL/well, and the plate was incubated at 37 °C for 1 h. The plate was washed 3 times, and the anti-mouse IgG(Fab specific)-HRP (Sigma, A3682; 1:5000 dilution) secondary antibody was added at 100 µL/well. The plate was incubated at 37 °C for 1 h. The plate was washed 3 times, and a TMB substrate solution (Huzhou InnoReagents Co., Ltd., EL0009) was added at 100 µL/well. After the plate was incubated at 37 °C for 10 min, 50 µL of 1 N hydrochloric acid was added to terminate the color development reaction. The values at OD450 nm were read on an ELISA plate reader, curves were fitted using GraphPad Prism6, and EC₅₀ values were calculated.

### 1.5.2 Assay on binding activity of murine antibodies to cells by FACS

CHOK1-humanBDCA2 and 293F-cynoBDCA2 cells cultured to the logarithmic growth phase were digested with TrypLE trypsin. The cells were collected and resuspended to 2 × 10^6 cells/mL in an FACS buffer (PBS + 2% FBS). The cells were added to a 3799 cell plate (Corning) at 100 µL/well and centrifuged at 300 g, and the supernatant was discarded. The antibody was serially diluted with the FACS buffer (same as above), and the diluted antibody was added to the centrifuged cells. The cells were resuspended and incubated at 4 °C for 1 h. The incubated cells were centrifuged at 300 g for 5 min and washed twice with the FACS buffer. 100 µL of Donkey anti-Mouse IgG (H+L) Highly Cross-Adsorbed Secondary Antibody-Alexa Fluor 488 (Invitrogen, A21202; 1:1000) secondary antibody was added. The cells were incubated at 4 °C for 1 h, washed twice with the FACS buffer, resuspended in PBS, and analyzed using an FACS (BD FACS CantoTM II) instrument. Curves were fitted using GraphPad Prism6, and EC₅₀ values were calculated.

### 1.5.3 Inhibitory ability of murine antibodies on release of cytokine IFNα from human PBMC cells stimulated by CpG-A

1) The cryopreserved human PBMC cells were thawed and incubated in a complete culture medium (1640 + 10% FBS + 1× NEAA + 1× L-Glutamine) overnight for 18 h. The next day, the PBMC cells were collected and centrifuged at 600 g for 8 min, and the supernatant was discarded. The cells were resuspended to 8 × 10^6 cells/mL in a culture medium (1640 + 10% FBS), and the mixture was added to a 3799 cell culture plate at 100 µL/well. The test antibody was diluted in a 10-fold gradient (with final concentrations of 0.0001 nM-10 nM) with the culture medium (1640 + 10% FBS), and the diluted antibody was added to the PBMCs at 50 µL/well. The cells were resuspended and then incubated at 37 °C for 6 h. 50 µL of CpG-A (2 µM, Invitrogen, tlrl-2216-5) diluted in the culture medium (1640 + 10% FBS) was added to each well and mixed, followed by incubation at 37 °C for another 20 h. On the third day, the cell culture plate was centrifuged at 400 g for 5 min, and the cell culture supernatant was collected and assayed for the cytokine IFNα in the supernatant.
2) Cytokine IFNα assay by ELISA method

The antibody MT1/3/5 (Mabtech, 3425-1H-20) was diluted to 4 µg/mL with PBS, and added to a CORNING ELISA plate at 100 µL/well. The plate was incubated and coated overnight at 4 °C. Subsequently, the coated antibody was discarded, and a blocking solution (PBS + 1% BSA) was added to the plate at 300 µL/well, followed by overnight incubation at 4 °C. The test supernatant was diluted to an appropriate concentration (selected from 1:3 to 1:7 as appropriate) with the blocking solution and added to the coated ELISA plate at 100 µL/well. An IFNα assay standard was diluted in a 2-fold gradient (at the highest concentration of 1000 pg/mL; 8 concentration points; the last concentration point was 0) with the blocking solution, and added to the coated ELISA plate at 100 µL/well. The ELISA plate was incubated in an incubator at 37 °C for 1 h. The plate was washed 3 times, and the assay antibody MT2/4/6 (1:1000) was added at 100 µL/well. The plate was incubated in the incubator at 37 °C for 1 h. The plate was washed 3 times, and the secondary antibody SA-HRP (1:1000) was added at 100 µL/well. The plate was incubated in the incubator at 37 °C for 0.5 h. The plate was washed 3 times, and a TMB substrate solution was added at 100 µL/well. After color development for an appropriate period of time, 1 M HCl was added at 50 µL/well to terminate the reaction. The values at OD450 nm were read on an ELISA plate reader. The inhibition rates were calculated, curves were fitted using GraphPad Prism6, and IC₅₀ values were calculated.

### Example 1.6. Determination of Variable Region Sequences of Anti-BDCA2 Murine Hybridomas (Monoclonal Antibodies) (According to Kabat)

Based on results of the binding ability of murine antibodies to antigens at the protein and cellular levels and the inhibitory ability of the antibodies in the PBMC system against the release of the cytokine IFNα stimulated by CpG-A, 16 hybridoma monoclones were selected for VH/VL sequence determination.

The DNA coding sequences corresponding to the variable regions of the anti-BDCA2 murine antibodies were sequenced by a degenerate primer PCR-based method. Candidate hybridoma cells were cultured and collected by centrifugation at 1000 rpm, and total RNA was extracted with Trizol. A first-strand cDNA was synthesized by using the total RNA as a template, and then the DNA coding sequences corresponding to the variable regions were amplified by PCR by using the first-strand cDNA as a subsequent template. The primer sequences used in the amplification reaction were complementary to the first framework regions of the variable regions and the constant regions of the antibody (Larrick, J.W. et al., 1990, Scand. J. Immunol., 32, 121-128 and Coloma, J.J. et al., (1991) BioTechniques, 11, 152-156). 1 µL of cDNA, 5 µL of 10× PCR buffer, 1 µL (25 pmol) of each of the upstream and downstream primers, 1 µL of dNTP, 1 µL of 25 mmol PL MgCl₂, and 39 µL of H₂O were added to a 50 µL reaction system. The system was pre-denatured at 95 °C for 10 min, and subjected to temperature cycling for PCR amplification after adding 1 µL of Taq enzyme. The reaction conditions were denaturation at 94 °C for 1 min, annealing at 58 °C for 1 min, and extension at 72 °C for 15 s, for 32 cycles in total, followed by incubation at 72 °C for 10 min. PCR products were recovered and purified. The products of the amplification were sequenced to give the amino acid sequences of the heavy chain variable regions and light chain variable regions of the anti-BDCA2 murine antibodies.

The NCBI Ig-Blast (http://www.ncbi.nlm.nih.gov/projects/igblast/) was used to search for consensus sequences in germline and rearranged Ig variable region sequence databases. The amino acid sequences of complementarity-determining regions (CDRs) were identified by sequence annotation and by Internet-based sequence analysis (http://www.imgt.org/IMGT_vquest/vquest and http://www.ncbi.nlm.nih.gov/igblast/), based on Kabat system (Wu, T. T and Kabat, E. A. 1970 J. Exp. Med., 132: 211-250) and IMGT system (Lefranc M.-P. et al., 1999 Nucleic Acids Research, 27, 209-212).

The selected anti-BDCA2 murine antibodies were subjected to multi-sequence alignment and evolutionary tree classification. According to the results, the sequences of the heavy chain variable regions VH in the 16 monoclonal antibodies were mainly divided into 5 categories: 1) mAb020, 022, 026; 2) mAb021, 027, 028, 030, 032, 034; 3) mAb019, 024, 033; 4) mAb001, 002; and 5) mAb005, 016, wherein mAb028 and mAb030 differ in heavy chain variable regions by only 2 amino acids, and mAb028 and mAb032 differ by only 1 amino acid. The light chain variable regions VL could be divided into 3 categories: 1) mAb019; 2) mAb021, 027, 028, 030, 032, 034; and 3) other antibodies, wherein the sequences of the light chain variable regions of mAb028, 030, and 032 are the same.

### Example 1.7. Construction of Anti-BDCA2 Chimeric Antibodies

According to the sequence detection results, 12 monoclonal antibody sequences were selected from the two fusions, and used for the construction of human-mouse chimeric antibodies. The sequence of the hIgG1 Fc segment was selected as a human constant region sequence during plasmid construction. The heavy and light chain variable region coding sequences of the anti-BDCA2 murine antibodies described above were synthesized by Genscript. Expi 293F cells were selected as host cells for antibody expression. The resulting chimeric antibodies were mab001c, mab002c, mab005c, mab016c, mab019c, mab020c, mab021c, mab022c, mab024c, mab027c, mab030c, and mab033c.

Various characterizations were performed on the expressed chimeric antibodies.

### Example 1.8. Screening of Chimeric Antibodies

### 1.8.1 Binding activity of anti-BDCA2 chimeric antibodies to proteins assayed by ELISA

The HumanBDCA2 Fc protein or cynoBDCA2 His protein was diluted to 1 µg/mL with PBS and added to an ELISA plate at 100 µL/well, and the plate was incubated at 4 °C overnight. After the coating was completed, the plate was blocked with an ELISA blocking solution (PBS phosphate buffer containing 1% BSA, pH 7.4, the percentage being mass percentage) at 37 °C for 2 h. An antibody diluted in a 10-fold gradient was added for a total of 8 concentration points (0.0001 nM-100 nM, including 0 concentration point as a control). The antibody was added at 100 µL/well, and the plate was incubated at 37 °C for 1 h. The plate was washed 3 times, and the anti-human IgG (Fab specific)-Peroxidase antibody (Sigma, A0293; 1:5000 dilution) secondary antibody was added at 100 µL/well. The plate was incubated at 37 °C for 1 h. The plate was washed 3 times, and a TMB substrate solution (Huzhou InnoReagents Co., Ltd., EL0009) was added at 100 µL/well. After the plate was incubated at 37 °C for 10 min, 50 µL of 1 N hydrochloric acid was added to terminate the color development reaction. The values at OD450 nm were read on an ELISA plate reader, curves were fitted using GraphPad Prism6, and EC₅₀ values were calculated.

The results are as follows: the test chimeric antibodies bound to the humanBDCA2 Fc protein and the cynoBDCA2 His protein, and the EC₅₀ values for binding to the humanBDCA2-Fc protein were 0.1-0.2 nM; most chimeric antibodies bound to the cynoBDCA2 His protein with an EC₅₀ value of less than 0.6 nM.

### 1.8.2 Binding activity of chimeric antibodies to cells assayed by FACS method

CHOK1-humanBDCA2 and 293F-cynoBDCA2 cells cultured to the logarithmic growth phase were digested with TrypLE trypsin. The cells were collected and resuspended to 2 × 10^6 cells/mL in an FACS buffer (PBS + 2% FBS). The cells were added to a 3799 cell plate at 100 µL/well and centrifuged at 300 g, and the supernatant was discarded. The antibody was serially diluted with the FACS buffer, and the diluted antibody was added to the centrifuged cells. The cells were resuspended and incubated at 4 °C for 1 h. The incubated cells were centrifuged at 300 g for 5 min and washed twice with the FACS buffer. 100 µL of Goat anti-Human IgG (H+L) Cross-Adsorbed Secondary Antibody, Alexa Fluor 488 (Sigma, A11013; 1:1000) secondary antibody was added. The cells were incubated at 4 °C for 1 h, washed twice with the FACS buffer, resuspended in PBS, and analyzed using an FACS (BD FACS CantoTM II) instrument. Curves were fitted using GraphPad Prism6, and EC₅₀ values were calculated.

As shown in FIG. 3 (3a-3c), the chimeric antibodies assayed all bound to CHOK1-humanBDCA2 cells, with EC₅₀ superior or comparable to the control antibody BIIB059. Meanwhile, the chimeric antibodies assayed all bound to 293F-cynoBDCA2 cells with an EC₅₀ value of about 0.3-2 nM, which was superior or comparable to the control antibody BIIB059.

### 1.8.3 Inhibitory ability of chimeric antibodies on release of cytokine IFNα from human PBMC cells stimulated by CpG-A

The cryopreserved human PBMC cells were thawed and incubated in a complete culture medium (1640 + 10% FBS + 1× NEAA + 1× L-Glutamine) overnight for 18 h. The next day, the PBMC cells were collected and centrifuged at 600 g for 8 min, and the supernatant was discarded. The cells were resuspended to 8 × 10^6 cells/mL in a culture medium (1640 + 10% FBS), and the mixture was added to a 3799 cell culture plate at 100 µL/well. The test antibody was diluted in a 10-fold gradient (with final concentrations of 0.0001 nM-10 nM) with the culture medium (1640 + 10% FBS), and the diluted antibody was added to the PBMCs at 50 µL/well. The cells were resuspended and then incubated at 37 °C for 6 h. 50 µL of CpG-A (2 µM, Invivogen, tlrl-2216-5) diluted in the culture medium (1640 + 10% FBS) was added to each well and mixed, followed by incubation at 37 °C for another 20 h. On the third day, the cell culture plate was centrifuged at 400 g for 5 min, and the cell culture supernatant was collected and assayed for the cytokine IFNα in the supernatant.

The antibody MT1/3/5 (Mabtech, 3425-1H-20) was diluted to 4 µg/mL with PBS, and added to a CORNING ELISA plate at 100 µL/well. The plate was incubated overnight at 4 °C. The coated antibody was discarded, and a blocking solution (PBS + 1% BSA) was added to the plate at 300 µL/well, followed by overnight incubation at 4 °C. The test supernatant was diluted to an appropriate concentration (generally 1:3 to 1:7) with the blocking solution and added to the coated ELISA plate at 100 µL/well. An IFNα assay standard was diluted in a 2-fold gradient (at the highest concentration of 1000 pg/mL; 8 concentration points; the last concentration point was 0) with the blocking solution, and added to the coated ELISA plate at 100 µL/well. The ELISA plate was incubated in an incubator at 37 °C for 1 h. The plate was washed 3 times, and the assay antibody MT2/4/6 (1:1000) was added at 100 µL/well. The plate was incubated in the incubator at 37 °C for 1 h. The plate was washed 3 times, and the secondary antibody SA-HRP (1:1000) was added at 100 µL/well. The plate was incubated in the incubator at 37 °C for 0.5 h. The plate was washed 3 times, and a TMB substrate solution was added at 100 µL/well. After color development for an appropriate period of time, 1 M HCl was added at 50 µL/well. The values at OD450 nm were read on an ELISA plate reader. The inhibition rates were calculated, curves were fitted using GraphPad Prism6, and IC₅₀ was calculated.

In the experiment in which CpG-A stimulated PBMCs to release the cytokine, 12 chimeric antibodies assayed all inhibited the secretion of the cytokine IFNα, wherein IC₅₀ values of mab005c, mab019c, mab020c, mab021c, mab022c, mab024c, mab027c, mab030c, and mab033c were 0.008359 nM, 0.01493 nM, 0.01853 nM, 0.01268 nM, 0.03239 nM, 0.01677 nM, 0.008976 nM, 0.01645 nM, and 0.008331 nM, respectively.

### 1.8.4 Assay on internalization ability of chimeric antibodies

CHOK1-humanBDCA2 cells cultured to the logarithmic growth phase were digested with TrypLE trypsin. The cells were collected and resuspended to 2 × 10^6 cells/mL in an FACS buffer (PBS + 2% FBS). The cells were added to a 3799 cell plate at 100 µL/well and centrifuged at 300 g, and the supernatant was discarded. The cells were placed in an ice box and cooled. The antibody was serially diluted with an FACS buffer (3 concentrations were set: 100 nM, 10 nM, and 1 nM; and for each concentration, 3 groups were set: a control at 0 h; 4 °C for 1 h; and 37 °C for 1 h). After the dilution, the antibody was placed in an ice box and cooled for 15 min. After 15 min, the antibody was added to the centrifuged cells, and the cells were resuspended and incubated at 4 °C for 40 min. The incubated cells were centrifuged at 300 g for 5 min and washed twice with the FACS buffer. The cells in the 0 h group were immobilized with paraformaldehyde at room temperature for 10 min, and washed twice with the FACS buffer. The cells in the other two groups were resuspended in 100 µL of the FACS buffer and separately incubated at 4 °C and 37 °C for 1 h. The cells in the two groups of 4 °C for 1 h and 37 °C for 1 h were immobilized with paraformaldehyde at room temperature for 10 min, and washed twice with the FACS buffer. 100 µL of the secondary antibody, Goat anti-Human IgG (H+L) Cross-Adsorbed Secondary Antibody-Alexa Fluor 488 (Sigma, A11013; 1:1000) was separately added to the cells in the 3 groups, and the cells were incubated at 4 °C for 1 h. The cells were washed twice with the FACS buffer, resuspended in PBS and analyzed using the FACS instrument. The surface signal percentages were calculated.

The chimeric antibodies all exhibited internalization at the concentrations of 100 nM, 10 nM, and 1 nM, and the 12 chimeric antibodies had small difference in the internalization mediating ability and were slightly better than the positive control antibody BIIB059. When the antibody concentration was 100 nM, the antibodies exhibited the cell surface signal values of 50%-60% after internalization at 37 °C for 1 h.

### Example 1.9. Humanization Engineering of Antibody Variable Regions

For the humanization engineering of antibody variable regions, human germline IgG genes homologous to the cDNA sequence of the murine antibody were retrieved in the human immunoglobulin gene database of the NCBI (http://www.ncbi.nlm.nih.gov/igblast/), and the amino acid sequences of the variable region CDRs and their precise boundaries were defined by the Kabat numbering system or the IMGT numbering system. In principle, human IGHV with high homology to the murine antibody was selected as a template for humanization and antibody variable regions were humanized by CDR grafting.

mAb005 and mAb033 were selected for humanization based on the sequences of the obtained murine antibodies described above. The humanization engineering comprises the following steps: A. aligning the gene sequence of the murine antibody with the gene sequence of the human germline antibody to find out a sequence with high homology; B. analyzing and investigating HLA-DR affinity, and selecting a human germline framework sequence with low affinity; and C. analyzing the framework amino acid sequences of the variable regions and their periphery by using a computer simulation technology and applying molecular docking to investigate their spatial and stereo combination modes. The key amino acid individuals that may interact with human BDCA2 and maintain the spatial framework in the gene sequence of the murine antibody were analyzed by calculating electrostatic force, Van der Waals' force, hydrophilicity and hydrophobicity, and entropy value, and grafted to the selected human germline gene framework. The amino acid sites of the framework regions that must be retained were mapped. After that, the humanized antibody was synthesized. On this basis, various humanized antibody variable region sequences were obtained, and the designed humanized anti-BDCA2 antibody variable regions were variously combined to obtain various humanized anti-BDCA2 antibodies.

### Example 1.10. Screening of Humanized Anti-BDCA2 Antibodies

### 1.10.1 Binding activity of humanized antibodies to BDCA2

CHOK1-humanBDCA2 and 293F-cynoBDCA2 cells cultured to the logarithmic growth phase were digested with TrypLE trypsin. The cells were collected and resuspended to 2 × 10^6 cells/mL in an FACS buffer (PBS + 2% FBS). The resuspended cells were added to a 3799 cell plate at 100 µL/well and centrifuged at 300 g, and the supernatant was discarded. The test antibody was diluted in a 5-fold gradient (with final concentrations of 0.012 nM-200 nM) with the FACS buffer, and the diluted antibody was added to the centrifuged cells. The cells were resuspended and incubated at 4 °C for 1 h. The incubated cells were centrifuged at 300 g for 5 min and washed twice with the FACS buffer. 100 µL of Goat anti-Human IgG (H+L) Cross-Adsorbed Secondary Antibody, Alexa Fluor 488 (Sigma, A11013; 1:1000) secondary antibody was added. The cells were incubated at 4 °C for 1 h, washed twice with the FACS buffer, and resuspended in PBS. The fluorescence signals of the antibodies for binding to the cell surface were analyzed using an FACS (BD FACS CantoTM II) instrument, curves were fitted using GraphPad Prism6, and EC₅₀ values of the antibodies for binding to the BDCA2 antigen were calculated. The BIIB059 antibody was selected as a positive control, and hIgG1 was selected as a negative control.

The binding activity of the candidate antibodies obtained by the humanization engineering of the mAb033 sequence to CHOK1-humanBDCA2 and 293F-cynoBDCA2 cells was superior to that of the positive control antibody BIIB059. The results are shown in Table 2. The affinity of the antibodies for human BDCA2 was determined by SPR, and the selected antibodies had a stronger affinity for human BDCA2 than that of the positive control antibody BIIB059.

**Table 2. Binding activity of the series of humanized antibodies from mAb033 to BDCA2**

| Antibody No. | CHOK1-hBDCA2 EC₅₀ (nM) | 293F-cynoBDCA2 EC₅₀ (nM) |
|---|---|---|
| BIIB059 | 1.30 | 1.63 |
| hIgG1 | - | - |
| mAb033c | 0.26 | 0.56 |
| Hu033-02(BM2) | 0.44 | 1.20 |
| Hu033-03(BM3) | 0.50 | 1.40 |
| Hu033-04(BM0) | 0.61 | 1.33 |
| Hu033-06(BM3) | 0.62 | 1.41 |
| Hu033-08(BM2) | 0.62 | 1.53 |
| Hu033-10(BM4) | 0.51 | 1.27 |
| Hu033-12(BM2) | 0.45 | 1.07 |
| Hu033-13(BM4) | 0.68 | 1.13 |
| Hu033-15(BM2) | 0.76 | 1.27 |
| Hu033-16(BM4) | 0.63 | 1.37 |
| Hu033-20(BM3) | 0.64 | 1.22 |
| Hu033-23(BM3) | 0.79 | 1.24 |

| | | |
|---|---|---|
| Note: "-" indicates not detected. | | |

The EC₅₀ values for binding of the candidate antibodies obtained by the humanization engineering of the mAb005 sequence to CHOK1-humanBDCA2 cells were superior to that of the positive control antibody BIIB059. The results are shown in Table 3. Meanwhile, the candidate antibodies obtained by the humanization engineering of the mAb005 sequence also had relatively good binding activity to 293F-cynoBDCA2 cells.

**Table 3. Binding activity of the series of humanized antibodies from mAb005 to BDCA2**

| Antibody No. | CHOK1-hBDCA2 EC₅₀ (nM) |
|---|---|
| mAb005c | 2.2 |
| BIIB059 | 3.1 |
| Hu005-03(BM4) | 2.1 |
| Hu005-04(BM6) | 2.3 |
| Hu005-07(BM5) | 2.0 |
| Hu005-08(BM7) | 2.3 |
| Hu005-24(BM6) | 1.9 |

### 1.10.2 Inhibitory ability of humanized antibodies on release of cytokine IFNα from human PBMC cells stimulated by CpG-A

A series of humanized candidate antibody molecules were selected and subjected to the function detection of inhibiting the release of the cytokine IFNα from the human PBMC cells stimulated by CpG-A. The cryopreserved human PBMC cells were thawed and incubated in a complete culture medium (1640 + 10% FBS + 1× NEAA + 1× L-Glutamine) overnight for 18 h. The next day, the PBMC cells were collected and centrifuged at 600 g for 8 min, and the supernatant was discarded. The cells were resuspended to 8 × 10^6 cells/mL in a culture medium (1640 + 10% FBS), and the mixture was added to a 3799 cell culture plate at 100 µL/well. The test antibody was diluted in a 10-fold gradient (0.0001 nM-10 nM, and 0 concentration point was used as a control) with the culture medium (1640 + 10% FBS), and the diluted antibody was added to the PBMCs at 50 µL/well. The cells were resuspended and then incubated at 37 °C for 6 h. 50 µL of CpG-A (2 µM, Invitrogen, tlrl-2216-5) diluted in the culture medium (1640 + 10% FBS) was added to each well and mixed, followed by incubation at 37 °C for another 20 h. On the third day, the cell culture plate was centrifuged at 400 g for 5 min, and the cell culture supernatant was collected and assayed for the cytokine IFNα in the supernatant. The BIIB059 antibody was selected as a positive control, and hIgG1 was selected as a negative control.

The antibody MT1/3/5 (Mabtech, 3425-1H-20) was diluted to 4 µg/mL with PBS, and added to a CORNING ELISA plate at 100 µL/well. The plate was incubated and coated overnight at 4 °C. Subsequently, the coated antibody was discarded, and a blocking solution (PBS + 1% BSA) was added to the plate at 300 µL/well, followed by overnight incubation at 4 °C. After the blocking was completed, the test supernatant was diluted to an appropriate concentration (generally 1:3 to 1:7) with the blocking solution and added to the coated ELISA plate at 100 µL/well. An IFNα assay standard was diluted in a 2-fold gradient (at the highest concentration of 1000 pg/mL; 8 concentration points; the last concentration point was 0) with the blocking solution, and added to the coated ELISA plate at 100 µL/well. The ELISA plate was incubated in an incubator at 37 °C for 1 h. The plate was washed 3 times, and the assay antibody MT2/4/6 (1:1000) was added at 100 µL/well. The plate was incubated in the incubator at 37 °C for 1 h. The plate was washed 3 times, and the secondary antibody SA-HRP (1:1000) was added at 100 µL/well. The plate was incubated in the incubator at 37 °C for 0.5 h. The plate was washed 3 times, and a TMB substrate solution was added at 100 µL/well. After color development for an appropriate period of time, 1 M HCl was added at 50 µL/well. The values at OD450 nm were read on an ELISA plate reader, and curves were fitted using GraphPad Prism6. OD values corresponding to the antibody concentration of 10 nM were selected, and the maximum inhibition rate was calculated as follows: inhibition rate = (OD (IgG1) - OD (Ab))/OD (IgG1). In the experiment in which CpG-A stimulated PBMCs to release the cytokine, the humanized antibodies assayed were able to inhibit the secretion of the cytokine IFNα, with EC₅₀ values less than that of the positive control antibody BIIB059. The results are further shown in Table 4.

**Table 4. Inhibition of humanized antibodies on release of cytokine IFNα from human PBMC cells stimulated by CpG-A**

| Antibody No. | EC₅₀ (nM) |
|---|---|
| BIIB059 | 0.04 |
| hIgG1 | - |
| mAb033c | 0.02 |
| Hu033-02(BM2) | 0.02 |
| Hu033-03(BM3) | 0.01 |
| Hu033-04(BM0) | 0.03 |
| Hu033-06(BM3) | 0.02 |
| Hu033-08(BM2) | 0.02 |
| Hu033-10(BM4) | 0.01 |
| Hu033-12(BM2) | 0.02 |
| Hu033-13(BM4) | 0.01 |
| Hu033-15(BM2) | 0.02 |
| Hu033-16(BM4) | 0.01 |
| Hu033-20(BM3) | 0.01 |
| Hu033-23(BM3) | 0.03 |

| | |
|---|---|
| Note: "-" indicates not detected. | |

### 1.10.3 Assay on internalization ability of humanized antibodies

CHOK1-humanBDCA2 cells cultured to the logarithmic growth phase were digested with TrypLE trypsin. The cells were collected and resuspended to 2 × 10^6 cells/mL in an FACS buffer (PBS + 2% FBS). The cells were added to a 3799 cell plate at 100 µL/well and centrifuged at 300 g, and the supernatant was discarded. The cells were placed in an ice box and cooled. The antibody was serially diluted with an FACS buffer (2 concentrations were set: 100 nM and 10 nM; and for each concentration, 3 groups were set: 0 h; 4 °C for 1 h; and 37 °C for 1 h). After the dilution, the antibody was placed in an ice box and cooled for 15 min. After 15 min, the antibody was added to the centrifuged cells, and the cells were resuspended and incubated at 4 °C for 40 min. The incubated cells were centrifuged at 300 g for 5 min and washed twice with the FACS buffer. The cells in the 0 h group were immobilized with paraformaldehyde at room temperature for 10 min, and washed twice with the FACS buffer. The cells in the other two groups were resuspended in 100 µL of the FACS buffer and separately incubated at 4 °C and 37 °C for 1 h. The cells in the two groups of 4 °C for 1 h and 37 °C for 1 h were immobilized with paraformaldehyde at room temperature for 10 min, and washed twice with the FACS buffer. 100 µL of the secondary antibody, Goat anti-Human IgG (H+L) Cross-Adsorbed Secondary Antibody-Alexa Fluor 488 (Sigma, A11013; 1:1000) was separately added to the cells in the 3 groups, and the cells were incubated at 4 °C for 1 h. The cells were washed twice with the FACS buffer, resuspended in PBS and analyzed using the FACS instrument. The surface signal percentages were calculated.

The results are shown in FIG. 4. Both groups (groups Hu005 and Hu033) of humanized antibodies exhibited internalization at the concentrations of 100 nM and 10 nM, and the ability of the humanized antibodies to mediate internalization was comparable to that of the positive control antibody BIIB059.

### 1.10.4 Affinity assay of humanized antibodies

The humanized antibodies of the present disclosure were assayed for affinity by the BIAcore experiment. The surface of a CM5 chip channel was activated with 50 mM NHS and 200 mM EDC (NHS and EDC from an amino coupling kit) mixed in a ratio of 1:1, and the anti-human IgG (Fc) antibody (diluted with a sodium acetate solution at pH 5.0, the concentration was 25 µg/mL) was injected for 420 s. Then 1 M ethanolamine was injected at a flow rate of 10 µL/min for 420 s to block excess active carboxyl groups on the chip. The humanized antibody was diluted to 1 µg/mL using a running buffer and injected into a detection channel flow cell at a flow rate of 10 µL/min. The sample injection was performed for 60 s for capture. The recombinant Human BDCA2 was diluted to 50 nM or 100 nM with 1× HBS-EP+ (pH 7.4), and diluted in a gradient of 1:2 to 0.39 nM. The test samples hBDCA2 at these concentration gradients were separately injected into the detection channel at a flow rate of 30 µL/min, wherein 2 samples at the concentration of 0 were used for removing background signals. The times for the antibody-antigen binding and dissociation were 180 s and 400 s, respectively. Data analysis was performed using Biacore Insight Evaluation Software (Version 2.0.15.12933). After subtraction of the signals from the reference channel (flow cell 1) and 0 concentration, a 1:1 binding model was selected for curve fitting, and kinetic parameters calculated. The assay results are shown in Table 5 below.

**Table 5**

| Antibody No. | SPR | | |
|---|---|---|---|
| Ab NO. | ka (1/Ms) | kd (1/s) | KD (M) |
| BIIB059 | 1.67E+05 | 1.79E-04 | 1.07E-09 |
| hIgG1 | | | |
| mAb033c | 6.98E+05 | 7.67E-05 | 1.10E-10 |
| Hu033-02(BM2) | 7.07E+05 | 1.69E-04 | 2.39E-10 |
| Hu033-03(BM3) | 5.76E+05 | 1.36E-04 | 2.36E-10 |
| Hu033-04(BM0) | 5.98E+05 | 1.93E-04 | 3.23E-10 |
| Hu033-06(BM3) | 7.33E+05 | 1.25E-04 | 1.70E-10 |
| Hu033-08(BM2) | 7.40E+05 | 1.69E-04 | 2.29E-10 |
| Hu033-10(BM4) | 5.89E+05 | 1.18E-04 | 2.00E-10 |
| Hu033-12(BM2) | 5.27E+05 | 1.49E-04 | 2.83E-10 |
| Hu033-13(BM4) | 4.54E+05 | 1.53E-04 | 3.38E-10 |
| Hu033-15(BM2) | 4.87E+05 | 2.27E-04 | 4.67E-10 |
| Hu033-16(BM4) | 5.69E+05 | 1.08E-04 | 1.91E-10 |
| Hu033-20(BM3) | 5.56E+05 | 1.49E-04 | 2.68E-10 |
| Hu033-23(BM3) | 4.66E+05 | 2.07E-04 | 4.44E-10 |

Reference antibody BIIB059: the light chain amino acid sequence is set forth in SEQ ID NO: 25, and the heavy chain amino acid sequence is set forth in SEQ ID NO: 26, prepared with reference to CN105452295B.

### Example 2. Preparation of Antibody-Drug Conjugates (ADCs)

### 2.1 Preparation of compounds

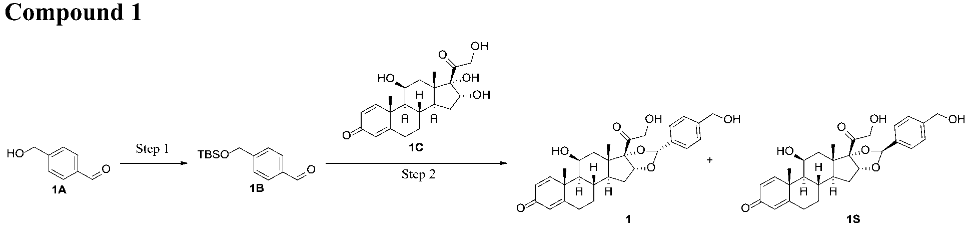

**Step 1** Compound **1A** (500 mg, 3.67 mmol) was dissolved in dichloromethane (10 mL), then DIEA (1.42 g, 10.99 mmol) was added, and TBSCl (830 mg, 5.51 mmol) in dichloromethane (5 mL) was added to the above reaction solution which was then stirred overnight for 17 h. After the reaction was completed as detected by TLC (PE/EA = 20/1), the reaction solution was directly concentrated by rotary evaporation under reduced pressure and subjected to column chromatography (PE:EA = 100:0-100:1) to give a milky-white oily liquid **1B** (800 mg, yield: 87%).

**Step 2** Compound **1B** (125 mg, 0.50 mmol) and Compound **1C** (188 mg, 0.50 mmol) were added to and dissolved in ultra-dry acetonitrile (8 mL). The reaction solution was purged with nitrogen, cooled to 0 °C, mixed with methanesulfonic acid (48 mg, 0.50 mmol) and MeCN (2 mL) added by a syringe, and then stirred overnight for 16 h. After the reaction was completed as detected by TLC (PE/EA = 3/1), sodium bicarbonate was added, and the reaction solution was filtered and concentrated by rotary evaporation to give a crude product, which was then purified by preparative chromatography to give Compound **1** (62 mg, yield: 25%) as a white powder and **1S** (7 mg, yield: 3%) as a white powder.

### Compound 1:

MS-ESI: m/z 495.3 [M+H]⁺.

¹H NMR (400 MHz, DMSO-*d*₆) δ 7.41 (d, *J =* 8.1 Hz, 2H), 7.34-7.28 (m, 3H), 6.16 (dd, *J* = 10.1, 1.9 Hz, 1H), 5.93 (s, 1H), 5.43 (s, 1H), 4.93 (d, *J* = 4.9 Hz, 1H), 4.79 (brs, 1H), 4.52 (d, *J* = 19.5 Hz, 1H), 4.48 (s, 2H), 4.34-4.25 (m, 1H), 4.19 (d, *J =* 19.5 Hz, 1H), 2.61-2.52 (m, 1H), 2.35-2.27 (m, 1H), 2.19-1.98 (m, 2H), 1.84-1.58 (m, 5H), 1.39 (s, 3H), 1.13-0.95 (m, 2H), 0.87 (s, 3H).

### Compound 1S:

MS-ESI: m/z 495.3 [M+H]⁺.

¹H NMR (400 MHz, DMSO-*d*₆) δ 7.35-7.27 (m, 3H), 7.22 (d, *J* = 8.1 Hz, 2H), 6.17 (dd, *J =* 10.1, 1.9 Hz, 1H), 6.09 (s, 1H), 5.95 (s, 1H), 5.30 (d, *J* = 6.8 Hz, 1H), 4.81-4.76 (m, 1H), 4.48 (s, 2H), 4.30 (s, 1H), 4.25 (d, *J =* 19.2 Hz, 1H), 4.01 (d, *J =* 19.2 Hz, 1H), 2.59-2.53 (m, 1H), 2.36-2.28 (m, 1H), 2.12-1.97 (m, 2H), 1.90-1.69 (m, 5H), 1.39 (s, 3H), 1.26-1.13 (m, 1H), 1.06 (dd, *J* = 11.0, 3.4 Hz, 1H), 0.89 (s, 3H).

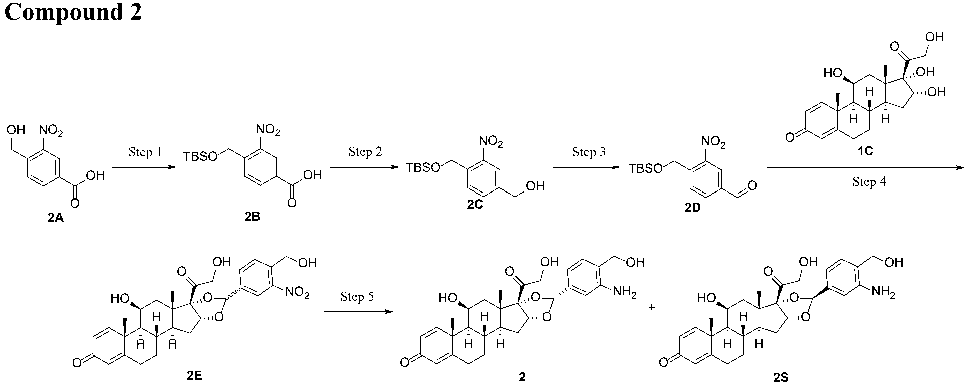

**Step 1** Compound **2A** (5.0 g, 25.4 mmol) and imidazole (2.59 g, 38.0 mmol) were dissolved in dichloromethane (50 mL), then TBSCl (4.9 g, 32.5 mmol) was added, and the reaction solution was reacted at 25 °C for 2 h. After the reaction was completed as detected by TLC, 100 mL of water was added, and the reaction solution was extracted twice with dichloromethane (100 mL × 2). The dichloromethane was combined, dried over anhydrous sodium sulfate, and concentrated by rotary evaporation, and the residue was directly stirred with silica gel and subjected to column chromatography (PE:EA = 20:1) to give a yellow solid **2B** (3 g, yield: 38%). MS-ESI: m/z 312.2 [M+H]⁺.

**Step 2** Compound **2B** (1.0 g, 3.2 mmol) was dissolved in THF (10 mL), and the reaction solution was purged with N₂ and cooled to 0 °C. BH₃/THF (6.4 mL, 6.4 mmol) was added, and the mixture was reacted for 17 h. After the reaction was completed as detected by LCMS, the reaction solution was added to methanol (40 mL) (maintained in an ice bath at 0 °C), quenched with BH₃, and then concentrated by rotary evaporation to give a colorless oil **2C** (500 mg, yield: 52%). MS-ESI: m/z 298.1 [M+H]⁺.

**Step 3** Compound **2C** (500 mg, 1.68 mmol) was dissolved in dichloromethane (8 mL), and the reaction solution was cooled to 0 °C. Dess-Martin reagent (1.43 g, 3.36 mmol) was added, and the mixture was reacted for 1.5 h. After the reaction was completed as detected by LCMS, the reaction solution was diluted with water (100 mL) and extracted twice with ethyl acetate (100 mL + 50 mL), and the organic phases were combined and washed with saturated brine, dried over anhydrous sodium sulfate, and concentrated by rotary evaporation to give a white solid **2D** (250 mg, yield: 50%). MS-ESI: m/z 296.0 [M+H]⁺.

**Step 4** Compound **2D** (100 mg, 0.34 mmol), Compound **1C** (127 mg, 0.34 mmol) and MgSO₄ (250 mg, 2.08 mmol) were dissolved in acetonitrile (10 mL), and the reaction solution was cooled to 0 °C and purged with nitrogen. Trifluoromethanesulfonic acid (150 mg, 1.0 mmol) was added, and the mixture was maintained in an ice bath for 2 h after the addition was completed. After the reaction was completed as detected by LCMS, the reaction solution was directly filtered, and the filter cake was rinsed three times with acetonitrile. The mother solution was concentrated by rotary evaporation and subjected to column chromatography (DCM:MeOH = 30:1) to give a white solid **2E** (100 mg, yield: 55%). MS-ESI: m/z 540.0 [M+H]⁺.

**Step 5** Compound **2E** (100 mg, 0.18 mmol) and iron powder (100 mg, 1.8 mmol) were added to ethanol (4 mL), NH₄Cl (96 mg, 1.8 mmol) was dissolved in water and added to ethanol, and the reaction solution was purged with N₂, heated to 60 °C and reacted for 2 h. After the reaction was completed as detected by LCMS, the reaction solution was filtered through celite, and the filter cake was rinsed three times with ethanol. The mother solution was concentrated by rotary evaporation and subjected to preparative chromatography and lyophilized to give a white solid powder **2** (40 mg, yield: 42%) and a white solid powder **2S**(5 mg, yield: 5%), respectively.

### Compound 2:

MS-ESI: m/z 532.2 [M+Na]⁺.

¹H NMR (400 MHz, DMSO-*d*₆) δ 7.32 (d, *J* = 10.1 Hz, 1H), 7.20 (d, *J* = 7.7 Hz, 1H), 6.94 (s, 1H), 6.89 (d, *J* = 7.7 Hz, 1H), 6.16 (dd, *J* = 10.1, 1.9 Hz, 1H), 5.93 (s, 1H), 5.34 (s, 1H), 4.95-4.89 (m, 1H), 4.80 (brs, 1H), 4.49 (d, *J* = 19.4 Hz, 1H), 4.44 (s, 2H), 4.33-4.27 (m, 1H), 4.18 (d, *J* = 19.4 Hz, 1H), 2.62-2.52 (m, 1H), 2.35-2.28 (m, 1H), 2.19-2.07 (m, 1H), 2.07-1.99 (m, 1H), 1.80-1.60 (m, 5H), 1.40 (s, 3H), 1.15-1.02 (m, 1H), 0.99 (dd, *J =* 11.2, 3.6 Hz, 1H), 0.86 (s, 3H).

### Compound 2S:

MS-ESI: m/z 532.3 [M+Na]⁺.

¹H NMR (400 MHz, DMSO-*d*₆) δ 7.32 (d, *J* = 10.1 Hz, 1H), 7.15-7.09 (m, 1H), 6.70 (s, 1H), 6.63 (d, *J* = 7.6 Hz, 1H), 6.17 (dd, *J* = 10.0, 1.9 Hz, 1H), 5.99 (s, 1H), 5.94 (s, 1H), 5.25 (d, *J* = 6.8 Hz, 1H), 4.78 (brs, 1H), 4.40 (s, 2H), 4.33-4.22 (m, 2H), 4.02 (d, *J =* 19.2 Hz, 1H), 2.60-2.53 (m, 1H), 2.36-2.28 (m, 1H), 2.11-1.97 (m, 2H), 1.90-1.69 (m, 5H), 1.39 (s, 3H), 1.26-1.13 (m, 1H), 1.09-1.02 (m, 1H), 0.88 (s, 3H).

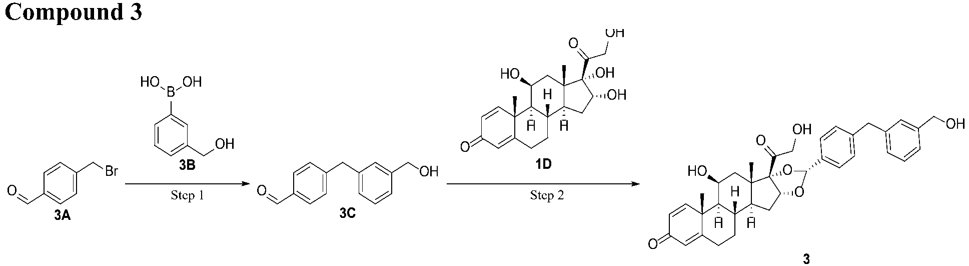

**Step 1** Compound **3A** (6.58 g, 33.08 mmol, 1.0 eq) and Compound **3B** (5.03 g, 33.08 mmol, 1.0 eq) were added into a 250-mL three-necked flask, and THF (50 mL), water (5 mL) and K₂CO₃ (13.71 g, 99.24 mmol, 3.0 eq) were added. Under nitrogen atmosphere, Pd(dppf)Cl₂ (1.21 g, 1.65 mmol, 0.05 eq) was added, and the reaction solution was purged three times with nitrogen, heated to 80 °C to reflux, and then stirred for 3-4 h. After the reaction was completed as detected by TLC (PE/EA = 5/1), the reaction solution was cooled to room temperature and filtered, and the filtrate was poured into 300 mL of water, and extracted with EA (200 mL). The organic phase was washed with water (100 mL × 3), washed once with saturated NaCl, dried over anhydrous Na₂SO₄, and concentrated by rotary evaporation, and the residue was stirred with silica gel and subjected to column chromatography (PE/EA = 7/1) to give a product which was concentrated by rotary evaporation to give a white solid **3C** (2.5 g, yield: 33%).

**Step 2** Compound **1D** (3 g, 7.97 mmol, 1.0 eq), Compound **3C** (1.8 g, 7.97 mmol, 1.0 eq) and MgSO₄ (2.88 g, 23.91 mmol, 3.0 eq) were added into a 100-mL three-necked flask, acetonitrile (30 mL) was added. Under nitrogen atmosphere, trifluoromethanesulfonic acid (1.2 g, 7.97 mmol, 1.0 eq) was added in an ice-water bath, and the reaction solution was heated to room temperature and reacted for 2 h after the addition was completed. After the reaction was completed as detected by TLC (PE/EA = 3/1), the reaction solution was filtered, the filtrate was concentrated by rotary evaporation, and the residue was stirred with silica gel and subjected to column chromatography (PE/EA = 2/1) to give a product which was concentrated by rotary evaporation to give a white solid 3 (3.7 g, yield: 79%).

MS-ESI: m/z 585.3 [M+H]⁺.

¹H NMR (400 MHz, DMSO-*d*₆) δ 7.36 (d, *J =* 7.8 Hz, 2H), 7.30 (d, *J* = 10.1 Hz, 1H), 7.24 (d, *J* = 7.7 Hz, 2H), 7.20 (d, *J =* 7.5 Hz, 1H), 7.15 (s, 1H), 7.11 (d, *J* = 7.6 Hz, 1H), 7.07 (d, *J =* 7.6 Hz, 1H), 6.16 (dd, *J =* 10.1, 1.9 Hz, 1H), 5.92 (s, 1H), 5.39 (s, 1H), 4.91 (d, *J* = 4.8 Hz, 1H), 4.78 (brs, 1H), 4.49 (d, *J =* 19.4 Hz, 1H), 4.43 (s, 2H), 4.32-4.25 (m, 1H), 4.17 (d, *J =* 19.5 Hz, 1H), 3.90 (s, 2H), 2.59-2.51 (m, 1H), 2.35-2.26 (m, 1H), 2.18-1.95 (m, 2H), 1.82-1.56 (m, 5H), 1.39 (s, 3H), 1.11-0.96 (m, 4H), 0.85 (s, 3H).

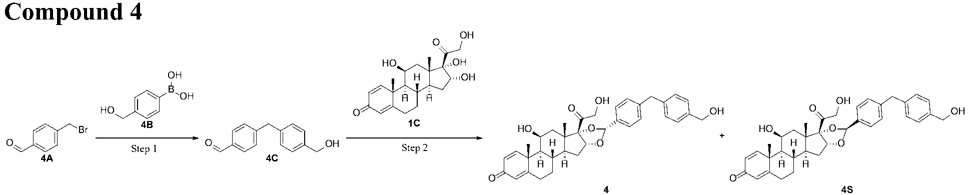

**Step 1** Compound **4A** (500 mg, 2.51 mmol) was dissolved in THF/H₂O (10/2 mL), and then Compound **4B** (573 mg, 3.77 mmol) and K₂CO₃ (1.04 g, 7.53 mmol) were added. The reaction solution was purged with nitrogen, and then Pd(dppf)Cl₂ (367 mg, 0.50 mmol) was added. The reaction solution was heated to 80 °C under nitrogen atmosphere and reacted for 2 h. The reaction solution was cooled to room temperature, diluted with water (20 mL), and extracted with ethyl acetate (20 mL × 3), the organic phases were combined and washed with saturated brine (10 mL), dried over anhydrous sodium sulfate, and concentrated, and the residue was subjected to column chromatography (PE/EA = 1:0 to 3:1) to give a yellow solid **4C** (270 mg, yield: 48%).

**Step 2** Compound **4C** (150 mg, 0.66 mmol) was dissolved in MeCN (10 mL), then Compound **1C** (250 mg, 0.66 mmol) and MgSO₄ (160 mg, 1.32 mmol) were added, and trifluoromethanesulfonic acid (299 mg, 1.99 mmol) was added at 0 °C under nitrogen atmosphere. The reaction solution was reacted at 0 °C for 1 h, diluted with water (20 mL), and extracted with dichloromethane (10 mL × 3), and the organic phases were combined and washed with brine (10 mL), dried, and then concentrated by rotary evaporation to give a crude product which was then subjected to preparative chromatography to give a white powdery solid **4** (120 mg, yield: 31%) and a white powdery solid **4S** (13 mg, yield: 3%), respectively.

### Compound 4:

MS-ESI: m/z 585.4 [M+H]⁺.

¹H NMR (400 MHz, DMSO-*d*₆) δ 7.35 (d, *J =* 8.0 Hz, 2H), 7.30 (d, *J =* 10.1 Hz, 1H), 7.24-7.16 (m, 4H), 7.14 (d, *J* = 8.1 Hz, 2H), 6.16 (dd, *J* = 10.1, 1.9 Hz, 1H), 5.92 (s, 1H), 5.38 (s, 1H), 4.90 (d, *J* = 4.8 Hz, 1H), 4.48 (d, *J* = 19.4 Hz, 1H), 4.42 (s, 2H), 4.31-4.25 (m, 1H), 4.16 (d, *J* = 19.4 Hz, 1H), 3.89 (s, 2H), 2.59-2.52 (m, 1H), 2.36-2.25 (m, 1H), 2.18-1.96 (m, 2H), 1.82-1.55 (m, 5H), 1.38 (s, 3H), 1.10-0.94 (m, 2H), 0.85 (s, 3H).

### Compound 4S:

MS-ESI: m/z 585.3 [M+H]⁺.

¹H NMR (400 MHz, Chloroform-*d*) δ 7.31-7.26 (m, 3H), 7.21-7.11 (m, 6H), 6.31 (dd, *J* = 10.1, 1.9 Hz, 1H), 6.08 (s, 2H), 5.43-5.38 (m, 1H), 4.65 (s, 2H), 4.52-4.45 (m, 1H), 4.27 (d, *J* = 19.9 Hz, 1H), 4.05 (d, *J* = 19.9 Hz, 1H), 3.95 (s, 2H), 2.66-2.54 (m, 1H), 2.23-2.07 (m, 3H), 1.95-1.83 (m, 2H), 1.80-1.68 (m, 1H), 1.63 (dd, *J* =14.1, 2.6 Hz, 1H), 1.46 (s, 3H), 1.31-1.13 (m, 2H), 0.99 (s, 3H).

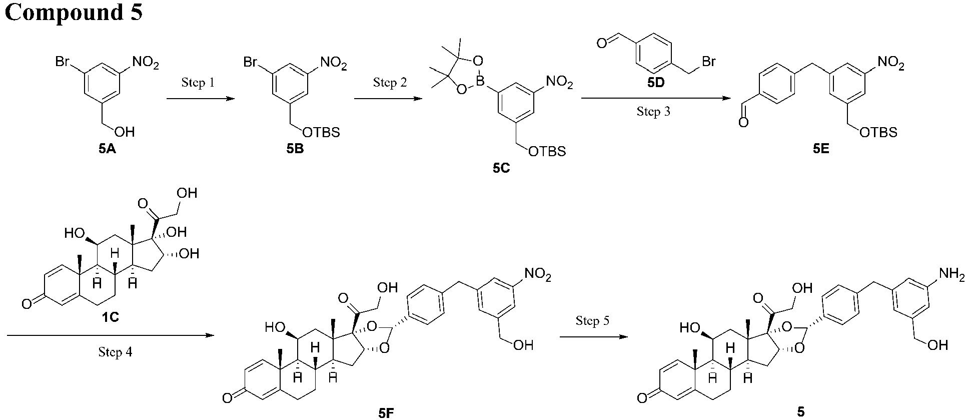

**Step 1** Compound **5A** (50 g, 216 mmol, 1.0 eq) and imidazole (22 g, 323 mmol, 1.5 eq) were added into a 2-L three-necked flask, the reaction mixture was dissolved in dichloromethane (700 mL), and then TBSCl (44 g, 292 mmol, 1.35 eq) was slowly added. After the starting material was consumed completely as detected by TLC (PE/EA = 9/1), the reaction solution was cooled to room temperature, filtered, and extracted twice with dichloromethane (100 mL) and H₂O (90 mL). The organic phase was dried over anhydrous sodium sulfate, concentrated by rotary evaporation, stirred with silica gel and purified by normal phase column chromatography (PE:EA = 10:1) to give a white solid **5B** (65 g, yield: 87%).

**Step 2** Compound **5B** (60 g, 173 mmol, 1.0 eq), bis(pinacolato)diboron (44 g, 173 mmol, 1.0 eq), Pd(dppf)Cl₂ (6.3 g, 8.6 mmol, 0.05 eq) and potassium acetate (51 g, 520 mmol, 3.0 eq) were added into a 2-L three-necked flask, and the reaction solution was dissolved in dioxane (1 L). Under N₂ atmosphere, the reaction solution was stirred at 110 °C for 12 h. After the reaction was completed as detected by TLC (PE:EA = 9:1), the reaction solution was cooled to room temperature, filtered, and extracted twice with ethyl acetate (1 L) and H₂O (1 L), and the organic phase was dried over anhydrous sodium sulfate, concentrated by rotary evaporation, stirred with silica gel and purified by normal phase column chromatography (PE:EA = 10:1) to give a white solid **5C** (60 g, yield: 88%).

**Step 3** Compound **5C** (30 g, 76 mmol, 1.0 eq), Compound **5D** (15.2 g, 76 mmol, 1 eq) and Pd(dppf)Cl₂ (3 g, 4 mmol, 0.05 eq) were added into a 1-L three-necked flask, then dioxane (600 mL) was added, and Cs₂CO₃ (74 g, 228 mmol, 3.0 eq) was dissolved in water (60 mL). Under nitrogen atmosphere, the reaction solution was reacted at 110 °C for 2 h. After the reaction was completed as detected by TLC (PE:EA = 9:1), the reaction solution was cooled to room temperature, filtered, and extracted twice with ethyl acetate (1 L) and H₂O (1 L). The organic phase was dried over anhydrous sodium sulfate, concentrated by rotary evaporation, stirred with silica gel and purified by normal phase column chromatography (PE:EA = 10:1) to give a white solid **5E** (20 g, yield: 68%).

**Step 4** Compound **5E** (2.0 g, 5.2 mmol, 1.0 eq), Compound **1C** (1.96 g, 5.2 mmol, 1.0 eq) and MgSO₄ (1.88 g, 15.6 mmol, 3.0 eq) were added into a 250-mL three-necked flask, and acetonitrile (50 mL) was added. The reaction solution was cooled to -20 °C with dry ice and ethanol, trifluoromethanesulfonic acid (2.34 g, 15.6 mmol, 3.0 eq) was slowly added, and the resulting mixture was stirred at 0 °C for 2 h. After the reaction was completed as detected by TLC (PE:EA = 1:1), sodium bicarbonate solution was added to adjust the pH to 7-8, acetonitrile was removed by rotary evaporation, and the reaction solution was extracted and slurried by PE:EA = 3:1 to give a crude product of Compound **5F** (3.2 g, yield: 98%) which was directly used in the next step. MS-ESI: m/z 630.1 [M+H]⁺.

**Step 5** Compound **5F** (3.2 g, 5.08 mmol, 1 eq) and Fe (2.84 g, 50.8 mmol, 10 eq) were added into a 250-mL three-necked flask, ethanol (80 mL) was added, NH₄Cl (2.72 g, 50.8 mmol, 10 eq) was dissolved in water (80 mL) and then added into the three-necked flask, and the reaction solution was reacted at 60 °C for 2 h. After the reaction was completed as detected by TLC (DCM:MOH = 10:1), ethanol was removed by evaporation under reduced pressure, and aqueous sodium bicarbonate was added to adjust the pH to 8. The reaction solution was extracted twice with 80 mL of dichloromethane and water, and the organic phase was dried over magnesium sulfate, filtered, and concentrated by rotary evaporation to give a yellow solid (2.6 g) as a crude product, 400 mg of which was subjected to reversed-phase column chromatography to give Compound **5** (43.9 mg, purity: 95% or more).

MS-ESI: m/z 600.1 [M+H]⁺.

¹H NMR (400 MHz, DMSO-*d*₆) δ 7.38 (d, *J* = 8.0 Hz, 2H), 7.31 (d, *J* = 10.1 Hz, 1H), 7.24 (d, *J* = 7.9 Hz, 2H), 6.96 (s, 1H), 6.90 (s, 1H), 6.76 (s, 1H), 6.16 (dd, *J* = 10.1, 1.8 Hz, 1H), 5.93 (s, 1H), 5.40 (s, 1H), 4.92 (d, *J* = 4.8 Hz, 1H), 4.79 (brs, 1H), 4.49 (d, *J* = 19.4 Hz, 1H), 4.43 (s, 2H), 4.32-4.26 (m, 1H), 4.17 (d, *J =* 19.5 Hz, 1H), 3.90 (s, 2H), 2.62-2.52 (m, 1H), 2.36-2.26 (m, 1H), 2.18-1.97 (m, 2H), 1.81-1.55 (m, 5H), 1.39 (s, 3H), 1.10-0.94 (m, 2H), 0.86 (s, 3H).

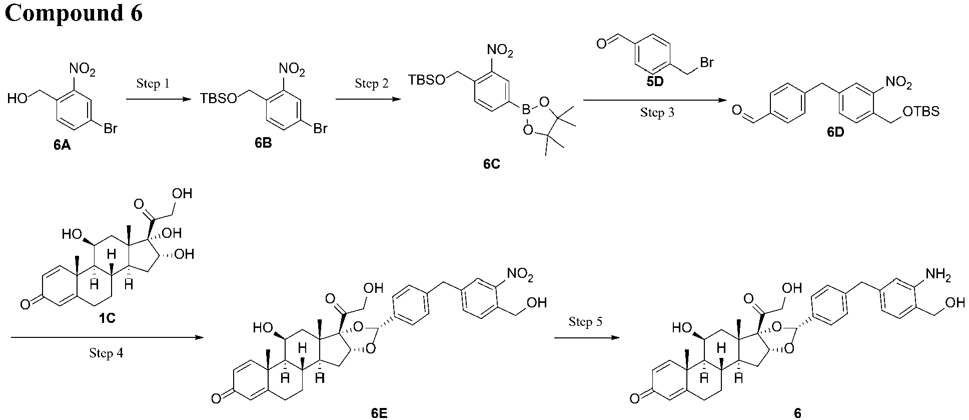

**Step 1** Compound **6A** (10 g, 43.1 mmol) was dissolved in dichloromethane (100 mL), then DIEA (16.7 g, 129.3 mmol) was added, and TBSCl (9.7 g, 64.6 mmol) in dichloromethane (100 mL) was added to the above reaction solution. The resulting mixture was stirred overnight for 3 d. After the reaction was completed as detected by TLC (PE/EA = 40/1), the reaction solution was directly concentrated by rotary evaporation under reduced pressure and subjected to column chromatography with pure petroleum ether to give a light yellow oily liquid **6B** (12.0 g, yield: 80%).

**Step 2** Compound **6B** (1 g, 2.89 mmol), bis(pinacolato)diboron (883 mg, 3.48 mmol), KOAc (853 mg, 8.69 mmol), 1,4-dioxane (10 mL) and Pd(dppf)Cl₂ (212 mg, 0.29 mmol) were added to 1,4-dioxane (10 mL), and the reaction solution was purged with nitrogen, heated (110 °C) and stirred overnight for 17 h. After the reaction was completed as detected by TLC (PE:EA = 40:1), the reaction solution was directly concentrated by rotary evaporation, and the residue was directly stirred with silica gel and subjected to column chromatography (petroleum ether:ethyl acetate = 50:1) to give a light yellow oily liquid **6C** (183 mg, yield: 16%). MS-ESI: m/z 394.3 [M+H]⁺.

**Step 3** Compound **6C** (180 mg, 0.46 mmol), Pd(dppf)Cl₂ (33.5 mg, 0.046 mmol), Compound **5D** (90.6 mg, 0.46 mg), Cs₂CO₃ (298 mg, 0.92 mmol), 1,4-dioxane (8 mL) and water (2 mL) were added, and the reaction solution was purged with N₂, heated (110 °C) and stirred overnight (20 h). After the reaction was completed as detected by TLC (PE/EA = 10/1), the reaction solution was directly concentrated by rotary evaporation, and the residue was directly stirred with silica gel and subjected to column chromatography (petroleum ether:ethyl acetate = 100:1) to give a light yellow oily liquid **6D** (95.8 mg, yield: 54%). MS-ESI: m/z 386.1 [M+H]⁺. **Step 4** Compound **6D** (500 mg, 1.30 mmol), Compound **1C** (488 mg, 1.30 mmol), MeCN (10 mL) and MgSO₄ (930 mg, 7.73 mmol) were added into a three-necked flask, and the reaction solution was purged with N₂ and cooled to 0 °C. Trifluoromethanesulfonic acid (585 mg, 3.90 mmol) was added by a constant pressure dropping funnel, and the resulting mixture was stirred (3 h). After the reaction was completed as detected by TLC (DCM/MeOH = 10/1), the reaction solution was directly concentrated by rotary evaporation, and the residue was directly stirred with silica gel and subjected to column chromatography (dichloromethane:MeOH = 50:1) to give a white solid **6E** (300 mg, yield: 37%); MS-ESI: m/z 630.1 [M+H]⁺.

**Step 5** Compound **6E** (4 g, 6.35 mmol) and ethanol (100 mL) were added into a single-necked flask, iron powder (2.36 g, 42.1 mmol) and NH₄Cl (3.4 g, 63.6 mmol) were dissolved in water (40 mL) and then added into the above single-necked flask, and the reaction solution was purged with N₂, heated to 60 °C and stirred (2 h). After the reaction was completed as detected by TLC (dichloromethane/CH₃OH = 10/1), NaHCO₃ aqueous solution was added to adjust the pH to 7-8, the reaction solution was stirred for 10 min and filtered, and the filtrate was concentrated by rotary evaporation. Dichloromethane was added for dissolution, and the resulting mixture was stirred and subjected to column chromatography (dichloromethane:methanol = 30:1) to give a white powdery solid **6** (3.5 g, yield: 92%), 50 mg of which was taken to be subjected to preparative thin layer chromatography and filtered, and the filtrate was concentrated by rotary evaporation. Acetonitrile and water (purified water) were added, and the resulting mixture was lyophilized to give the product **6** (38 mg, purity: 90.08%).

MS-ESI: m/z 622.3 [M+Na]⁺.

¹H NMR (400 MHz, DMSO-*d*₆) δ 7.35 (d, *J =* 8.0 Hz, 2H), 7.30 (d, *J* = 10.2 Hz, 1H), 7.22-7.16 (m, 2H), 6.93 (d, *J =* 7.6 Hz, 1H), 6.45-6.40 (m, 1H), 6.40-6.34 (m, 1H), 6.16 (dd, *J* = 10.1, 1.9 Hz, 1H), 5.93 (s, 1H), 5.39 (s, 1H), 5.14-5.04 (m, 1H), 4.98-4.83 (m, 3H), 4.78 (d, *J* = 3.4 Hz, 1H), 4.54-4.45 (m, 1H), 4.34-4.26 (m, 3H), 4.22-4.12 (m, 1H), 3.75 (s, 2H), 2.60-2.53 (m, 1H), 2.36-2.26 (m, 1H), 2.17-1.96 (m, 2H), 1.81-1.60 (m, 5H), 1.39 (s, 3H), 1.09-0.97 (m, 2H), 0.85 (s, 3H).

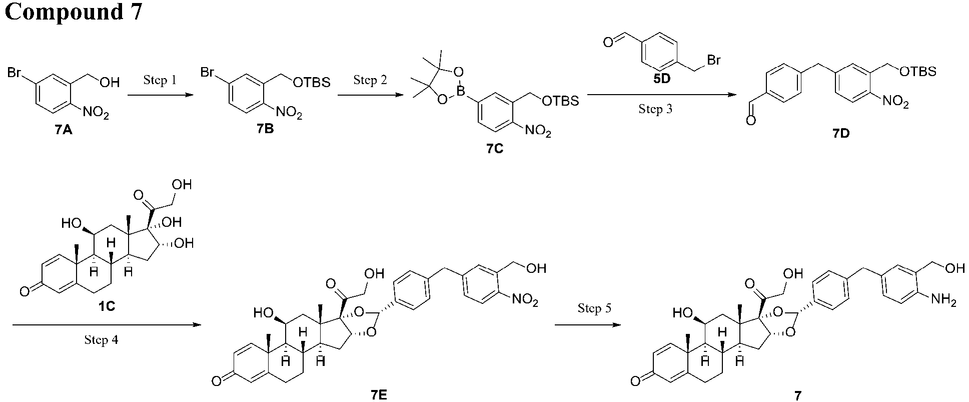

**Step 1** Compound **7A** (65 g, 280 mmol, 1.0 eq) and imidazole (29 g, 420 mmol, 1.5 eq) were added into a 2-L three-necked flask, the reaction mixture was dissolved in dichloromethane (700 mL), and then TBSCl (59 g, 392 mmol, 1.4 eq) was slowly added. After the starting material was consumed completely as detected by TLC (PE/EA = 9/1), the reaction solution was cooled to room temperature, filtered, and extracted twice with dichloromethane (100 mL) and H₂O (90 mL). The organic phase was dried over anhydrous sodium sulfate, concentrated by rotary evaporation, stirred with silica gel and purified by normal phase column chromatography (PE:EA = 10:1) to give a white solid **7B** (80 g, yield: 82%).

**Step 2** Compound **7B** (70 g, 203 mmol, 1.0 eq), bis(pinacolato)diboron (51 g, 203 mmol, 1.0 eq), Pd(dppf)Cl₂ (7.4 g, 10.1 mmol, 0.05 eq) and potassium acetate (60 g, 609 mmol, 3.0 eq) were added into a 2-L three-necked flask, and the mixture was dissolved in dioxane (1 L). Under N₂ atmosphere, the reaction solution was stirred at 110 °C for 12 h. After the reaction was completed as detected by TLC (PE:EA = 10:1), the reaction solution was cooled to room temperature, filtered, and extracted twice with ethyl acetate (1 L) and H₂O (1 L), and the organic phase was dried over anhydrous sodium sulfate, concentrated by rotary evaporation, stirred with silica gel and purified by normal phase column chromatography (PE:EA = 10:1) to give a white solid **7C** (60 g, yield: 75%).

**Step 3** Compound **7C** (15 g, 38 mmol, 1.0 eq), Compound **5D** (7.6 g, 38 mmol, 1 eq) and Pd(dppf)Cl₂ (1.4 g, 1.9 mmol, 0.05 eq) were added into a 1-L three-necked flask, then dioxane (600 mL) was added, Cs₂CO₃ (37 g, 114 mmol, 3.0 eq) was dissolved in water (50 mL), and under nitrogen atmosphere, the reaction solution was reacted at 110 °C for 2 h. After the reaction was completed as detected by TLC (PE:EA = 10:1), the reaction solution was cooled to room temperature, filtered, and extracted twice with ethyl acetate (1 L) and H₂O (1 L). The organic phase was dried over anhydrous sodium sulfate, concentrated by rotary evaporation, stirred with silica gel and purified by normal phase column chromatography (PE:EA = 10:1) to give a white solid **7D** (10 g, yield: 68%).

**Step 4** Compound **7D** (1 g, 2.6 mmol, 1.0 eq), Compound **1C** (0.98 g, 2.6 mmol, 1.0 eq) and MgSO₄ (0.94 g, 7.8 mmol, 3.0 eq) were added into a 250-mL three-necked flask, and acetonitrile (50 mL) was added. The resulting mixture was cooled to -20 °C with dry ice and ethanol, trifluoromethanesulfonic acid (1.17 g, 7.8 mmol, 3.0 eq) was slowly added, and the resulting mixture was stirred at 0 °C for 2 h. After the reaction was completed as detected by TLC (dichloromethane:MeOH = 10:1), sodium bicarbonate solution was added to adjust the pH to 7-8, acetonitrile was removed by rotary evaporation, and the reaction solution was extracted and slurried by PE:EA = 3:1 to give Compound **7E** (1.4 g, yield: 86%); MS-ESI: m/z 630.1 [M+H]⁺.

**Step 5** Compound **7E** (200 mg, 0.317 mmol, 1 eq) and iron powder (177 mg, 3.17 mmol, 10 eq) were added into a 25-mL three-necked flask, ethanol (6 mL) was added, NH₄Cl (170 mg, 3.17 mmol, 10 eq) was dissolved in water (6 mL) and then added into the three-necked flask, and the reaction solution was reacted at 60 °C for 2 h. After the reaction was completed as detected by TLC (DCM:MeOH = 10:1), ethanol was removed by evaporation under reduced pressure, and aqueous sodium bicarbonate was added to adjust the pH to 8. The reaction solution was extracted twice with 80 mL of dichloromethane and water, and the organic phase was dried over magnesium sulfate, filtered, and concentrated by rotary evaporation to give a yellow solid (200 mg) which was subjected to reversed-phase column chromatography to give Compound **7** (42.6 mg, yield: 22%).

MS-ESI: m/z 600.8 [M+H]⁺.

¹H NMR (400 MHz, DMSO-*d*₆) δ 7.36 (d, *J* = 8.0 Hz, 2H), 7.31 (d, *J* = 10.1 Hz, 1H), 7.22 (d, *J* = 7.9 Hz, 2H), 7.20-7.14 (m, 1H), 7.07 (d, *J* = 8.0 Hz, 1H), 6.98 (d, *J* = 8.0 Hz, 1H), 6.16 (dd, *J* = 10.1, 1.9 Hz, 1H), 5.93 (s, 1H), 5.39 (s, 1H), 4.91 (d, *J* = 4.7 Hz, 1H), 4.79 (s, 1H), 4.54-4.45 (m, 3H), 4.31-4.26 (m, 1H), 4.17 (d, *J* = 19.5 Hz, 1H), 3.87 (s, 2H), 2.60-2.53 (m, 1H), 2.36-2.26 (m, 1H), 2.18-1.96 (m, 2H), 1.81-1.54 (m, 5H), 1.39 (s, 3H), 1.12-0.94 (m, 2H), 0.86 (s, 3H).

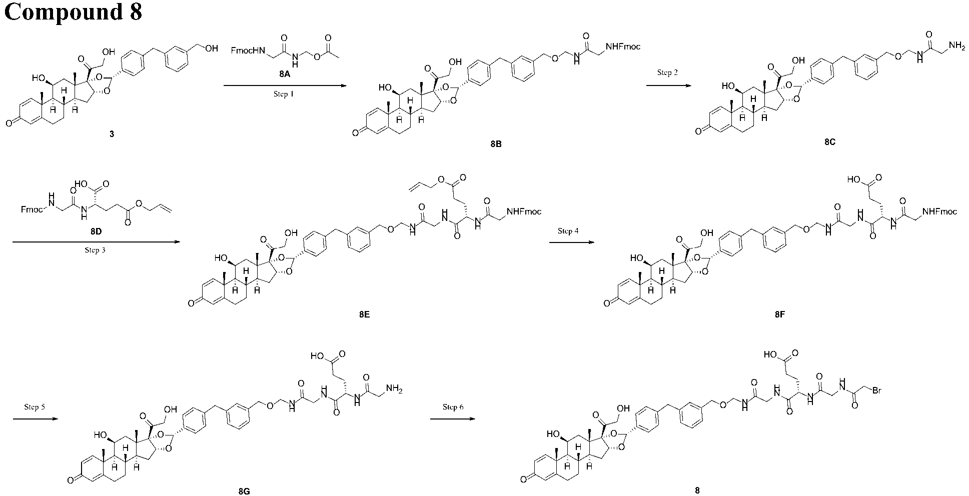

**Step 1** Compound 3 (2 g, 3.42 mmol, 1.0 eq) and Compound **8A** (2.52 g, 6.84 mmol, 2.0 eq) were added into a reaction flask, and the reaction mixture was dissolved in THF (30 mL). Under nitrogen atmosphere, TsOH (0.63 g, 3.66 mmol, 1.07 eq) was added in an ice-water bath, and the resulting mixture was reacted at 5-10 °C for 30-40 min. After the reaction was completed as detected by TLC (PE/EA = 1/2), the reaction solution was added to water, the product was extracted with EA (100 mL), the organic phase was washed water (50 mL × 3), dried over anhydrous sodium sulfate and concentrated, and the residue was stirred with silica gel and subjected to column chromatography (PE/EA = 2/1) to give the product which was concentrated by rotary evaporation to give a white solid **8B** (2.3 g, yield: 75%). MS-ESI: m/z 893.4 [M+H]⁺.

**Step 2** Compound **8B** (2 g, 2.24 mmol) was dissolved in DCM (20 mL), DEA (6 mL) was added dropwise in an ice-water bath under nitrogen atmosphere, and then the resulting mixture was reacted at room temperature for 2 h after the addition was completed. After the reaction was completed as detected by TLC (PE/EA = 1/2), the reaction solution was concentrated by rotary evaporation, extracted three times with DCM, subjected to reversed-phase column chromatography and lyophilized to give an off-white solid **8C** (600 mg, yield: 40%). MS-ESI: m/z 671.3 [M+H]⁺.

**Step 3** Compound **8C** (300 mg, 0.447 mmol, 1.0 eq) and Compound **8D** (230 mg, 0.492 mmol, 1.1 eq) were added into a reaction flask, and the reaction solution was dissolved in DMF (3.5 mL). DIEA (173 mg, 1.341 mmol, 3.0 eq) was added in an ice-water bath under nitrogen atmosphere, a solution of HATU (221 mg, 0.581 mmol, 1.3 eq) in 1 mL of DMF was added dropwise, and then the resulting mixture was stirred at 0-5 °C for 30 min after the addition was completed. After the reaction was completed as detected by LC-MS, the reaction solution was poured into ice water, EA (100 mL) was added, and the product was extracted. The organic phase was washed with water (50 mL × 3), dried over anhydrous sodium sulfate, and concentrated by rotary evaporation to give a yellow solid **8E** (430 mg, yield: 86%). MS-ESI: m/z 1119.4 [M+H]⁺.

**Step 4** Under nitrogen atmosphere, Compound **8E** (300 mg, 0.268 mmol, 1 eq), Pd(PPh₃)₄ (62 mg, 0.0536 mmol, 0.2 eq) and N-methylmorpholine (452 mg, 4.47 mmol, 16.7 eq) were added into a reaction flask, and the reaction mixture was reacted at room temperature for 1 h under nitrogen atmosphere. After the reaction was completed as detected by TLC, the reaction solution was directly purified by reversed-phase column chromatography to give a white solid **8F** (210 mg, yield: 72%). MS-ESI: m/z 1079.3 [M+H]⁺.

**Step 5** Under nitrogen atmosphere, Compound **8F** (200 mg, 0.185 mmol) was dissolved in DCM (2.4 mL), DEA (0.8 mL) was added dropwise in an ice-water bath, and the resulting mixture was heated to room temperature for 2 h after the addition was completed. After the reaction was completed as detected by LCMS, the reaction solution was directly extracted and washed three times with PE (60 mL × 3), the product precipitated and stuck to the wall of the bottle, and after acetonitrile (3 mL) was dissolved, the reaction solution was purified by reversed-phase column chromatography to give a white solid **8G** (62 mg, yield: 39%). MS-ESI: m/z 857.6 [M+H]⁺.

**Step** 6 Compound **8G** (60 mg, 0.07 mmol, 1.0 eq) was dissolved in THF (3 mL), the reaction solution was dissolved in water (0.6 mL), TEA (414 mg, 4.1 mmol, 58 eq) was added in an ice-water bath, and the reaction mixture was stirred for 1 min. Bromoacetyl bromide (113 mg, 0.56 mmol, 8.0 eq) was added, and the resulting mixture was stirred at 0-5 °C for 5 min. After the reaction was completed as detected by LC-MS, the reaction solution was stored with dry ice and ethanol, subjected to preparative chromatography and lyophilized to give a white solid **8** (11 mg, yield: 16%).

MS-ESI: m/z 977.3 [M+H]⁺.

¹H NMR (400 MHz, DMSO-*d*₆) δ 8.62 (t, *J* = 6.6 Hz, 1H), 8.47 (t, *J* = 5.6 Hz, 1H), 8.24 (t, *J=* 5.9 Hz, 1H), 8.18 (d, *J* = 7.7 Hz, 1H), 7.40-7.34 (m, 2H), 7.31 (d, *J* = 10.1 Hz, 1H), 7.27-7.20 (m, 3H), 7.16 (brs, 1H), 7.14-7.07 (m, 2H), 6.16 (dd, *J* = 10.1, 1.9 Hz, 1H), 5.93 (s, 1H), 5.39 (s, 1H), 4.91 (d, *J* = 5.1 Hz, 1H), 4.77 (brs, 1H), 4.65-4.55 (m, 2H), 4.49 (d, *J* = 19.4 Hz, 1H), 4.39 (s, 2H), 4.33-4.25 (m, 2H), 4.17 (d, *J* = 19.5 Hz, 1H), 3.94-3.89 (m, 4H), 3.80 (d, *J* = 5.6 Hz, 2H), 3.72 (d, *J* = 5.8 Hz, 2H), 2.57-2.52 (m, 2H), 2.30-2.24 (m, 2H), 2.18-2.06 (m, 1H), 2.06-1.89 (m, 2H), 1.83-1.59 (m, 6H), 1.39 (s, 3H), 1.12-0.97 (m, 2H), 0.85 (s, 3H).

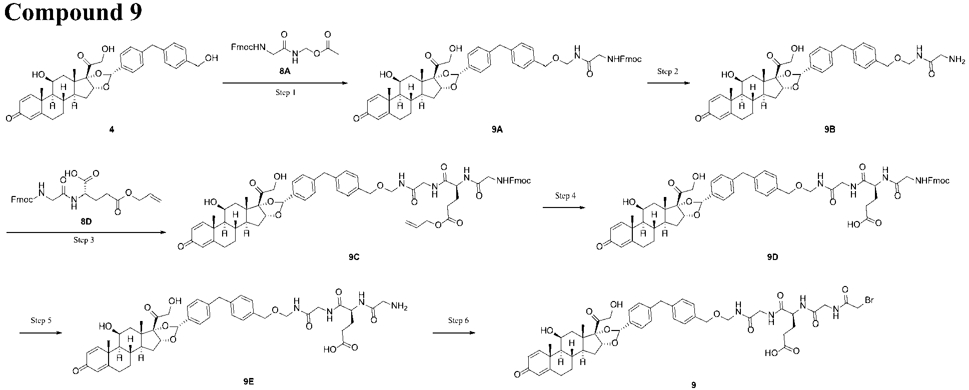

**Step 1** Compound **4** (2.4 g, 4.1 mmol, 1.0 eq) and Compound **8A** (4.5 g, 12.3 mmol, 3.0 eq) were added into a reaction flask, and the reaction mixture was dissolved in THF (30 mL). Under nitrogen atmosphere, TsOH (1.1 g, 6.6 mmol, 1.6 eq) was added in an ice-water bath, and the resulting mixture was reacted at 5-10 °C for 30-40 min. After the reaction was completed as detected by TLC (PE/EA = 1/2), the reaction solution was added to water, the product was extracted with EA (100 mL), the organic phase was washed water (50 mL × 3), dried over anhydrous sodium sulfate and concentrated, and the residue was stirred with silica gel and subjected to column chromatography (PE/EA = 2/1) to give the product which was concentrated by rotary evaporation to give a white solid **9A** (1.5 g, yield: 41%). MS-ESI: m/z 893.4 [M+H]⁺.

**Step 2** Compound **9A** (1.3 g, 1.46 mmol) was dissolved in DCM (13 mL), DEA (2.6 mL) was added dropwise in an ice-water bath under nitrogen atmosphere, and then the resulting mixture was reacted at room temperature for 2 h after the addition was completed. After the reaction was completed as detected by TLC (PE/EA = 1/2), the reaction solution was concentrated by rotary evaporation, extracted three times with DCM, subjected to reversed-phase column chromatography and lyophilized to give an off-white solid **9B** (350 mg, yield: 36%). MS-ESI: m/z 670.8 [M+H]⁺.

**Step 3** Compound **9B** (350 mg, 0.52 mmol, 1.0 eq) and Compound **8D** (243 mg, 0.52 mmol, 1.0 eq) were added into a reaction flask, and the reaction solution was dissolved in DMF (3.5 mL). DIEA (168 mg, 1.3 mmol, 2.5 eq) was added in an ice-water bath under nitrogen atmosphere, a solution of HATU (240 mg, 0.63 mmol, 1.21 eq) in 1 mL of DMF was added dropwise, and then the resulting mixture was stirred at 0-5 °C for 30 min after the addition was completed. After the reaction was completed as detected by LC-MS, the reaction solution was poured into ice water, EA (100 mL) was added, and the product was extracted. The organic phase was washed with water (50 mL × 3), dried over anhydrous sodium sulfate, and concentrated by rotary evaporation to give a foamy yellow solid **9C** (600 mg), with negligible excess yield of crude product. MS-ESI: m/z 1119.4 [M+H]⁺.

**Step 4** Under nitrogen atmosphere, Compound **9C** (400 mg, 0.35 mmol, 1.0 eq), Pd(PPh₃)₄ (82 mg, 0.071 mmol, 0.2 eq) and *N*-methylmorpholine (353 mg, 3.5 mmol, 10.0 eq) were added into a reaction flask, and the reaction mixture was reacted at room temperature for 1 h under nitrogen atmosphere. After the reaction was completed as detected by LC-MS, the reaction solution was directly purified by reversed-phase column chromatography to give a white solid **9D** (150 mg, yield: 39%). MS-ESI: m/z 1079.4 [M+H]⁺.

**Step 5** Under nitrogen atmosphere, Compound **9D** (150 mg, 0.14 mmol) was dissolved in DCM (1.5 mL), DEA (0.3 mL) was added dropwise in an ice-water bath, and the resulting mixture was heated to room temperature for 2 h after the addition was completed. After the reaction was completed as detected by LCMS, the reaction solution was directly concentrated by rotary evaporation, extracted three times with DCM, and purified by reversed-phase column chromatography to give a white solid **9E** (60 mg, yield: 50%). MS-ESI: m/z 857.4 [M+H]⁺. **Step 6** Compound **9E** (50 mg, 0.058 mmol, 1.0 eq) was dissolved in THF (3 mL), 3 drops of water was added for dissolving, and TEA (117 mg, 1.16 mmol, 20.0 eq) was added in an ice-water bath. The reaction mixture was stirred for 1 min, bromoacetyl bromide (93 mg, 0.46 mmol, 8.0 eq) was added, and the resulting mixture was stirred at 0-5 °C for 5 min. After the reaction was completed as detected by LC-MS, the reaction solution was stored with dry ice, subjected to preparative chromatography and lyophilized to give a white solid 9 (11 mg, yield: 19%).

MS-ESI: m/z 977.3 [M+H]⁺.

¹H NMR (400 MHz, DMSO-*d*₆) δ 8.62 (t, *J =* 6.6 Hz, 1H), 8.49 (t, *J* = 5.6 Hz, 1H), 8.26 (t, *J=* 5.8 Hz, 1H), 8.19 (d, *J=* 7.5 Hz, 1H), 7.36 (d, *J* = 7.9 Hz, 2H), 7.30 (d, *J* = 10.1 Hz, 1H), 7.26-7.14 (m, 6H), 6.16 (dd, *J =* 10.1, 1.9 Hz, 1H), 5.93 (s, 1H), 5.39 (s, 1H), 4.91 (d, *J* = 4.9 Hz, 1H), 4.79 (brs, 1H), 4.61-4.54 (m, 2H), 4.49 (d, *J =* 19.4 Hz, 1H), 4.37 (s, 2H), 4.32-4.24 (m, 2H), 4.16 (d, *J=* 19.4 Hz, 1H), 3.95-3.88 (m, 4H), 3.79 (d, *J* = 5.5 Hz, 2H), 3.72 (d, *J* = 5.8 Hz, 2H), 2.58-2.52 (m, 2H), 2.30-2.24 (m, 2H), 2.17-2.05 (m, 1H), 2.05-1.89 (m, 2H), 1.82-1.58 (m, 6H), 1.39 (s, 3H), 1.11-0.96 (m, 2H), 0.85 (s, 3H).

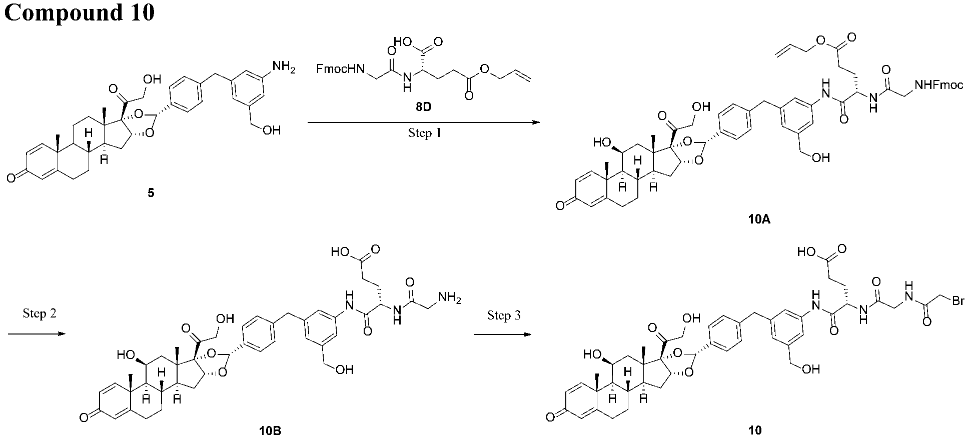

**Step 1** Compound **5** (1.9 g, 3.21 mmol, 1.0 eq), Compound **8D** (1.5 g, 3.21 mmol, 1.0 eq) and HATU (1.6 g, 4.24 mmol, 1.3 eq) were added into a 100-mL three-necked flask, the reaction mixture was dissolved in DMF (45 mL), and then 2,6-lutidine (1.4 g, 12.72 mmol, 4.0 eq) was slowly added. After the starting material was consumed completely as detected by LCMS, the reaction solution was added slowly and dropwise to water (300 mL) and filtered to give the product which was dried by oil pump under vacuum to give a yellow solid **10A** (3.3 g, yield: 94%). MS-ESI: m/z 1048.4 [M+H]⁺.

**Step 2** Compound **10A** (1.8 g, 1.72 mmol, 1.0 eq) and Pd(PPh₃)₄ (392 mg, 0.34 mmol, 0.2 eq) were added into a 50-mL three-necked flask, and the reaction mixture was dissolved in dichloromethane (30 mL). Under nitrogen atmosphere, *N*-methylmorpholine (868 mg, 8.6 mmol, 5.0 eq) was slowly added, and the resulting mixture was reacted for 0.5 h. After the reaction was completed as detected by LCMS, diethylamine (6 mL) was added slowly into the three-necked flask and stirred at room temperature for 1 h. After the reaction was completed as detected by LCMS, DMF (20 mL) was added to the reaction solution, and the resulting mixture was subjected to rotary evaporation under vacuum at room temperature until 20 mL of the solvent remained, then the rotary evaporation was stopped, and the residue was subjected to reversed-phase column chromatography (ACN in water from 30%-70%) and lyophilized to give a light yellow solid **10B** (455 mg, yield: 34%).

**Step 3** Compound **10B** (150 mg, 0.19 mmol, 1.0 eq) was dissolved in THF (5 mL) and water (0.2 mL), triethylamine (190 mg, 1.9 mmol, 10 eq) was added, and the reaction solution was cooled to 0 °C in an ice-water bath. Bromoacetyl bromide (153 mg, 0.76 mmol, 4.0 eq) was dissolved in 1 mL THF, and then the resulting solution was added slowly to the above reaction solution and reacted at 0 °C for 15 min. After the reaction was completed as detected by LCMS, the reaction solution was subjected to preparative chromatography to give a white solid **10** (20 mg, yield: 12%).

MS-ESI: m/z 906.3 [M+H]⁺.

¹H NMR (400 MHz, DMSO-*d*₆) δ 9.87 (s, 1H), 8.52 (s, 1H), 8.22 (d, *J* = 7.6 Hz, 1H), 7.43 (s, 1H), 7.38 (d, *J* = 7.9 Hz, 2H), 7.31 (d, *J* = 9.7 Hz, 2H), 7.23 (d, *J* = 7.9 Hz, 2H), 6.87 (s, 1H), 6.16 (d, *J* = 10.3 Hz, 1H), 5.93 (s, 1H), 5.39 (s, 1H), 4.91 (d, *J =* 5.1 Hz, 1H), 4.78 (s, 1H), 4.49 (d, *J =* 19.2 Hz, 1H), 4.41 (s, 2H), 4.39-4.32 (m, 1H), 4.29 (s, 1H), 4.17 (d, *J* = 19.5 Hz, 1H), 3.93 (s, 2H), 3.88 (s, 2H), 3.82-3.77 (m, 2H), 2.59-2.53 (m, 1H), 2.35-2.21 (m, 4H), 2.04-1.92 (m, 2H), 1.84-1.63 (m, 6H), 1.39 (s, 3H), 1.09-0.99 (m, 2H), 0.86 (s, 3H).

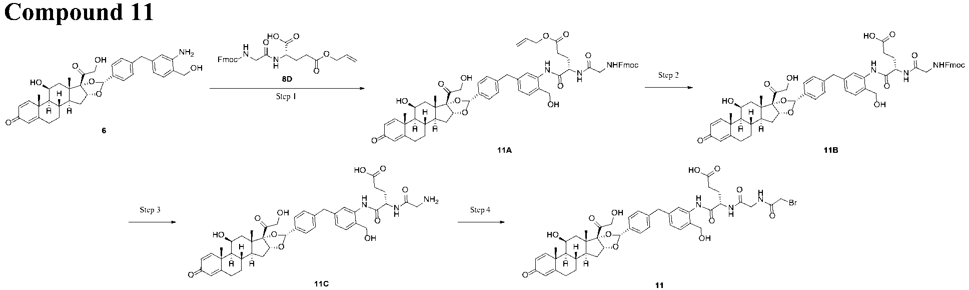

**Step 1** Compound 6 (300 mg, 0.5 mmol, 1.0 eq), Compound **8D** (233 mg, 0.5 mmol, 1.0 eq) and HATU (228 mg, 0.6 mmol, 1.2 eq) were added into a 25-mL three-necked flask, the reaction mixture was dissolved in DMF (5 mL), and then 2,6-lutidine (150 mg, 1.4 mmol, 2.8 eq) was slowly added. After the starting material was consumed completely as detected by LCMS, the reaction solution was added slowly and dropwise to water (100 mL) and filtered to give the product which was dried by oil pump under vacuum to give a yellow solid **11A** (450 mg, yield: 86%). MS-ESI: m/z 1048.4 [M+H]⁺.

**Step 2** Compound **11A** (450 mg, 0.43 mmol, 1.0 eq) and Pd(PPh₃)₄ (92 mg, 0.08 mmol, 0.2 eq) were added into a 50-mL three-necked flask, and the reaction mixture was dissolved in THF (10 mL). Under nitrogen atmosphere, N-methylmorpholine (200 mg, 1.98 mmol, 4.6 eq) was slowly added, and the resulting mixture was reacted for 0.5 h. After the reaction was completed as detected by LCMS, the reaction solution was subjected to reversed-phase column chromatography (ACN in water from 30%-70%) and lyophilized to give a light yellow solid **11B** (250 mg, yield: 58%). MS-ESI: m/z 1008.3 [M+H]⁺.

**Step 3** Compound **11B** (250 mg, 0.25 mmol) was added into a 25-mL three-necked flask, and the reaction mixture was dissolved in DCM (10 mL). Under nitrogen atmosphere, diethylamine (2 mL) was added, and the resulting mixture was reacted for 0.5 h. After the reaction was completed as detected by LCMS, the reaction solution was subjected to reversed-phase column chromatography (ACN in water from 20%-50%) and lyophilized to give a light yellow solid **11C** (130 mg, yield: 67%). MS-ESI: m/z 786.4 [M+H]⁺.

**Step 4** Compound **11C** (120 mg, 0.153 mmol, 1.0 eq) was dissolved in THF (5 mL) and water (0.2 mL), triethylamine (155 mg, 1.53 mmol, 10.0 eq) was added, and the reaction solution was cooled to 0 °C in an ice-water bath. Bromoacetyl bromide (123 mg, 0.61 mmol, 4.0 eq) was dissolved in 1 mL THF, and then the resulting solution was added slowly to the above reaction solution and reacted at 0 °C for 15 min. After the reaction was completed as detected by LCMS, the reaction solution was subjected to prep-HPLC to give a white solid **11** (12.2 mg, yield: 9%).

MS-ESI: m/z 906.3 [M+H]⁺.

¹H NMR (400 MHz, DMSO-*d*₆) δ 9.51 (s, 1H), 8.58-8.45 (m, 1H), 8.37 (d, *J =* 7.6 Hz, 1H), 7.51-7.46 (m, 1H), 7.40-7.34 (m, 2H), 7.31 (d, *J =* 10.0 Hz, 1H), 7.28-7.19 (m, 3H), 7.01-6.94 (m, 1H), 6.16 (dd, *J =* 10.0, 1.9 Hz, 1H), 5.93 (s, 1H), 5.39 (s, 1H), 4.91 (d, *J =* 5.0 Hz, 1H), 4.77 (brs, 1H), 4.49 (d, *J =* 19.5 Hz, 1H), 4.42 (d, *J =* 3.7 Hz, 1H), 4.39-4.32 (m, 1H), 4.29 (brs, 1H), 4.17 (d, *J =* 19.5 Hz, 1H), 3.98-3.77 (m, 6H), 2.60-2.52 (m, 2H), 2.34-2.28 (m, 2H), 2.18-1.96 (m, 3H), 1.88-1.55 (m, 6H), 1.39 (s, 3H), 1.13-0.98 (m, 2H), 0.85 (s, 3H).

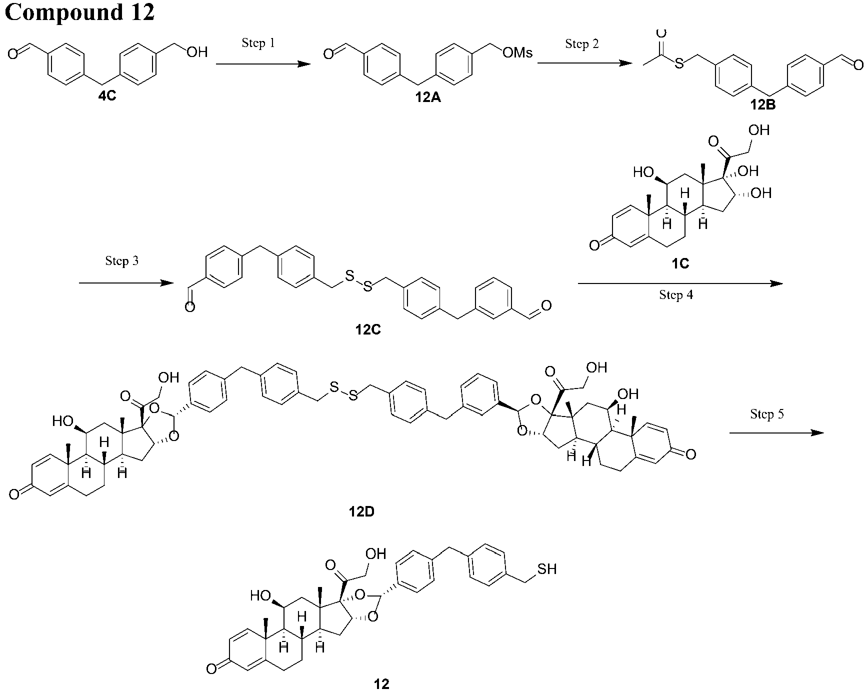

Step 1 TEA (8.94 g, 88.39 mmol, 12.30 mL) was added to a solution of Compound **4C** (10.0 g, 44.2 mmol) in DCM (70 mL), and the reaction solution was cooled to 0 °C. MsCl (6.08 g, 53.0 mmol, 4.10 mL) was added, and the resulting mixture was stirred at 25 °C for 12 h. After the starting material was consumed completely as detected by LCMS, the reaction solution was diluted with DCM (50 mL), quenched with water (200 mL), and extracted with DCM (100 mL × 3). The organic phases were combined, washed with saturated brine (100 mL), dried over anhydrous Na₂SO₄, and filtered. and the filtrate was concentrated by rotary evaporation to give Compound **12A** (8.60 g, crude) as a yellow oil.

**Step 2** Thioglycolic acid (7.21 g, 63.15 mmol) was added to a solution of Compound **12A** (8.60 g, 31.58 mmol) in DMF (51.6 mL), and the reaction solution was stirred at 25 °C for 5 h. After the starting material was consumed completely as detected by TLC (PE/EA = 5:1), Water (100 mL) was added to the reaction solution, and the mixture was extracted with ethyl acetate (100 mL × 3). The organic phases were combined, washed with a saturated saline solution and then with 5% aqueous LiCl solution, dried over anhydrous Na₂SO₄, and filtered, and the filtrate was concentrated by rotary evaporation to give Compound **12B** (5.75 g, yield: 57.82%, purity: 90.2%) as a brown oil. MS-ESI: m/z 285.2 [M+H]⁺.

**Step 3** Compound **12B** (12.0 g, 42.2 mmol) was dissolved in methanol (240 mL), K₂CO₃ (8.75 g, 63.3 mmol) was added, and the reaction solution was stirred at 20 °C for 3 h. After the starting material was consumed completely as detected by LCMS, the reaction solution was poured into DCM (240 mL), and 0.5 M HCl (100 mL) was added, followed by liquid separation. The organic phase was dried over anhydrous sodium sulfate, and concentrated to give an off-white solid **12C** (10.0 g, yield: 97.7%). MS-ESI: m/z 483.2 [M+H]⁺.

**Step 4** Compound **12C** (9.00 g, 37.1 mmol), Compound **1C** (13.9 g, 37.1 mmol) and anhydrous magnesium sulfate (13.4 g, 111 mmol) were added to acetonitrile (270 mL), TfOH (16.7 g, 111 mmol, 9.84 mL) was added dropwise at 0 °C, and the reaction solution was gradually heated to 20 °C after the addition was completed, and stirred for 3 h. After the starting material was consumed completely as detected by LCMS, the reaction solution was quenched with saturated aqueous sodium bicarbonate (60 mL), and extracted with ethyl acetate (100 mL × 2). The organic phase was washed with saturated brine (80 mL), dried over anhydrous sodium sulfate, and concentrated to give an off-white solid **12D** (15.0 g, yield: 33.6%). MS-ESI: m/z 1199.4 [M+H]⁺.

**Step 5** Compound **12D** (290 mg, crude) was added to DMF (4 mL), then DL-dithiothreitol (200 mg, 1.30 mmol) was added, and the reaction solution was reacted at room temperature for 16 h under nitrogen atmosphere. After the reaction was completed as detected by LCMS, the reaction solution was directly subjected to prep-HPLC to give a white powdery solid **12** (20 mg, yield: 13.8%). MS-ESI: m/z 601.2 [M+H]⁺.

¹H NMR (400 MHz, DMSO) δ 7.37 (d, *J =* 8.1 Hz, 2H), 7.31 (d, *J =* 10.1 Hz, 1H), 7.23 (dd, *J =* 8.1, 3.3 Hz, 4H), 7.14 (d, *J* = 8.0 Hz, 2H), 6.16 (dd, *J* = 10.1, 1.7 Hz, 1H), 5.93 (s, 1H), 5.39 (s, 1H), 4.91 (d, *J =* 5.0 Hz, 1H), 4.78 (s, 1H), 4.49 (d, *J =* 19.4 Hz, 1H), 4.29 (s, 1H), 4.17 (d, *J =* 19.5 Hz, 1H), 3.88 (d, *J* = 11.3 Hz, 2H), 3.67 (d, *J =* 7.6 Hz, 2H), 2.77 (t, *J =* 7.6 Hz, 1H), 2.50-2.55 (m, 1H), 2.45-2.25 (m, 1H), 2.25-2.05 (m, 2H), 1.80 - 1.53 (m, 5H), 1.38 (s, 3H), 1.14 - 0.94 (m, 2H), 0.86 (s, 3H).

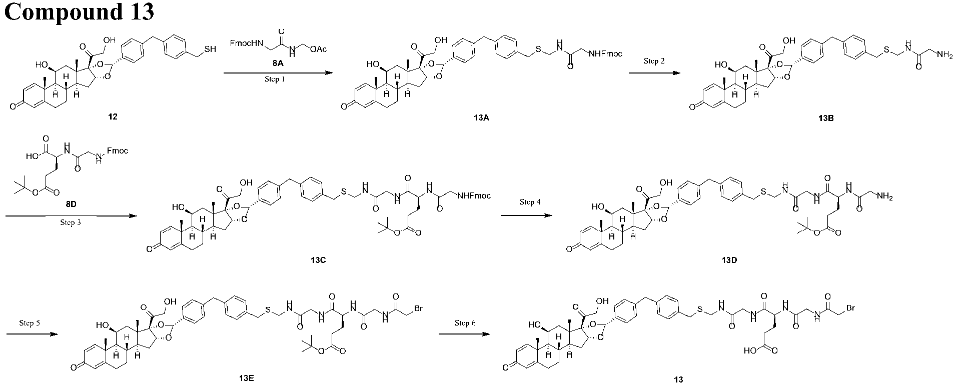

**Step 1** Compound **12** (7.00 g, 11.6 mmol) and Compound **8A** (4.29 g, 11.6 mmol) were added to DMF (49.0 mL), TfOH (3.50 g, 23.3 mmol, 2.06 mL) was added, and the reaction solution was stirred at 25 °C for 16 h. After the starting material was consumed completely as detected by LCMS, the reaction solution was quenched with saturated aqueous sodium bicarbonate (20 mL), and extracted with ethyl acetate (100 mL × 3). The organic phase was washed with saturated brine (100 mL), dried over anhydrous sodium sulfate, and concentrated to give Compound **13A** as a yellow oil (15.0 g, with negligible yield of crude product). MS-ESI: m/z 909.3 [M+H]⁺.

**Step 2** Compound **13A** (15.0 g, 16.5 mmol) was added to acetonitrile (105 mL), diethylamine (6.03 g, 82.5 mmol, 8.50 mL) was added, and the reaction solution was stirred at 25 °C for 16 h. After the starting material was consumed completely as detected by LCMS, the reaction solution was slurried with acetonitrile (20.0 mL) and filtered, the resulting filter cake was drained, and the crude product was subjected to preparative reversed-phase chromatography (water:acetonitrile = 25%-55%, 20 min), and lyophilized to give Compound **13B** as a white solid (0.900 g, yield: 7.94%). MS-ESI: m/z 687.3 [M+H]⁺.

**Step 3** Compound **13B** (500 mg, 727 µmol) and Compound **8D** (526 mg, 1.09 mmol) were added to DMF (5 mL), HATU (830 mg, 2.18 mmol) and 2,6-lutidine (56.0 mg, 1.46 mmol, 169 µL) were added, and the reaction solution was stirred at 20 °C for 3 h. After the starting material was consumed completely as detected by LCMS, the reaction solution was dried under vacuum to give a crude product (550 mg), 250 mg of which was subjected to preparative reversed-phase chromatography to give Compound **13C** (100 mg, yield: 26.2%) as a white solid. MS-ESI: m/z 1151.4 [M+H]⁺.

**Step 4** Compound **13C** (50.0 mg, 43.4 µmol) was added to acetonitrile (1 mL), diethylamine (15.8 mg, 217 µmol, 22.3 µL) was added, and the reaction solution was stirred at 25 °C for 6 h. After the starting material was consumed completely as detected by LCMS, the reaction solution was concentrated by rotary evaporation and slurried with methyl *tert*-butyl ether (3.00 mL) to give Compound **13D** as a yellow solid (40.0 mg, yield: 99.1%). MS-ESI: m/z 929.2 [M+H]⁺.

**Step 5** EEDQ (31.9 mg, 129 µmol) and bromoacetic acid (11.3 mg, 81.8 µmol) were dissolved in DMF (0.8 mL), and the reaction solution was stirred at room temperature for 1 h. Compound **13D** (40.0 mg, 43.0 µmol) was added, and the resulting mixture was stirred at 25 °C for 2 h. After the starting material was consumed completely as detected by LCMS, the reaction solution was concentrated by rotary evaporation to give Compound **13E** as a yellow solid (the crude product was directly used in the next step). MS-ESI: m/z 1049.2 [M+H]⁺.

**Step 6** Compound **13E** (45.0 mg, 42.8 µmol) was dissolved in DCM (1.50 mL), trifluoroacetic acid (770 mg, 6.75 mmol, 500 µL) was added, and the reaction solution was stirred at 25 °C for 1 h. After the starting material was consumed completely as detected by LCMS, the reaction solution was concentrated by rotary evaporation, and then the crude product was purified by preparative reversed-phase chromatography to give Compound 13 (3.06 mg, yield: 7.18%) as a yellow solid. MS-ESI: m/z 993.2 [M+H]⁺.

¹HNMR (400 MHz, DMSO-*d*₆) *δ* 7.83 (br d, 1H, *J =* 7.6 Hz), 7.63 (br d, 1H, *J =* 7.2 Hz), 7.4 (m, 2H), 7.26 (br d, 2H, *J* = 8.0 Hz), 7.16 (br d, 1H, *J* = 7.6 Hz), 4.92 (br s, 1H), 4.48 (br s, 1H), 4.30 (br s, 2H), 4.1-4.3 (m, 2H), 4.02 (br d, 1H, *J* = 13.6 Hz), 3.92 (br d, 2H, *J =* 8.4 Hz), 3.81 (br d, 2H, *J* = 5.2 Hz), 3.74 (br s, 3H), 3.63 (br s, 2H), 2.91 (s, 2H), 2.75 (br s, 1H), 2.3-2.4 (m, 2H), 2.27 (br s, 2H), 2.1-2.1 (m, 1H), 2.02 (br s, 1H), 1.92 (br s, 1H), 1.77 (br s, 3H), 1.3-1.5 (m, 8H), 1.25 (s, 2H), 1.18 (br d, 1H, *J=* 7.2 Hz), 1.02 (br s, 1H), 0.8-1.0 (m, 2H).

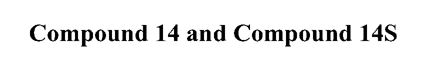

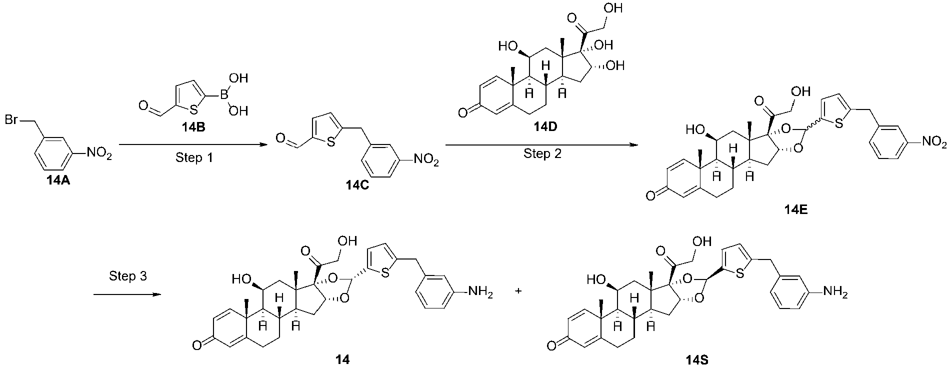

Step 1 Compound 14A(8 g, 37 mmol), Compound 14B (5.8 g, 37 mmol) and K₂CO₃ (15.3 g, 111 mmol) were added to THF (80 mL), and water (4 mL) and Pd(dppf)Cl₂ (2.7 g, 3.7 mmol) were added. Under nitrogen atmosphere, the external temperature was raised to 80 °C, and the mixed solution was refluxed for 16 h. After the reaction was completed as detected by TLC (PE:EA = 5:1), the reaction solution was cooled to room temperature and filtered, and water (300 mL) and EA (300 mL) were added to extract the product. The organic phase was washed with water (100 mL × 3), dried over anhydrous sodium sulfate, concentrated by rotary evaporation, stirred with silica gel and purified by column chromatography (PE:EA = 10:1) to give a white foamy solid 14C (2.86 g, yield: 31%). MS-ESI: m/z 248.1 [M+H]⁺.

Step 2 Compound 14C (200 mg, 0.81 mmol), Compound 14D (304 mg, 0.81 mmol), acetonitrile (6 mL) and MgSO₄ (388 mg, 3.24 mmol) were added to a reaction flask. Under nitrogen atmosphere, the mixed solution was cooled to 0 °C. Acetonitrile (1 mL) was dissolved in methanesulfonic acid (233 mg, 2.43 mmol) and added dropwise to the reaction system. After addition, the mixed solution was stirred at 0 °C to 10 °C for 6 h. After the content of the product was 70% as detected by LCMS, the reaction solution was poured into a saturated aqueous sodium bicarbonate solution (40 mL), and the product was extracted with EA (40 mL × 2). The organic phase was washed with water (40 mL × 3), dried over anhydrous sodium sulfate and concentrated by rotary evaporation to give a yellow solid 14E (480 mg, with negligible yield of crude product). MS-ESI: m/z 606.2 [M+H]⁺.

Step 3 Compound 14E (240 mg, 0.40 mmol), iron powder (222 mg, 4.00 mmol) and NH₄Cl (212 mg, 4.00 mmol) were added to a reaction flask, and ethanol (3 mL) and water (0.75 mL) were added. Under nitrogen atmosphere, the mixed solution was heated to 60 °C and reacted for 1 h. After the reaction was completed as detected by LCMS, the reaction solution was cooled to room temperature and filtered, and water (40 mL) and EA (100 mL) were added to extract the product. The organic phase was washed with water (40 mL × 2), dried over anhydrous sodium sulfate, concentrated by rotary evaporation and purified by preparative thin layer chromatography (PE:EA = 1:2) to give a white solid (40 mg). The solid was purified by reversed-phase preparative chromatography and lyophilized to give Compound 14 (15 mg, yield: 6%) and Compound 14S (4.4 mg, yield: 2%).

### Compound 14:

MS-ESI: m/z 576.2 [M+H]⁺.

¹H NMR (400 MHz, DMSO-*d*₆) δ 7.31 (d, *J* = 10.1 Hz, 1H), 7.12 - 7.06 (m, 1H), 7.02 (t, *J=* 8.0 Hz, 1H), 6.73 (d, *J* = 3.5 Hz, 1H), 6.69 - 6.53 (m, 3H), 6.18 (dd, *J* = 10.1, 1.9 Hz, 1H), 5.97 (s, 1H), 5.73 (s, 1H), 4.88 (d, *J* = 4.3 Hz, 1H), 4.80 (s, 1H), 4.48 (d, *J* = 19.4 Hz, 1H), 4.29 (d, *J* = 4.2 Hz, 1H), 4.15 (d, *J* = 19.5 Hz, 1H), 3.98 (s, 2H), 2.60 - 2.54 (m, 1H), 2.37 - 2.28 (m, 1H), 2.18 - 1.94 (m, 2H), 1.79 - 1.54 (m, 5H), 1.39 (s, 3H), 1.02 - 0.91 (m, 1H), 0.88 (dd, *J* = 11.2, 3.5 Hz, 1H), 0.84 (s, 3H).

### Compound 14S:

MS-ESI: m/z 576.3 [M+H]⁺.

¹H NMR (400 MHz, DMSO-*d*₆) δ 7.31 (d, *J =* 10.1 Hz, 1H), 7.15 - 7.08 (m, 1H), 7.00 (d, *J=* 3.5 Hz, 1H), 6.75 (d, *J* = 3.5 Hz, 1H), 6.74 - 6.59 (m, 3H), 6.29 (s, 1H), 6.17 (dd, *J=* 10.1, 1.9 Hz, 1H), 5.94 (s, 1H), 5.20 (d, *J =* 7.0 Hz, 1H), 4.78 (s, 1H), 4.36 (d, *J =* 19.2 Hz, 1H), 4.29 (s, 1H), 4.10 - 3.94 (m, 3H), 2.59 - 2.53 (m, 1H), 2.32 - 2.26 (m, 1H), 2.11 - 1.94 (m, 3H), 1.86 - 1.61 (m, 5H), 1.38 (s, 3H), 1.21 - 1.12 (m, 1H), 1.03 (dd, *J =* 11.2, 3.4 Hz, 1H), 0.86 (s, 3H).

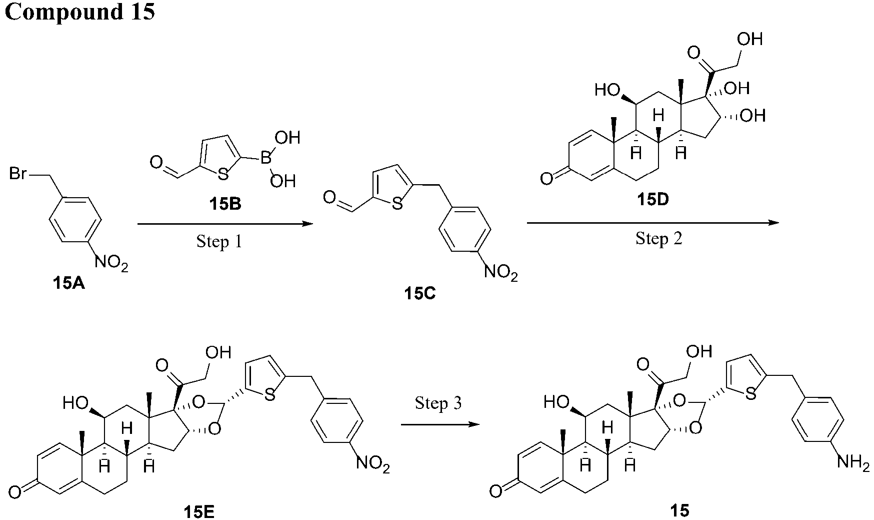

**Step 1** Compound 15A (5 g, 23.1 mmol), 15B (3.6 g, 23.1 mmol) and K₂CO₃ (9.6 g, 69.3 mmol) were dissolved in THF (50 mL), and water (5 mL) was added. Under nitrogen atmosphere, Pd(dppf)Cl₂ (3.38 g, 4.6 mmol) was added, the external temperature was raised to 80 °C, and the mixed solution was refluxed for 16 h. After the reaction was completed as detected by TLC (PE:EA = 5:1), the reaction solution was cooled to room temperature and filtered, and water (300 mL) and EA (300 mL) were added to extract the product. The organic phase was washed with water (100 mL × 3), dried over anhydrous sodium sulfate, stirred with silica gel, concentrated and purified by column chromatography (PE:EA = 15:1) to give a yellow solid **15C** (830 mg, yield: 14%). MS-ESI: m/z 248.1 [M+H]⁺.

**Step 2** Under nitrogen atmosphere, compound **15C** (800 mg, 3.2 mmol), **15D** (1.2 g, 3.2 mmol), acetonitrile (20 mL) and MgSO₄ (15.5 g, 12.9 mmol) were added to a reaction flask. The mixed solution was cooled to -20 °C, and a solution of trifluoromethanesulfonic acid (1.46 g, 9.7 mmol) in acetonitrile (5 mL) was cooled and added dropwise to the reaction system. After addition, the resulting solution was stirred at -20 °C for 1 h. After the reaction was completed as detected by LCMS, the reaction solution was poured into a saturated aqueous sodium bicarbonate solution (100 mL), and the product was extracted with EA (100 mL × 2). The organic phase was washed with water (50 mL × 3), dried over anhydrous sodium sulfate, concentrated, stirred with silica gel and purified by column chromatography to give a yellow solid **15E** (1 g, yield: 51%). MS-ESI: m/z 606.3 [M+H]⁺.

**Step 3** Compound **15E** (100 mg, 0.165 mmol), iron powder (93 mg, 1.65 mmol) and NH₄Cl (88 mg, 1.65 mmol) were added to a reaction flask, and ethanol (0.8 mL) and water (0.2 mL) were added. Under nitrogen atmosphere, the mixed solution was heated to 60 °C and reacted for 1 h. After the reaction was completed as detected by LCMS, the reaction solution was cooled to room temperature and filtered, and water (40 mL) and EA (100 mL) were added to extract the product. The organic phase was washed with water (40 mL × 2), purified by preparative thin layer chromatography (PE:EA = 1:3) and lyophilized to give a yellow solid 15 (46 mg, yield: 48%).

MS-ESI: m/z 576.3 [M+H]⁺.

¹H NMR (400 MHz, DMSO-*d*₆) δ 7.36 - 7.28 (m, 1H), 7.04 (d, *J* = 3.5 Hz, 1H), 6.84 (d, *J* = 8.3 Hz, 2H), 6.66 (d, *J* = 3.5 Hz, 1H), 6.45 (d, *J* = 8.4 Hz, 2H), 6.19 (dd, *J=* 10.1, 1.9 Hz, 1H), 6.00 (s, 1H), 5.73 (s, 1H), 5.07 (t, *J =* 5.9 Hz, 1H), 4.93 - 4.85 (m, 3H), 4.82 - 4.76 (m, 1H), 4.48 (dd, *J* = 19.5, 6.3 Hz, 1H), 4.34 - 4.26 (m, 1H), 4.15 (dd, *J =* 19.5, 5.6 Hz, 1H), 3.88 (s, 2H), 2.60 - 2.52 (m, 1H), 2.38 - 2.30 (m, 1H), 2.17 - 1.96 (m, 2H), 1.78 - 1.57 (m, 5H), 1.39 (s, 3H), 1.06 - 0.93 (m, 1H), 0.91 - 0.86 (m, 1H), 0.84 (s, 3H).

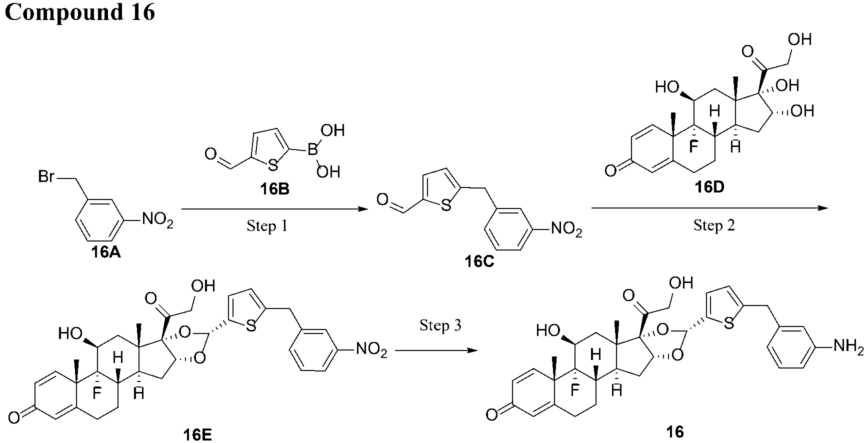

**Step 1** Compound **16A** (7.6 g, 35.2 mmol) and **16B** (5.5 g, 35.2 mmol) were added to a 500-mL single-necked flask and dissolved in **THF** (60 mL), and **K₂CO₃** (14.6 g, 105.6 mmol) and H₂O (10 mL) were added. Under nitrogen atmosphere, **Pd(dppf)Cl₂** (5.2 g, 7.04 mmol) was added. The resulting solution was refluxed at 80 °C for 2 h. After the starting materials were substantially consumed as detected by LCMS, the reaction solution was filtered, and the mother liquor was extracted with EA (100 mL) and H₂O (70 mL) and subjected to liquid separation. The organic phase was dried, concentrated by rotary evaporation and purified by column chromatography (PE:EA = 3:1) to give a yellow solid product **16C** (1.2 g, yield: 14%). MS-ESI: m/z 248.1 [M+H]⁺.

**Step 2** Compound **16C** (1.12 g, 4.53 mmol) and **16D** (1.79 g, 4.53 mmol) were dissolved in **MeCN** (10 mL), and **MgSO₄** (1.64 g, 13.6 mmol) was added. Under nitrogen atmosphere, TfOH (1.36 g, 9.07 mmol) was added at 0 °C, and the mixed solution was reacted for 3 h. After a product was generated as detected by LCMS, the reaction solution was filtered, directly concentrated by rotary evaporation, stirred with silica gel and purified (DCM:MeOH = 13:1) to give an orange-red solid **16E** (750 mg, yield: 26%). MS-ESI: m/z 624.3 [M+H]⁺.

**Step 3** Compound **16E** (700 mg, 1.124 mmol) and **Fe** (629 mg, 11.24 mmol) were dissolved in **EtOH** (9 mL), a solution of **NH₄Cl** (596 mg, 11.24 mmol) in H₂O (3 mL) was added, and the mixed solution was reacted at 60 °C for 2 h under nitrogen atmosphere. After the starting materials were substantially consumed as detected by LCMS, the reaction solution was filtered, extracted with water (10 mL) and EA (15 mL) and subjected to liquid separation. The organic phase was concentrated by rotary evaporation to give a yellow solid **16** (600 mg, yield: 90%).

MS-ESI: m/z 594.3 [M+H]⁺.

¹H NMR (400 MHz, DMSO-*d*₆) δ 7.29 (d, *J* = 10.1 Hz, 1H), 7.18 (t, *J* = 7.8 Hz, 1H), 7.08 (d, *J* = 3.5 Hz, 1H), 6.89 (d, *J* = 7.6 Hz, 1H), 6.86 - 6.80 (m, 2H), 6.75 (d, *J* = 3.5 Hz, 1H), 6.24 (dd, *J* = 10.1, 1.9 Hz, 1H), 6.05 (s, 1H), 5.74 (s, 1H), 5.43 (s, 1H), 4.92 - 4.85 (m, 1H), 4.49 (d, *J =* 19.4 Hz, 1H), 4.22 - 4.13 (m, 2H), 4.06 (s, 2H), 2.71 - 2.52 (m, 2H), 2.49 - 2.40 (m, 1H), 2.40 - 2.31 (m, 1H), 2.20 - 2.07 (m, 1H), 2.05 - 1.95 (m, 1H), 1.88 - 1.79 (m, 1H), 1.71 - 1.57 (m, 3H), 1.50 (s, 3H), 1.44 - 1.29 (m, 1H), 0.85 (s, 3H).

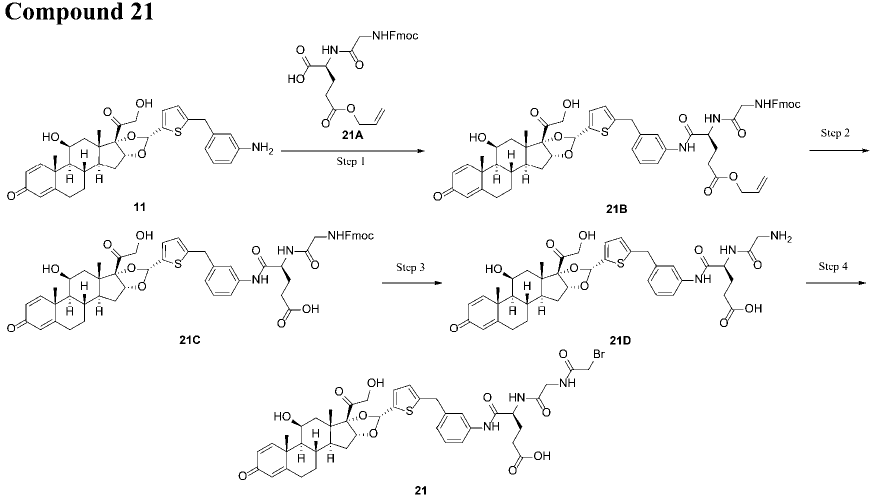

**Step 1** Compound **14** (2 g, 3.48 mmol), 21A (1.63 g, 3.48 mmol) and HATU (1.59 g, 4.18 mmol) were added to a 100-mL three-necked flask, DMF (30 mL) was added for dissolution, and 2,6-lutidine (1.12 g, 10.44 mmol) was slowly added. After the starting materials were completely consumed as detected by LCMS, the reaction solution was slowly added dropwise to water (300 mL) and filtered to give a product, which was dried by oil pump under vacuum to give a white solid **21B** (3.5 g, yield: 98%). MS-ESI: m/z 1024.4 [M+H]⁺.

**Step 2** Compound **19B** (1.0 g, 1.0 mmol, 1.0 eq) and **Pd(PPh₃)₄** (392 mg, 0.339 mmol, 0.35 eq) were added to a 50-mL three-necked flask, and DCM (30 mL) was added for dissolution. Under nitrogen atmosphere, NMMP (868 mg, 8.6 mmol, 8.8 eq) was slowly added. After the mixed solution was reacted for 0.5 h, the reaction was completed as detected by LCMS, stirred with silica gel, concentrated and purified by column chromatography (DCM in MeOH from 0% to 15%) to give a yellow solid **21C** (0.88 g, yield: 92%). MS-ESI: m/z 984.4 [M+H]⁺.

**Step 3** Compound **21C** (700 mg, 0.71 mmol) was dissolved in DMF (10 mL) and DEA (2 mL), and the mixed solution was reacted at room temperature for 15 min. After the reaction was completed as detected by LCMS, the reaction solution was purified by reversed-phase column chromatography (ACN in water from 20% to 50%) to give a yellow solid **21D** (300 mg, yield: 55%). MS-ESI: m/z 762.3 [M+H]⁺.

**Step 4** Compound **21D** (100 mg, 0.13 mmol) was dissolved in THF (5 mL) and water (0.5 mL), and 2,6-lutidine (139 mg, 1.3 mmol) was added at room temperature. Bromoacetyl bromide (130 mg, 0.65 mmol) was dissolved in THF (1 mL), and the resulting solution was slowly added to the reaction solution. The mixed solution was reacted at room temperature for 15 min. After the reaction was completed as detected by LCMS, the reaction solution was purified by prep-HPLC (ACN in 0.1% TFA) to give a white solid **21** (19.4 mg, yield: 17%).

MS-ESI: m/z 882.2 [M+H]⁺.

¹H NMR (400 MHz, DMSO-*d*₆) δ 12.14 (brs, 1H), 9.92 (s, 1H), 8.52 (t, *J =* 5.6 Hz, 1H), 8.25 (d, *J =* 7.8 Hz, 1H), 7.51 - 7.42 (m, 2H), 7.31 (d, *J =* 10.1 Hz, 1H), 7.23 - 7.14 (m, 1H), 7.08 (d, *J =* 3.5 Hz, 1H), 6.96 - 6.89 (m, 1H), 6.73 (d, *J =* 3.5 Hz, 1H), 6.18 (dd, *J =* 10.1, 1.9 Hz, 1H), 5.97 (s, 1H), 5.73 (s, 1H), 4.88 (d, *J =* 4.8 Hz, 1H), 4.79 (s, 1H), 4.49 (d, *J* = 19.5 Hz, 1H), 4.44 - 4.35 (m, 1H), 4.29 (s, 1H), 4.15 (d, *J =* 19.5 Hz, 1H), 4.07 (s, 2H), 3.94 (s, 2H), 3.86 - 3.75 (m, 2H), 2.59 - 2.52 (m, 1H), 2.38 - 2.17 (m, 3H), 2.17 - 1.91 (m, 3H), 1.89 - 1.78 (m, 1H), 1.78 - 1.54 (m, 5H), 1.39 (s, 3H), 1.01 - 0.91 (m, 1H), 0.88 (dd, *J* = 11.2, 3.5 Hz, 1H), 0.84 (s, 3H).

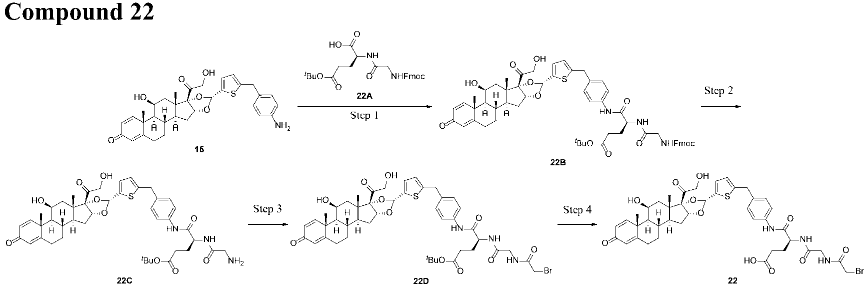

**Step 1** Compound **15** (350 mg, 0.6 mmol) and compound **22A** (289 mg, 0.6 mmol) were added to a 25-mL three-necked flask, and DMF (8 mL) was added. Under nitrogen atmosphere, 2,6-lutidine (196 mg, 1.8 mmol) was added in an ice-water bath, and the mixed solution was stirred for 10 min. HATU (301 mg, 0.79 mmol) was added dropwise to DMF solution (1 mL). After addition, the resulting solution was heated to room temperature and stirred for 1 h. After the reaction was completed as detected by LCMS, the reaction solution was slowly added dropwise to water (100 mL), a large amount of solid was precipitated, and the mixture was stirred for 30 min and filtered. The solid was lyophilized to give a white solid **22B** (525 mg, yield: 83%). MS-ESI: m/z 1040.4 [M+H]⁺.

**Step 2** Compound **22B** (200 mg, 0.192 mmol) and DCM (4 mL) were added to a 25-mL single-necked flask, and the mixed solution was stirred for complete dissolution under nitrogen atmosphere. DEA (0.57 mL) was added dropwise, and after addition, the resulting solution was heated to room temperature and reacted for 4 h. After the reaction was completed as detected by LCMS, the reaction solution was concentrated to dryness and purified by reversed-phase preparative chromatography to give a white solid **22C** (70 mg, yield: 44%). MS-ESI: m/z 818.4 [M+H]⁺.

**Step 3** Compound **22C** (70 mg, 0.086 mmol) was dissolved in THF (5 mL) and water (0.5 mL), 2,6-lutidine (46 mg, 0.43 mmol) was added in an ice-water bath, and the resulting solution was stirred for 5 min. Bromoacetyl bromide (86 mg, 0.43 mmol) was added, and after addition, the mixed solution was reacted in an ice-water bath for 10 min. After the reaction was completed as detected by LCMS, the reaction solution was added to water, the pH was adjusted to 1-2 with TFA, and the product was extracted with EA (20 mL). The organic phase was washed with water (10 mL × 2), dried over anhydrous sodium sulfate, concentrated to a small volume and directly used in the next step with negligible yield of crude product. MS-ESI: m/z 938.3 [M+H]⁺.

**Step** 4 Under nitrogen atmosphere, the organic phase from the previous step (3 mL) was added to TFA (1.5 mL) at room temperature. The resulting solution was stirred at room temperature for 4 h. After the reaction was completed as detected by LCMS, the reaction solution was directly purified by reversed-phase preparative chromatography and lyophilized to give a white solid **22** (6.5 mg, two-step yield: 9%).

MS-ESI: m/z 882.2 [M+H]⁺.

¹H NMR (400 MHz, DMSO-*d*₆) δ 12.14 (brs, 1H), 9.91 (s, 1H), 8.52 (t, *J =* 5.6 Hz, 1H), 8.27 (d, *J=* 7.9 Hz, 1H), 7.52 (d, *J* = 8.6 Hz, 2H), 7.32 (d, *J=* 10.1 Hz, 1H), 7.15 (d, *J* = 8.6 Hz, 2H), 7.07 (s, 1H), 6.70 (d, *J* = 3.5 Hz, 1H), 6.19 (dd, *J* = 10.1, 1.9 Hz, 1H), 5.99 (s, 1H), 5.72 (s, 1H), 4.88 (d, *J* = 4.7 Hz, 1H), 4.80 (s, 1H), 4.49 (d, *J* = 19.5 Hz, 1H), 4.45 - 4.37 (m, 1H), 4.30 (s, 1H), 4.20 - 4.11 (m, 1H), 4.11 - 3.98 (m, 2H), 3.94 (s, 2H), 3.81 (dd, *J* = 5.7, 1.6 Hz, 2H), 2.60 - 2.53 (m, 1H), 2.38 - 2.24 (m, 3H), 2.17 - 1.93 (m, 3H), 1.89 - 1.77 (m, 1H), 1.77 - 1.70 (m, 2H), 1.70 - 1.57 (m, 3H), 1.39 (s, 3H), 1.05 - 0.92 (m, 1H), 0.91 (dd, *J =* 11.1, 3.4 Hz, 1H), 0.84 (s, 3H).

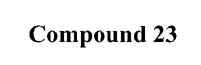

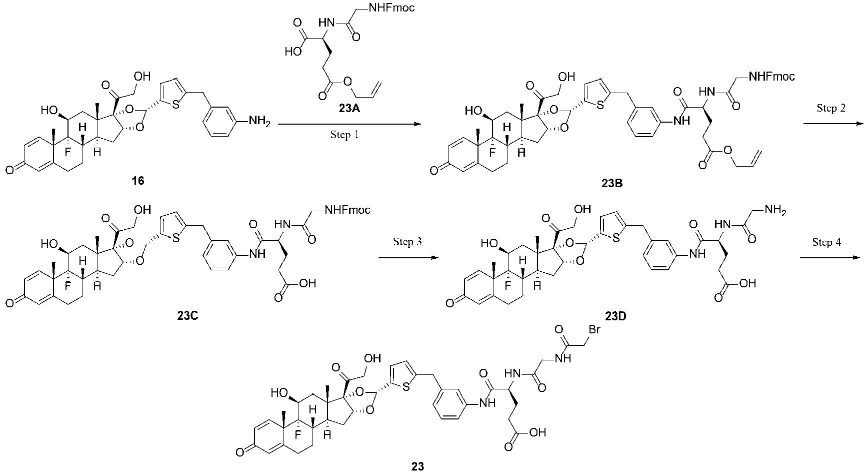

**Step 1** Compound **16** (540 mg, 0.91 mmol), compound **23A** (353 mg, 0.76 mmol) and 2,6-lutidine (121 mg, 1.14 mmol) were added to a three-necked flask (25 mL), DMF (4 mL) was added for dissolution, and HATU (316 mg, 0.833 mmol) was added. The mixed solution was stirred for 17 h. After the reaction was completed as detected by LCMS, the reaction solution was slowly added to water (80 mL), and a large amount of yellow solid was precipitated. The resulting mixture was filtered, and the filter cake was washed with water (80 mL) and lyophilized to give a yellow solid **23B** (700 mg, yield: 88%). MS-ESI: m/z 1042.4 [M+H]⁺.

**Step 2** Compound **23B** (700 mg, 0.67 mmol), N-methylmorpholine (679 mg, 6.7 mmol) and Pd(PPh₃)₄ (310 mg, 0.27 mmol) were added to a 25-mL three-necked flask, and THF (5 mL) was added for dissolution. The mixed solution was purged with nitrogen, cooled to 0 °C and reacted for 2 h. After the reaction was completed as detected by LCMS, the reaction solution was directly stirred with silica gel and purified by normal phase column chromatography (DCM/MeOH = 80/20) to give a yellow powder **23C** (450 mg, yield: 66%). MS-ESI: m/z 1002.3 [M+H]⁺.

**Step 3** Compound **23C** (450 mg) was dissolved in DCM (3 mL), diethylamine (1.5 mL) was added, and the mixed solution was stirred at room temperature for 2 h. After the reaction was completed as detected by LCMS, the reaction solution was added dropwise to PE (60 mL), and a large amount of yellow solid was precipitated. After the resulting mixture was left to stand for 5 min, the reaction solution was filtered to give a yellow powder (200 mg), which was purified by reversed-phase preparative chromatography to give a white solid **23D** (15 mg, yield: 4%). MS-ESI: m/z 780.3 [M+H]⁺.

**Step 4** Compound **23D** (15 mg, 0.019 mmol) was dissolved in THF (0.8 mL) and H₂O (0.2 mL). The resulting solution was cooled to 5 °C in an ice-water bath, and 2,6-lutidine (20.5 mg, 0.19 mmol) and bromoacetyl bromide (15.8 mg, 0.07 mmol) were added. The mixed solution was stirred for 5 min. After the reaction was completed as detected by LCMS, the reaction solution was purified by reversed-phase preparative chromatography to give a white powder 23 (6 mg, yield: 34%). MS-ESI: m/z 900.2 [M+H]⁺.

¹H NMR (400 MHz, DMSO-*d*₆) δ 9.93 (s, 1H), 8.55 - 8.48 (m, 1H), 8.24 (d, *J* = 7.8 Hz, 1H), 7.49 (d, *J* = 8.2 Hz, 1H), 7.44 (s, 1H), 7.29 (d, *J* = 10.1 Hz, 1H), 7.23 - 7.17 (m, 1H), 7.09 - 7.06 (m, 1H), 6.97 - 6.90 (m, 1H), 6.72 (d, *J* = 3.5 Hz, 1H), 6.24 (dd, *J* = 10.1, 1.9 Hz, 1H), 6.06 (s, 1H), 5.74 (s, 1H), 5.44 - 5.39 (m, 1H), 4.91 - 4.87 (m, 1H), 4.50 (d, *J* = 19.4 Hz, 1H), 4.42 - 4.36 (m, 1H), 4.17 (d, *J* = 19.5 Hz, 3H), 4.07 (s, 2H), 3.94 (s, 2H), 3.83 - 3.78 (m, 2H), 2.40 - 2.09 (m, 2H), 2.05 - 1.75 (m, 3H), 1.69 - 1.57 (m, 3H), 1.49 (s, 3H), 1.45 - 1.28 (m, 1H), 0.84 (s, 3H).

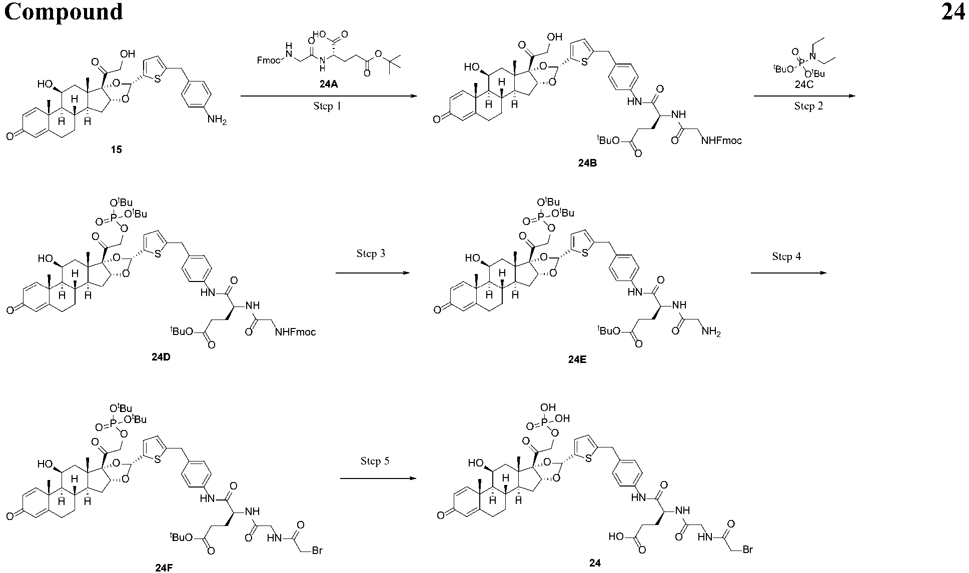

**Step 1** Compound **24A** (4.53 g, 9.38 mmol) was dissolved in pyridine (36.0 mL) and DMF (36.0 mL), **EDCI** (3.60 g, 18.8 mmol) was added, and compound 15 (4.53 g, 9.38 mmol) was added to the reaction solution at 0 °C. The resulting solution returned to room temperature and stirred for 1 h. After the starting materials were completely consumed as detected by LCMS, the mixture was poured into water (118 mL). The resulting mixture was diluted with ethyl acetate (118 mL) and extracted with ethyl acetate (100 mL, 50.0 mL). The organic phases were combined, washed with a saturated aqueous sodium chloride solution (30.0 mL), dried over anhydrous sodium sulfate, filtered and concentrated to give a yellow solid **24B** (crude, 6.50 g, with negligible yield of crude product). MS-ESI: m/z 1040.4 [M+H]⁺.

**Step 2** Compound **24B** (6.30 g, 6.06 mmol), tetrazole (4.24 g, 60.6 mmol, 5.37 mL) and compound **24C** (9.06 g, 36.3 mmol) were dissolved in DMF (44.0 mL). The mixed solution was stirred at 20 °C for 2.5 h, and the internal temperature was reduced to 0 °C. H₂O₂ (3.78 g, 33.3 mmol, 3.20 mL, 30% purity) was added to the reaction solution at 0 °C, and the resulting solution returned to room temperature and stirred for 1 h. After the starting materials were completely consumed as detected by LCMS, the mixture was poured into a saturated aqueous sodium sulfite solution (44.0 mL). The resulting mixture was diluted with water (88.0 mL) and extracted with ethyl acetate (100 mL, 50.0 mL, 30.0 mL). The organic phases were combined, washed with a saturated aqueous sodium chloride solution (20.0 mL), dried over anhydrous sodium sulfate, filtered and concentrated to give a yellow solid **24D** (crude, 7.46 g, with negligible yield of crude product). MS-ESI: m/z 1232.5 [M+H]⁺.

**Step 3** Compound **24D** (7.16 g, 5.81 mmol) and piperidine (4.20 g, 49.4 mmol, 4.88 mL) were dissolved in acetonitrile (40.0 mL), and the mixed solution was stirred at 20 °C for 1 h. After the starting materials were completely consumed as detected by LCMS, the reaction solution was concentrated under reduced pressure to remove acetonitrile. The residue after concentration was slurried with petroleum ether for 2 h and filtered, and the filter cake was dried under vacuum to give a yellow solid **24E** (6.37 g, yield: 77.7%), which was confirmed by LCMS (LCMS_ ET54964-22-p1b1). MS-ESI: m/z 1010.5 [M+H]⁺.

**Step 4** Bromoacetic acid (1.61 g, 11.6 mmol, 837 µL) and EEDQ (2.87 g, 11.6 mmol) were dissolved in DMF (41.0 mL), the mixed solution was stirred at 20 °C for 1 h, and compound **24E** (5.87 g, 5.81 mmol) was added to the reaction solution at 20 °C. The resulting solution was reacted at 20 °C for 2.5 h. After the starting materials were completely consumed as detected by LCMS, the reaction solution was diluted with dichloromethane (220 mL), washed twice with 1 N aqueous hydrobromic acid solution (160 mL × 2), washed once with a saturated aqueous sodium bicarbonate solution (110 mL), washed once with a saturated aqueous sodium chloride solution (50.0 mL), dried over anhydrous sodium sulfate, filtered and concentrated to give a yellow oily liquid **24F** (crude, 6.57 g, with negligible yield of crude product). MS-ESI: m/z 1130.3 [M+H]⁺.

**Step 5** Compound **24F** (6.57 g, 5.81 mmol) was dissolved in dichloromethane (50.0 mL), and TFA (25.0 mL) was added to the reaction solution at 20 °C. The resulting solution was stirred for 1 h. After the starting materials were completely consumed as detected by LCMS, the reaction solution was concentrated under reduced pressure to give a crude product, which was purified by reversed-phase preparative chromatography [water (NH₄HCO₃)-acetonitrile] to give a yellow solid **24** (355 mg, yield: 6.36%). MS-ESI: m/z 961.9 [M+H]⁺.

¹H NMR (400 MHz, DMSO-*d*₆) δ 11.65 (brs, 1H), 9.93 (s, 1H), 8.54 (t, *J =* 5.6 Hz, 1H), 8.32 - 8.24 (m, 1H), 7.52 (d, *J =* 8.5 Hz, 2H), 7.32 (d, *J =* 10.1 Hz, 1H), 7.15 (d, *J =* 8.6 Hz, 2H), 7.06 (d, *J* = 3.6 Hz, 1H), 6.70 (d, *J* = 3.5 Hz, 1H), 6.19 (dd, *J* = 10.1, 1.9 Hz, 1H), 5.99 (s, 1H), 5.81 (s, 1H), 4.94 - 4.84 (m, 2H), 4.56 (dd, *J* = 18.2, 7.9 Hz, 1H), 4.47 - 4.37 (m, 1H), 4.34 - 4.28 (m, 1H), 4.09 - 3.97 (m, 2H), 3.94 (s, 2H), 3.87 - 3.75 (m, 2H), 2.59 - 2.52 (m, 1H), 2.39 - 2.18 (m, 3H), 2.18 - 2.06 (m, 1H), 2.06 - 1.92 (m, 2H), 1.90 - 1.79 (m, 1H), 1.75 (s, 2H), 1.71 - 1.54 (m, 3H), 1.39 (s, 3H), 0.99 - 0.88 (m, 2H), 0.86 (s, 3H).

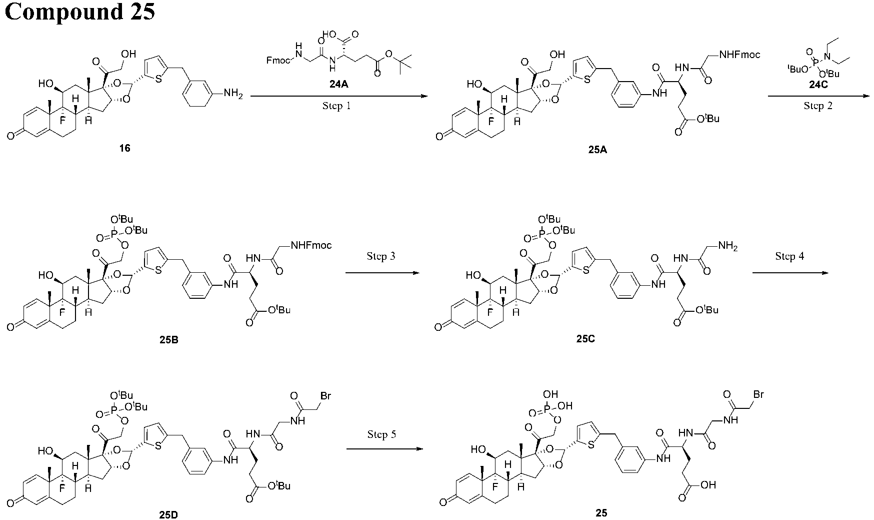

**Step 1** Compound **24A** (6.29 g, 13.0 mmol) was dissolved in pyridine (50.0 mL) and DMF (50.0 mL), EDCI (4.99 g, 26.0 mmol) was added to the reaction solution, and compound **16** (5.00 g, 8.68 mmol) was added to the reaction solution at 0 °C. The resulting solution returned to 10 °C to 20 °C and stirred for 12 h. After the starting materials were completely consumed as detected by LCMS, the reaction solution was poured into water (150 mL), and the resulting mixture was extracted with ethyl acetate (150 mL, 100 mL, 50.0 mL). The organic phases were combined, washed with a saturated aqueous sodium chloride solution (30.0 mL), dried over anhydrous sodium sulfate, filtered and concentrated to give a yellow solid **25A** (crude, 9.03 g, with negligible yield of crude product). MS-ESI: m/z 1058.4 [M+H]⁺.

**Step 2** Compound **25A** (9.03 g, 8.68 mmol), tetrazole (4.40 g, 62.8 mmol) and compound **24C** (13.0 g, 52.1 mmol) were dissolved in DMF (66.0 mL). The mixed solution was stirred at 20 °C for 2.5 h, and the internal temperature was reduced to 0 °C. H₂O₂ (3.93 g, 34.7 mmol, 3.33 mL, 30% purity) was added to the reaction solution at 0 °C, and the resulting solution returned to 10 °C to 20 °C and stirred for 1 h. After the starting materials were completely consumed as detected by LCMS, the mixture was poured into a saturated aqueous sodium sulfite solution (66.0 mL). The resulting mixture was diluted with water (132 mL) and extracted with ethyl acetate (200 mL, 100 mL). The organic phases were combined, washed with a saturated sodium chloride solution (50.0 mL), dried over anhydrous sodium sulfate, filtered and concentrated to give a yellow solid **25B** (crude, 10.7 g, with negligible yield of crude product). MS-ESI: m/z 1250.5 [M+H]⁺.

**Step 3** Compound **25B** (10.7 g, 8.68 mmol) and piperidine (6.28 g, 73.8 mmol, 7.29 mL) were dissolved in acetonitrile (60.0 mL), and the mixed solution was stirred at 20 °C for 1 h. After the starting materials were completely consumed as detected by LCMS, the reaction solution was concentrated under reduced pressure to remove acetonitrile. The residue after concentration was slurried with petroleum ether (100 mL) for 2 h and filtered, and the filter cake was dried under vacuum to give a yellow solid **25C** (8.77 g, yield: 83.8%). MS-ESI: m/z 1028.4 [M+H]⁺.

**Step 4** Bromoacetic acid (2.48 g, 17.8 mmol) and EEDQ (4.41 g, 17.8 mmol) were dissolved in DMF (63.0 mL), and the resulting solution was stirred at 20 °C for 1 h. Compound **25C** (9.00 g, 8.91 mmol) was added to the reaction solution at 20 °C, and the resulting solution was reacted at 20 °C for 2.5 h. After the starting materials were completely consumed as detected by LCMS, the reaction solution was diluted with dichloromethane (220 mL), washed twice with 1 N hydrobromic acid (160 mL × 2), washed once with a saturated aqueous sodium bicarbonate solution (110 mL), washed once with a saturated aqueous sodium chloride solution (50.0 mL) and dried over anhydrous sodium sulfate. The organic phase was concentrated to dryness to give a yellow oily liquid **25D** (crude, 10.1 g, with negligible yield of crude product). MS-ESI: m/z 1148.4 [M+H]⁺.

**Step 5** Compound **25D** (10.1 g, 8.91 mmol) was dissolved in dichloromethane (80.0 mL), and TFA (40.0 mL) was added to the reaction solution at 20 °C. The resulting solution was stirred for 1 h. After there were 54.7% of the product as detected by LCMS, the reaction solution was concentrated under reduced pressure to give a crude product, which was purified by reversed-phase preparative chromatography [water (TFA)-acetonitrile] to give a yellow solid 25 (500 mg, yield: 8.58%, purity: 93.4%).

MS-ESI: m/z 980.0 [M+H]⁺.

¹H NMR (400 MHz, DMSO-*d*₆) δ 11.66 (s, 2H), 9.93 (s, 1H), 8.51 (t, *J* = 5.7 Hz, 1H), 8.24 (d, *J* = 7.8 Hz, 1H), 7.50 (d, *J =* 8.3 Hz, 1H), 7.46 - 7.40 (m, 1H), 7.30 (d, *J* = 10.2 Hz, 1H), 7.20 (t, *J =* 7.8 Hz, 1H), 7.07 (d, *J* = 3.5 Hz, 1H), 6.93 (d, *J =* 7.6 Hz, 1H), 6.73 (d, *J* = 3.5 Hz, 1H), 6.25 (dd, *J* = 10.1, 1.9 Hz, 1H), 6.06 (s, 1H), 5.83 (s, 1H), 5.51 (d, *J* = 4.5 Hz, 1H), 4.96 - 4.84 (m, 2H), 4.57 (dd, *J* = 18.2, 8.1 Hz, 1H), 4.44 - 4.35 (m, 1H), 4.23 - 4.16 (m, 1H), 4.07 (s, 2H), 3.94 (s, 2H), 3.88 - 3.73 (m, 2H), 2.70 - 2.54 (m, 2H), 2.40 - 2.32 (m, 1H), 2.32 - 2.22 (m, 2H), 2.22 - 2.09 (m, 1H), 2.06 - 1.91 (m, 2H), 1.90 - 1.76 (m, 3H), 1.71 - 1.57 (m, 3H), 1.49 (s, 4H), 1.44 - 1.29 (m, 1H), 0.87 (s, 3H).

### 2.2 Preparation of antibody-drug conjugates targeting BDCA2

In the present application, an aqueous buffer of 20 mM histidine (pH = 5.5) was obtained by adjusting the pH of an aqueous 20 mM Histidine solution to 5.5 with HOAc. Stock solution **1:** 10 mM EDTA buffer, pH = 4.7; stock solution **2**: 800 mM aqueous sodium citrate buffer, pH = 8.4; stock solution **3:** 500 mM aqueous urea solution; stock solution **4:** 10 mM aqueous tris(2-carboxyethyl)phosphine solution.

The protein purity of the ADCs in the present application was detected by the SEC method, and the parameters are shown below.

| Instrument | Agilent Technologies 1260 | |
|---|---|---|
| Chromatography column | TOSON - G3000SWXL, 300*7.8 mm, 5 µm | |
| Column temperature | 22 °C | |
| Wavelength | 280 nm | |
| Injection volume | 30 ~ 50 µg | |
| Mobile phase | 0.2 M K₂HPO₄/KH₂PO₄, 0.25 M KCl, 15%(v/v) 2-propanol, pH 7.0 | |

| | Time (min) | Flow rate (mL/min) |
|---|---|---|
| Gradient | 0.0 | 0.75 |
| | 18.0 | 0.75 |

The DAR value of the ADC in the present application can be detected by LC-MS method. The experimental procedure was as follows: To 40.0 µg of an ADC sample solution were added 75.0 µL of 8.0 mol/L guanidine hydrochloride (Gdn-HCl), 5.0 µL of 1.0 mol/L Tris-HCl, and 2.0 µL of 1 mol/L DTT. The solution was diluted to 100.0 µL with ultrapure water, mixed well, and incubated at 22 °C for 30 min. Drug/antibody ratio (DAR) was then analyzed using LC-MS. The LC parameters are shown below.

| Instrument | Agilent Technologies 1260 |
|---|---|
| Reversed-phase chromatography column | Agilent,PLRP-S 2.1*50 mm,8 µm |
| Column temperature | 80 °C |
| Sample temperature | 2~8 °C |
| Detection wavelength | 280 nm |
| Injection volume | 10.0 µL |

| Mobile phase | Mobile phase A: 0.025% TFA and 0.1% FA in ultrapure water | | | |
|---|---|---|---|---|
| | Mobile phase B: 0.025% TFA and 0.1% FA in ACN | | | |
| Gradient | Time (min) | %A | %B | Flow rate (mL/min) |
| | 0.0 | 75 | 25 | 0.5 |
| | 0.0 | 75 | 25 | |
| | 0.7 | 66 | 34 | |
| | 5.0 | 55 | 45 | |
| | 6.0 | 10 | 90 | |
| | 7.0 | 10 | 90 | |
| | 7.1 | 75 | 25 | |
| | 10.0 | 75 | 25 | |

The MS parameters are shown below.

| Instrument | Agilent Technologies 6224 TOF LC/MS |
|---|---|
| Ionization mode | Positive |
| Gas temperature | 350 °C |
| Air flow | 13 L/min |
| Atomizer pressure | 45 psig |
| Capillary voltage | 5000 V |
| Crushing voltage | 250 V |
| Voltage of taper hole | 65 V |
| Peak value of radio frequency voltage on octupole | 750 V |
| Acquisition mode | MS (seg) |
| Mass range | 500-8000 m/z |
| Acquisition rate | 1 spectra/s |
| Acquisition time | 1.4-7.0 min |

The DAR value of the ADC in the present application can also be detected by HIC method, and the parameters are shown below.

| | | | |
|---|---|---|---|
| Chromatography column | TSKgel Butyl-NPR, 4.6 mm *3.5 cm, 2.5 µm | | |
| Column temperature | 25 °C | | |
| Detection wavelength | 280 nm | | |
| Sample load | 8 µL | | |
| Mobile phase | Mobile phase A: 1.5 M (NH₄)₂SO₄, 0.05 M K₂HPO₄.3H₂O (pH = 7.0) | | |
| | Mobile phase B: 21.3 mM KH₂PO₄, 28.6 mM K₂HPO₄.3H₂O, 25% (V/V) IPA (pH = 7.0) | | |
| Gradient | Time (min) | % B | Flow rate (mL/min) |
| | 0.0 | 0.0 | 0.6 |
| | 2.0 | 0.0 | |
| | 15.0 | 100.0 | |
| | 16.0 | 100.0 | |
| | 17.0 | 0.0 | |
| | 20.0 | 0.0 | |

Hu033-03, Hu033-20, and Hu005-04 are humanized anti-BDCA2 antibodies, the antibody subtype of which is IgG1. Conjugate construction of the immunomodulatory antibody-drug conjugate directed against BDCA2 was performed using Hu033-03, Hu033-20 and Hu005-04. Conjugation of Hu033-03 antibody-drug conjugate (Hu033-03-ADC)

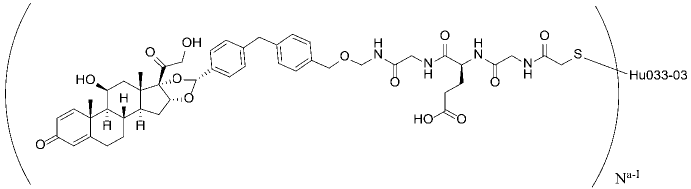

To an aqueous buffer (0.02 M aqueous histidine buffer at pH = 6.0; 1.88 mL, 8.0 mg/mL, 0.101 µmol) of the antibody Hu033-03 was added the prepared aqueous solution of tris(2-carboxyethyl)phosphine (10 mM, 0.028 mL, 0.283 µmol) at 22 °C, and the reaction solution was placed in a water bath shaker, shaken and reacted at 22 °C for 3 h before the reaction was stopped.

Compound 9 (1.5 mg, 1.52 µmol) was dissolved in 0.15 mL of DMSO, the resulting solution was added to the above solution, and the mixed solution was placed in the water bath shaker, shaken and reacted at 22 °C for 2 h before the reaction was stopped. 1/3 volumes of 100 mM aqueous histidine buffer (pH = 5.5) were added to the reaction solution, and the mixed solution was desalted and purified by using a Zeba desalting spin column (40K MWCO) (eluent: 0.02 M aqueous histidine buffer at pH 5.5) to give antibody-drug conjugate Hu033-03-ADC (11 mg, 3.27 mg/mL, yield: 73%).

N^{a-I} was found to be 4.01 as detected by HIC.

Conjugation of Hu033-20 antibody-drug conjugate (Hu033-20-ADC)

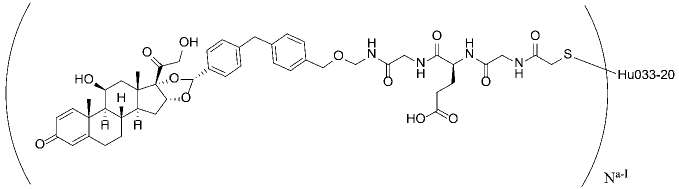

To an aqueous PBS buffer (0.05 M aqueous PBS buffer at pH = 7.4; 1.88 mL, 8.0 mg/mL, 0.101 µmol) of the antibody Hu033-20 was added the prepared aqueous solution of tris(2-carboxyethyl)phosphine (10 mM, 0.030 mL, 0.303 µmol) at 22 °C, and the reaction solution was placed in a water bath shaker, shaken and reacted at 22 °C for 3 h before the reaction was stopped.

Compound 9 (1.5 mg, 1.52 µmol) was dissolved in 0.15 mL of DMSO, the resulting solution was added to the above solution, and the mixed solution was placed in the water bath shaker, shaken and reacted at 22 °C for 2 h before the reaction was stopped. The reaction solution was desalted and purified (eluent: 0.02 M aqueous histidine buffer at pH 5.5) using a Zeba desalting spin column (40K MWCO) to give antibody-drug conjugate Hu033-20-ADC (12.5 mg, 4.28 mg/mL, yield: 83%).

N^{a-I} was found to be 4.46 as detected by HIC.

Conjugation of Hu005-04 antibody-drug conjugate (Hu005-04-ADC)

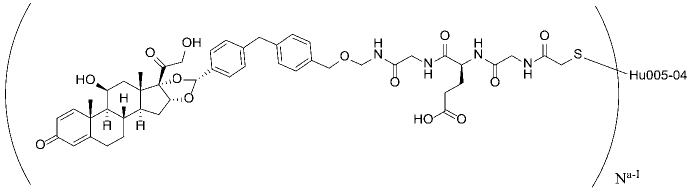

To an aqueous PBS buffer (0.05 M aqueous PBS buffer at pH = 7.4; 1.88 mL, 8.0 mg/mL, 0.101 µmol) of the antibody Hu005-04 was added the prepared aqueous solution of tris(2-carboxyethyl)phosphine (10 mM, 0.028 mL, 0.283 µmol) at 22 °C, and the reaction solution was placed in a water bath shaker, shaken and reacted at 22 °C for 3 h before the reaction was stopped.

Compound 9 (1.5 mg, 1.52 µmol) was dissolved in 0.15 mL of DMSO, the resulting solution was added to the above solution, and the mixed solution was placed in the water bath shaker, shaken and reacted at 22 °C for 2 h before the reaction was stopped. The reaction solution was desalted and purified (eluent: 0.02 M aqueous histidine buffer at pH 5.5) using a Zeba desalting spin column (40K MWCO) to give antibody-drug conjugate Hu005-04-ADC (9 mg, 3.27 mg/mL, yield: 60%).

N^{a-I} was found to be 4.34 as detected by HIC.

Control ADC (BIIB059-ADC)

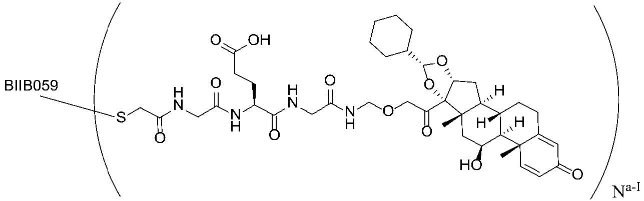

Reference was made to Preparation Example 1.9 in WO2022171101A1 for the preparation of the control ADC, in which the antibody was BIIB059.

### Example 3. Inhibition of Activity of Human Peripheral Blood Mononuclear Cells by Antibody-Drug Conjugates Targeting BDCA2

Inhibition of CpG-A-induced production of IFNα and TNFα from human peripheral blood mononuclear cells by BDCA2-ADC

### Objective

The inhibitory effects of the antibody-drug conjugate molecules of the present application on the production from peripheral blood mononuclear cells were detected. Peripheral blood mononuclear cells produce a large number of cytokines after the stimulation of the TLR9 agonist CpG-A. The peripheral blood mononuclear cells were treated *in vitro* with the BDCA2 antibody-drug conjugate at different concentrations, and IFNα and TNFα produced by the peripheral blood mononuclear cells were quantitatively detected after the stimulation of 0.5 µM CpG-A for a certain period of time. The *in vitro* activity of the ligand-drug conjugates was evaluated based on the extent of cytokine inhibition.

### Experiment process

The cryopreserved human peripheral blood mononuclear cells were thawed, resuspended in a complete culture medium and counted, and the cell suspension was adjusted to 2.5 × 10^6 cells/mL with the culture medium and cultured overnight. After the suspension was centrifuged to collect the cells the next day, the cells were resuspended using fresh culture medium. The test compound was diluted to 4-fold final concentration in cell culture medium, 50 µL of the compound was added into a 96-well cell plate, and 100 µL of diluted cells (800,000 cells/well) were added into each well of the 96-well plate containing the compound, followed by addition of 50 µL CpG-A to a final concentration of 0.5 µM. The cell plate was incubated at 37 °C with 5% CO₂ for 24 h. The supernatant was collected, and the TNF-α and IFN-α concentration levels in the supernatant were detected using an ELISA kit. Dose-response data were fitted to an S-shaped curve using non-linear regression, and IC₅₀ values were calculated.

TNF-α concentration assay was performed using a Dakewe TNF-α ELISA kit (Cat. No. 1117202). 100 µL of serially diluted cytokine standard was added to each standard well, 100 µL of 10-fold diluted test sample was added to each sample well, and 100 µL of 1× dilution buffer R was added to each blank control well. 50 µL of biotinylated antibody working solution was added to each well. After mixing well, the plate was covered with a plate-sealing film and incubated at room temperature for 3 h. The plate was washed 3 times: the liquid in each well was removed, and 300 µL of 1× washing buffer working solution was added to each well; after 1 minute of residence, the liquid in each well was discarded. This procedure was repeated 3 times. 100 µL of streptavidin-HRP working solution was added to each well. The plate was covered with a plate-sealing film and incubated at room temperature for 20 min. The plate was washed 3 times, 100 µL of TMB liquid was added to each well, and the plate was incubated at room temperature (18-25 °C) in the dark for 25 min. The reaction was terminated by quickly adding a stop solution at 100 µL/well. The reading at wavelength 450 nm was detected using an M2e instrument within 10 min after the termination.

IFN-α concentration assay was performed using a Dakewe IFN-α ELISA kit (Cat. No. 1110012). 100 µL of serially diluted cytokine standard was added to each standard well, 100 µL of 2-fold diluted test sample was added to each sample well, and 100 µL of 1× dilution buffer R was added to each blank control well. 50 µL of biotinylated antibody working solution was added to each well. After mixing well, the plate was covered with a plate-sealing film and incubated at room temperature for 3 h. The plate was washed 3 times: the liquid in each well was removed, and 300 µL of 1× washing buffer working solution was added to each well; and after 1 minute of residence, the liquid in each well was discarded. This procedure was repeated 3 times. 100 µL of streptavidin-HRP working solution was added to each well. The plate was covered with a plate-sealing film and incubated at room temperature for 20 min. The plate was washed 3 times, 100 µL of TMB liquid was added to each well, and the plate was incubated at room temperature (18-25 °C) in the dark for 25 min. The reaction was stopped by quickly adding a stop solution at 100 µL/well. The reading at wavelength 450 nm was detected using an M2e instrument within 10 min after the termination.

The analysis was performed using GraphPad Prism software. OD450 values were converted to TNF-α and IFN-α contents, and compared to the reference therapeutic antibody BIIB059 for inhibition of cytokine secretion. Isotype was used as a negative control antibody. The results are shown in FIGs. 5a and 5b.

The results show that compared to the reference therapeutic antibody BIIB059, the BDCA2 antibody-drug conjugates of the present application had a stronger ability to inhibit IFN-α secretion from peripheral blood mononuclear cells, and achieved complete inhibition of IFN-α secretion at high concentrations. The antibody-drug conjugates showed a dose-dependent inhibitory effect on TNF-α secretion, while BIIB059 did not have an inhibitory effect on TNF-α secretion. The BDCA2 antibody-drug conjugates of the present application had a stronger IFN-α inhibitory ability and a broader inflammatory factor inhibitory effect, and had a stronger treatment effect on systemic lupus erythematosus or other related autoimmune diseases compared to the therapeutic antibody BIIB059.

Inhibition of R848-induced production of IFN-α and TNF-α from human peripheral blood mononuclear cells by BDCA2-ADC

### Objective

The inhibitory effects of the antibody-drug conjugate molecules of the present application on the production from peripheral blood mononuclear cells were detected. Peripheral blood mononuclear cells produce a large number of cytokines after the stimulation of the TLR7/8 agonist R848. The peripheral blood mononuclear cells were treated *in vitro* with the BDCA2 antibody-drug conjugate at different concentrations, and IFNα and TNFα produced by the peripheral blood mononuclear cells were quantitatively detected after the stimulation of 5 µM R848 for a certain period of time. The *in vitro* activity of the ligand-drug conjugates was evaluated based on the extent of cytokine inhibition.

### Experiment process

The cryopreserved human peripheral blood mononuclear cells were thawed, resuspended in a complete culture medium and counted, and the cell suspension was adjusted to 2.5 × 10^6 cells/mL with the culture medium and cultured overnight. After the suspension was centrifuged to collect the cells the next day, the cells were resuspended using fresh culture medium. The test compound was diluted to 4-fold final concentration in cell culture medium, 50 µL of the compound was added into a 96-well cell plate, and 100 µL of diluted cells (800,000 cells/well) were added into each well of the 96-well plate containing the compound, followed by addition of 50 µL R848 to a final concentration of 5 µM. The cell plate was incubated at 37 °C with 5% CO₂ for 24 h. The supernatant was collected, and the TNF-α and IFN-α concentration levels in the supernatant were detected using an ELISA kit. Dose-response data were fitted to an S-shaped curve using non-linear regression, and IC50 values were calculated.

TNF-α concentration assay was performed using a Dakewe TNF-α ELISA kit (Cat. No. 1117202). 100 µL of serially diluted cytokine standard was added to each standard well, 100 µL of 10-fold diluted test sample was added to each sample well, and 100 µL of 1× dilution buffer R was added to each blank control well. 50 µL of biotinylated antibody working solution was added to each well. After mixing well, the plate was covered with a plate-sealing film and incubated at room temperature for 3 h. The plate was washed 3 times: the liquid in each well was removed, and 300 µL of 1× washing buffer working solution was added to each well; after 1 minute of residence, the liquid in each well was discarded. This procedure was repeated 3 times. 100 µL of streptavidin-HRP working solution was added to each well. The plate was covered with a plate-sealing film and incubated at room temperature for 20 min. The plate was washed 3 times, 100 µL of TMB liquid was added to each well, and the plate was incubated at room temperature (18-25 °C) in the dark for 25 min. The reaction was terminated by quickly adding a stop solution at 100 µL/well. The reading at wavelength 450 nm was detected using an M2e instrument within 10 min after the termination.

IFN-α concentration assay was performed using a Dakewe IFN-α ELISA kit (Cat. No. 1110012). 100 µL of serially diluted cytokine standard was added to each standard well, 100 µL of 2-fold diluted test sample was added to each sample well, and 100 µL of 1× dilution buffer R was added to each blank control well. 50 µL of biotinylated antibody working solution was added to each well. After mixing well, the plate was covered with a plate-sealing film and incubated at room temperature for 3 h. The plate was washed 3 times: the liquid in each well was removed, and 300 µL of 1× washing buffer working solution was added to each well; and after 1 minute of residence, the liquid in each well was discarded. This procedure was repeated 3 times. 100 µL of streptavidin-HRP working solution was added to each well. The plate was covered with a plate-sealing film and incubated at room temperature for 20 min. The plate was washed 3 times, 100 µL of TMB liquid was added to each well, and the plate was incubated at room temperature (18-25 °C) in the dark for 25 min. The reaction was stopped by quickly adding a stop solution at 100 µL/well. The reading at wavelength 450 nm was detected using an M2e instrument within 10 min after the termination.

The analysis was performed using GraphPad Prism software, and OD450 values were converted to TNF-α and IFN-α contents.

### a) Inhibition of cytokine secretion compared to the reference antibody BIIB059.

The results are shown in FIGs. 6a and 6b.

The results show that compared to the reference antibody BIIB059, the BDCA2 antibody-drug conjugates of the present application had a stronger ability to inhibit IFN-α secretion from peripheral blood mononuclear cells, and a higher inhibitory effect on IFN-α secretion at equivalent effective concentrations. BIIB059 did not have an inhibitory effect on TNF-α secretion. However, BDCA2 antibody-drug conjugates of the present application exhibited a dose-dependent inhibitory effect on TNF-α secretion. The BDCA2 antibody-drug conjugates of the present application had a stronger IFN-α/TNF-α inhibitory ability and a broader inflammatory factor inhibitory effect, and had a stronger treatment effect on autoimmune diseases compared to the antibody BIIB059.

### b) Inhibition of cytokine secretion compared to control ADC.

The results are shown in FIGs. 6c-6f.

The results show that the BDCA2 antibody-drug conjugate Hu033-03-ADC of the present application had a stronger ability to inhibit IFN-α secretion from peripheral blood mononuclear cells, which was significantly superior to that of the control ADC. The IC50 values of Hu033-03-ADC and the control ADC were 0.2801 and 1.718, respectively. The BDCA2 antibody-drug conjugate Hu033-03-ADC of the present application had a stronger ability to inhibit TNF-α secretion from peripheral blood mononuclear cells, which was significantly superior to that of the control ADC. The IC₅₀ values of Hu033-03-ADC and the control ADC were 20.86 and 2040, respectively. The control ADC did not have an inhibitory effect on TNF-α secretion. The BDCA2 antibody-drug conjugates of the present application had a stronger IFN-α/TNF-α inhibitory ability and a broader inflammatory factor inhibitory effect, and had a stronger treatment effect on autoimmune diseases compared to the control ADC.

### Example 4. Binding of Antibody-Drug Conjugates Targeting BDCA2 to BDCA2 Antigen

The inhibitory effect of the BDCA2 antibody-drug conjugate on the function of human peripheral blood mononuclear cells was investigated, and Hu033-03-ADC was selected for subsequent development and verification.

CHOK1-human BDCA2 and 293F-cynoBDCA2 cells cultured to the logarithmic growth phase were digested with TrypLE trypsin. The cells were collected and resuspended to 2 × 10^6 cells/mL in an FACS buffer (PBS + 2% FBS). The resuspended cells were added to a 3799 cell plate at 100 µL/well and centrifuged at 300 g, and the supernatant was discarded. The test sample was diluted in a 5-fold gradient (with final concentrations of 0.0025 nM-200 nM) with the FACS buffer, and the diluted test sample was added to the centrifuged cells. The cells were resuspended and incubated at 4 °C for 1 h. The incubated cells were centrifuged at 300 g for 5 min and washed twice with the FACS buffer. 100 µL of Goat anti-Human IgG (H+L) Cross-Adsorbed Secondary Antibody, Alexa Fluor 488 (Sigma, A11013; 1:1000) secondary antibody was added. The cells were incubated at 4 °C for 1 h, washed twice with the FACS buffer, and resuspended in PBS. The fluorescence signals of the antibodies for binding to the cell surface were analyzed using an FACS (BD FACS CantoTM II) instrument, curves were fitted using GraphPad Prism6, and EC50 values of the antibodies or antibody-drug conjugates for binding to the BDCA2 antigen were calculated. The BIIB059 antibody was selected as a positive control, and hIgG1(ISO) was selected as a negative control antibody.

The binding activity of the BDCA2 antibody-drug conjugate of the present application to CHOK1-humanBDCA2 and 293F-cynoBDCA2 cells was superior to that of the positive control antibody BIIB059. The results are shown in Table 6 and FIGs. 7a/7b.

**Table 6. Binding activity of BDCA2 antibody-drug conjugate to BDCA2**

| No. | CHOKl-hBDCA2 EC₅₀ (nM) | 293F-cynoBDCA2 EC₅₀ (nM) |
|---|---|---|
| BIIB059 | 3.290 | 3.046 |
| hIgG1 | - | - |
| Hu033-03 | 1.233 | 1.905 |
| Hu033-03-ADC | 1.604 | 2.364 |

### Example 5. Internalization Assay of Antibody-Drug Conjugate Targeting BDCA2

CHOK1-humanBDCA2 cells cultured to the logarithmic growth phase were digested with TrypLE trypsin. The cells were collected and resuspended to 2 × 10^6 cells/mL in an FACS buffer (PBS + 2% FBS). The cells were added to a 3799 cell plate at 100 µL/well and centrifuged at 300 g, and the supernatant was discarded. The cells were placed in an ice box and cooled. The test sample was serially diluted with an FACS buffer (3 concentrations were set: 100 nM, 10 nM, and 1 nM; and for each concentration, 5 groups were set: 0 h; 4 °C for 2 h; 37 °C for 2 h; 4 °C for 4 h; and 37 °C for 4 h). After the dilution, the test sample was placed in an ice box and cooled for 15 min. After 15 min, the test sample was added to the centrifuged cells, and the cells were resuspended and incubated at 4 °C for 40 min. The incubated cells were centrifuged at 300 g for 5 min and washed twice with the FACS buffer. The cells in the 0 h group were immobilized with paraformaldehyde at room temperature for 10 min, and washed twice with the FACS buffer. The cells in the other two groups were resuspended in 100 µL of the FACS buffer and separately incubated at 4 °C and 37 °C for 1 h and for 4 h. The cells in the two groups incubated at 4 °C and 37 °C were immobilized with paraformaldehyde at room temperature for 10 min, and washed twice with the FACS buffer. 100 µL of the secondary antibody, Goat anti-Human IgG (H+L) Cross-Absorbed Secondary Antibody-Alexa Fluor 488 (Sigma, A11013; 1:1000) was separately added to the cells in the 3 groups, and the cells were incubated at 4 °C for 1 h. The cells were washed twice with the FACS buffer, resuspended in PBS and analyzed using the FACS instrument. The surface signal percentages were calculated. The results are shown in FIGs. 8A-8f. The antibody-drug conjugate showed more cell surface binding at concentrations of 1 nM and 10 nM and more internalization into cells at the same concentration compared to BIIB059.

### Example 6. Bystander Killing Effect Assay of Immunomodulatory Antibody-Drug Conjugate Targeting BDCA2

HEK293 cells cultured to the logarithmic growth phase were digested with TrypLE trypsin. The cells were collected, and the cell concentration was adjusted to 2 × 10⁵ cells/mL using DMEM + 10% FBS. 30 mL of the cells were seeded into a culture flask, and 1.5 mL of a transfection reagent was prepared according to the table doses.

| Reagent | Volume (µL) |
|---|---|
| pGL5 (100 ng/uL) | 75 |
| pBIND-GR (100 ng/uL) | 75 |
| Opti-MEM | 750.0 |
| P3000 | 30.00 |
| Opti-MEM | 750.0 |
| Lipofectamine | 22.5 |

1.5 mL of the transfection reagent was mixed with 30 mL of the cells, and after 24 h of culture, GR reporter gene cells were collected. The GR reporter gene cells obtained by transfection were mixed with HEK293-humanBDCA2 cells or HEK293 cells at a ratio of 1:1 and added to a 96-well culture plate at 2 × 10⁴ cells/well. The test reagent, positive control (dexamethasone, compound 4 in Example 2) and negative control (isotype IgG-ADC) were added at concentrations of 0.01-100 nM as designed for the experiment. After 24 h of incubation, the Promega Dual-Glo Luciferase Assay System detection reagent was added according to the manufacturer's instructions, and the Firefly and Renilla fluorescence signals were detected by Enspire reader. The glucocorticoid receptor signal produced by the bystander killing effect of the conjugate was calculated by dividing the Firefly signal by the Renilla signal.

The results are shown in FIGs. 9a and 9b. Compared to the isotype control antibody conjugate, the BDCA2 antibody-drug conjugate Hu033-03-ADC specifically activated GR reporter gene cells in the presence of BDCA2 expression, showing a bystander killing effect.

### Example 7. Inhibitory Effect of BDCA2 Immunomodulatory Antibody-Drug Conjugate on pDC Function Shown in Cynomolgus Monkeys

Based on the mechanism of action of the BDCA2 target and the drug effect of the ADC, the pDC phenotype in cynomolgus monkeys after injection of the BDCA2 antibody-drug conjugate and the expression level of IFNα *in vitro* stimulation experiment were tested to measure the biological activity of the antibody-drug conjugate (EMBO Mol Med (2015) 7: 464-476).

Nine female cynomolgus monkeys were divided into 3 groups, 3 cynomolgus monkeys per group. A blank control (solvent control, group 1, cynomolgus monkeys 1, 8, and 15), the antibody-drug conjugate Hu033-03-ADC at a dose of 10 mg/kg (group 2, cynomolgus monkeys 5, 7, and 12), and the antibody BIIB059 at a dose of 10 mg/kg (group 3, cynomolgus monkeys 3, 4, and 10) were separately injected intravenously into cynomolgus monkeys in each group. For each group, whole blood was collected at -28 d, -21 d, -10 d, 0 h, 2 h, 24 h, 48 h, 72 h, 96 h, 168 h, 240 h, 336 h, 504 h, 672 h, 840 h, 1008 h, 1176 h, 1344 h, 1512 h, and 1680 h, analyzed by flow cytometry, and subjected to *in vitro* stimulation experiments. For groups 2 and 3, serum and plasma were also collected in addition to whole blood collection, and all antibody serum and ADC concentrations were detected by ELISA.

The pDC percentage in the peripheral blood and the BDCA2 expression level on the surface of pDCs were detected by FACS.

Venous blood of the cynomolgus monkeys was collected by using a heparin anticoagulation tube, 6 mL of red blood cell lysis buffer was added to 300 µL of the blood, and the resulting mixture was placed at room temperature for 10 min. The cells were collected by centrifugation at 400 g for 5 min at room temperature. The cells were washed with PBS and resuspended in 100 µL of cell staining buffer (PBS + 1% FBS). 5 µL of Fc Block was added to the cell suspension and left to stand at 4 °C for 10 min. To the cell suspension were added Brilliant Violet 421^{™} anti-human HLA-DR antibody (Biolegend, 307636), APC/Cyanine7 anti-human CD20 antibody (Biolegend, 302314), APC/Cyanine7 anti-human CD14 antibody (Biolegend, 301820), PE Mouse Anti-Human CD123 antibody (BD, 554529), and AF647-coupled hu033-03 antibody sequentially, and the cells were incubated at 4 °C for 40 min. The cells were washed twice with PBS, resuspended in 200 µL of the cell staining buffer, and analyzed by using a flow cytometer.

Cytokine expression was detected by whole blood stimulation *in vitro.*

Venous blood of the cynomolgus monkeys was collected by using a heparin anticoagulation tube, added to a 96-well plate at 150 µL blood/well, and 100 µL of CpG-A was added to a final concentration of 200 µg/mL. The cells were cultured at 37 °C for 18 h, and the supernatant was collected. The TNF-α and IFN-α concentration levels in the supernatant were detected using an ELISA kit.

TNF-α concentration assay was performed using a R&D Primate TNF-α DuoSet ELISA kit (Cat. No. DY1070). 50 µL of serially diluted cytokine standard was added to each standard well, 50 µL of test sample was added to each sample well, and 50 µL of PBS was added to each blank control well. The cells were incubated at 37 °C for 2 h. The plate was washed 3 times: the liquid in each well was removed, and 300 µL of 1× PBST buffer working solution was added to each well; and after 1 minute of residence, the liquid in each well was discarded. 100 µL of detection antibody working solution was added to each well, and the cells were incubated at 37 °C for 1 h. The plate was washed 3 times, the liquid in each well was removed, and 300 µL of 1× PBST working solution was added to each well; and after 1 minute of residence, the liquid in each well was discarded. This procedure was repeated 3 times. 100 µL of streptavidin-HRP working solution was added to each well. The plate was covered with a plate-sealing film and incubated at 37 °C for 0.5 h. The plate was washed 3 times, 100 µL of TMB liquid was added to each well, and the plate was incubated at room temperature (18-25 °C) in the dark for 5 min. The reaction was stopped by quickly adding 1 M hydrochloric acid at 50 µL/well. The reading at wavelength 450 nm was detected using a Molecular device spectra max plus384 instrument within 10 min after the reaction was stopped.

IFN-α concentration assay was performed using a Mabtech Human IFN-α (pan specific) ELISA kit (Cat. No. 3425-1H-20). 50 µL of serially diluted cytokine standard was added to each standard well, 50 µL of test sample was added to each sample well, and 50 µL of PBS was added to each blank control well. The cells were incubated at 37 °C for 2 h. The plate was washed 3 times, the liquid in each well was removed, and 300 µL of 1× PBST buffer working solution was added to each well; and after 1 minute of residence, the liquid in each well was discarded. 100 µL of detection antibody working solution was added to each well, and the cells were incubated at 37 °C for 1 h. The plate was washed 3 times, the liquid in each well was removed, and 300 µL of 1× PBST working solution was added to each well; and after 1 minute of residence, the liquid in each well was discarded. This procedure was repeated 3 times. 100 µL of streptavidin-HRP working solution was added to each well. The plate was covered with a plate-sealing film and incubated at 37 °C for 0.5 h. The plate was washed 3 times, 100 µL of TMB liquid was added to each well, and the plate was incubated at room temperature (18-25 °C) in the dark for 5 min. The reaction was stopped by quickly adding 1 M hydrochloric acid at 50 µL/well. The reading at wavelength 450 nm was detected using a Molecular device spectra max plus384 instrument within 10 min after the reaction was stopped.

The results are shown in FIGs. 10-13.

Based on FIGs. 10 and 11, there was no significant change in the pDC percentage in the blood of the antibody-drug conjugate group or the BIIB059 group compared to the blank control. In addition, the BDCA2 level on the surface of pDCs in the blood of the antibody-drug conjugate group or the BIIB059 group was reduced compared to the blank control, and the BDCA2 reduction levels in the antibody-drug conjugate group and the BIIB059 group were comparable. As can be seen, the antibody-drug conjugate Hu033-03-ADC of the present disclosure has good safety, and can significantly reduce the BDCA2 level on the surface of pDCs.

Evaluation criteria in FIGs. 12-13: the average expression level changes of IFNα/TNFα were evaluated after *in vitro* stimulation of the whole blood with CpGA at 200 µg/mL on days 0-33 after administration. In FIG. 12b, the average level of IFNα in the plasma after *in vitro* whole blood stimulation in the animals before administration was 34.2 pg/mL, and the average expression level of IFNα was 7.6 pg/mL on days 0-33 after administration; in FIG. 12c, the average expression level of IFNα in the plasma after *in vitro* whole blood stimulation in the animals before administration was 34.4 pg/mL, and the average expression level of IFNα was 12.6 pg/mL on days 0-33 after administration. In FIG. 13b, the average expression level of TNFα in the plasma after *in vitro* whole blood stimulation in the animals before administration was 5515 pg/mL, and the average expression level of TNFα was 4131 pg/mL on days 0-33 after administration; in FIG. 13c, the average expression level of TNFα in the plasma after *in vitro* whole blood stimulation in the animals before administration was 3977 pg/mL, and the average expression level of TNFα was 3706 pg/mL on days 0-33 after administration.

According to FIGs. 12-13, the *in vitro* CpGA-stimulated whole blood experiments showed that IFN-α expression levels were reduced in both the antibody-drug conjugate and BIIB059 groups compared to the blank control group, and the average expression level of IFN-α in the antibody-drug conjugate group (7.6 pg/mL) was lower than that in the BIIB059 group (12.6 pg/mL). Compared to the blank control group, the TNFα expression level in the BIIB059 group had no significant change, while the TNFα expression level in the antibody-drug conjugate group was significantly reduced. The antibody-drug conjugate Hu033-03-ADC of the present disclosure exhibited a better cytokine inhibitory effect than that of BIIB059.

Various modifications and changes in the methods and systems described herein will be apparent to those skilled in the art without departing from the scope and spirit of the present disclosure. Although the present disclosure has been described in connection with specific preferred embodiments, it should be understood that the present disclosure as claimed shall not be improperly limited to such specific embodiments. Indeed, various modifications of the modes for implementing the present disclosure, which are apparent to those skilled in molecular biology, immunology, or related fields, are intended to be within the scope of the appended claims.

Sequence list (all sequences are amino acid sequences; for the definition/description of the sequences, refer to the previous sections herein):

| Sequence No. | Sequence |
|---|---|
| SEQ ID NO:1 | SYGMS |
| SEQ ID NO:2 | TISSGDSYTYYPDSVKG |
| SEQ ID NO:3 | QIYYDYAYYFDF |
| SEQ ID NO:4 | RASESVSFRTSHLMH |
| SEQ ID NO:5 | GASNLES |
| SEQ ID NO:6 | QQSIEDPPT |
| SEQ ID NO:7 | NYGVH |
| SEQ ID NO:8 | VIWSGESTDYDAAFIS |
| SEQ ID NO:9 | RRSHYYGYVMDY |
| SEQ ID NO:10 | KASQSIDYDAIGYLN |
| SEQ ID NO:11 | AASNLES |
| SEQ ID NO:12 | QQSNEDPPT |
| SEQ ID NO:13 | EVQLVESGGGLVKPGGSLRLSCAASGFTFSSYGMSWVRQAPG KGLEWVSTISSGDSYTYYPDSVKGRFTISRDNAKNSLYLQMNS LRAEDTAVYYCARQIYYDYAYYFDFWGQGTTVTVSS |
| SEQ ID NO:14 | DIVLTQSPASLAVSPGQRATITCRASESVSFRTSHLMHWYQQKP GQPPKLLIYGASNLESGVPARFSGSGSETDFTLTINPVEAEDTAN YFCQQSIEDPPTFGGGTKVEIK |
| SEQ ID NO:15 | EVQLVESGGGLVKPGGSLRLSCAASGFTFRSYGMSWVRQAPG KRLEWVSTISSGDSYTYYPDSVKGRFTISRDNAKNSLYLQMNS LKAEDTAVYYCARQIYYDYAYYFDFWGQGTTVTVSS |
| SEQ ID NO:16 | DIVMTQSPDSLAVSLGERATINCRASESVSFRTSHLMHWYQQK PGQPPKLLIYGASNLESGVPDRFSGSGSGTDFTLTISSLQAEDVA VYYCQQSIEDPPTFGGGTKVEIK |
| SEQ ID NO:17 | QVQLQQSGPGLVKPSETLSLTCTVSGGSLTNYGVHWIRQPPGK GLEWIGVIWSGESTDYDAAFISRVTISKDNSKNQVSLKLSSVTA ADTAVYYCARRRSHYYGYVMDYWGQGTTVTVSS |
| SEQ ID NO:18 | DIVLTQSPASLAVSPGQRATITCKASQSIDYdaIGYLNWYQQKPG QPPKLLIYAASNLESGVPARFSGSGSGTDFTLTINPVEANDTAN YYCQQSNEDPPTFGQGTKLEIK |
| SEQ ID NO:19 | EVQLVESGGGLVKPGGSLRLSCAASGFTFSSYGMSWVRQAPG KGLEWVSTISSGDSYTYYPDSVKGRFTISRDNAKNSLYLQMNS LRAEDTAVYYCARQIYYDYAYYFDFWGQGTTVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNTK VDKKVEPKSCDKTHTCPPCPAPELLGGPSVFLFPPKPKDTLMIS RTPEVTCVVVDVSHEDPEVKFNWYVDGVEVHNAKTKPREEQY NSTYRVVSVLTVLHQDWLNGKEYKCKVSNKALPAPIEKTISKA KGQPREPQVYTLPPSRDELTKNQVSLTCLVKGFYPSDIAVEWES NGQPENNYKTTPPVLDSDGSFFLYSKLTVDKSRWQQGNVFSCS VMHEALHNHYTQKSLSLSPGK |
| SEQ ID NO:20 | DIVLTQSPASLAVSPGQRATITCRASESVSFRTSHLMHWYQQKP GQPPKLLIYGASNLESGVPARFSGSGSETDFTLTINPVEAEDTAN YFCQQSIEDPPTFGGGTKVEIKRTVAAPSVFIFPPSDEQLKSGTA SVVCLLNNFYPREAKVQWKVDNALQSGNSQESVTEQDSKDST YSLSSTLTLSKADYEKHKVYACEVTHQGLSSPVTKSFNRGEC |
| SEQ ID NO:21 | EVQLVESGGGLVKPGGSLRLSCAASGFTFRSYGMSWVRQAPG |
| | KRLEWVSTISSGDSYTYYPDSVKGRFTISRDNAKNSLYLQMNS LKAEDTAVYYCARQIYYDYAYYFDFWGQGTTVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNTK VDKKVEPKSCDKTHTCPPCPAPELLGGPSVFLFPPKPKDTLMIS RTPEVTCVVVDVSHEDPEVKFNWYVDGVEVHNAKTKPREEQY NSTYRVVSVLTVLHQDWLNGKEYKCKVSNKALPAPIEKTISKA KGQPREPQVYTLPPSRDELTKNQVSLTCLVKGFYPSDIAVEWES NGQPENNYKTTPPVLDSDGSFFLYSKLTVDKSRWQQGNVFSCS VMHEALHNHYTQKSLSLSPGK |
| SEQ ID NO:22 | DIVMTQSPDSLAVSLGERATINCRASESVSFRTSHLMHWYQQK PGQPPKLLIYGASNLESGVPDRFSGSGSGTDFTLTISSLQAEDVA VYYCQQSIEDPPTFGGGTKVEIKRTVAAPSVFIFPPSDEQLKSGT ASVVCLLNNFYPREAKVQWKVDNALQSGNSQESVTEQDSKDS TYSLSSTLTLSKADYEKHKVYACEVTHQGLSSPVTKSFNRGEC |
| SEQ ID NO:23 | QVQLQQSGPGLVKPSETLSLTCTVSGGSLTNYGVHWIRQPPGK GLEWIGVIWSGESTDYDAAFISRVTISKDNSKNQVSLKLSSVTA ADTAVYYCARRRSHYYGYVMDYWGQGTTVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRTP EVTCVVVDVSHEDPEVKFNWYVDGVEVHNAKTKPREEQYNS TYRVVSVLTVLHQDWLNGKEYKCKVSNKALPAPIEKTISKAKG QPREPQVYTLPPSRDELTKNQVSLTCLVKGFYPSDIAVEWESNG QPENNYKTTPPVLDSDGSFFLYSKLTVDKSRWQQGNVFSCSVM HEALHNHYTQKSLSLSPGK |
| SEQ ID NO:24 | DIVLTQSPASLAVSPGQRATITCKASQSIDYdaIGYLNWYQQKPG QPPKLLIYAASNLESGVPARFSGSGSGTDFTLTINPVEANDTAN YYCQQSNEDPPTFGQGTKLEIKRTVAAPSVFIFPPSDEQLKSGT ASVVCLLNNFYPREAKVQWKVDNALQSGNSQESVTEQDSKDS TYSLSSTLTLSKADYEKHKVYACEVTHQGLSSPVTKSFNRGEC |
| SEQ ID NO:25 | DVQLVESGGGLVKPGGSLRLSCAASGFTFSTYTMSWVRQAPG KGLEWVATISPGDSFGYYYPDSVQGRFTISRDNAKNSLYLQMN SLRAEDTAVYYCTRDIYYNYGAWFAYWGQGTLVTVSSASTKG PSVFPLAPSSKSTSGGTAALGCLVKDYFPEPVTVSWNSGALTSG VHTFPAVLQSSGLYSLSSVVTVPSSSLGTQTYICNVNHKPSNTK VDKKVEPKSCDKTHTCPPCPAPELLGGPSVFLFPPKPKDTLMIS RTPEVTCVVVDVSHEDPEVKFNWYVDGVEVHNAKTKPREEQY NSTYRVVSVLTVLHQDWLNGKEYKCKVSNKALPAPIEKTISKA KGQPREPQVYTLPPSRDELTKNQVSLTCLVKGFYPSDIAVEWES NGQPENNYKTTPPVLDSDGSFFLYSKLTVDKSRWQQGNVFSCS VMHEALHNHYTQKSLSLSPG |
| SEQ ID NO:26 | DIQLTQSPSSLSASVGDRVTITCKASQSVDYDGDSYMNWYQQK PGKAPKLLIYAASTLESGVPSRFSGSGSGTDFTLTISSLQPEDFAT |
| | YYCQQANEDPRTFGQGTKVEIKRTVAAPSVFIFPPSDEQLKSGT ASVVCLLNNFYPREAKVQWKVDNALQSGNSQESVTEQDSKDS TYSLSSTLTLSKADYEKHKVYACEVTHQGLSSPVTKSFNRGEC |

## Claims

1. An antibody-drug conjugate, comprising an antibody targeting BDCA2 or an antigen-binding fragment thereof, a linker fragment (L), and a glucocorticoid molecule, wherein the antibody targeting BDCA2 or the antigen-binding fragment thereof comprises:
(1) HCDR1 with the amino acid sequence set forth in SEQ ID NO: 1, HCDR2 with the amino acid sequence set forth in SEQ ID NO: 2, HCDR3 with the amino acid sequence set forth in SEQ ID NO: 3, LCDR1 with the amino acid sequence set forth in SEQ ID NO: 4, LCDR2 with the amino acid sequence set forth in SEQ ID NO: 5, and LCDR3 with the amino acid sequence set forth in SEQ ID NO: 6; or
(2) HCDR1 with the amino acid sequence set forth in SEQ ID NO: 7, HCDR2 with the amino acid sequence set forth in SEQ ID NO: 8, HCDR3 with the amino acid sequence set forth in SEQ ID NO: 9, LCDR1 with the amino acid sequence set forth in SEQ ID NO: 10, LCDR2 with the amino acid sequence set forth in SEQ ID NO: 11, and LCDR3 with the amino acid sequence set forth in SEQ ID NO: 12.

2. The antibody-drug conjugate according to claim 1, wherein the antibody or the antigen-binding fragment thereof comprises:
(1) a heavy chain variable region with the amino acid sequence set forth in SEQ ID NO: 13, and a light chain variable region with the amino acid sequence set forth in SEQ ID NO: 14; or
(2) a heavy chain variable region with the amino acid sequence set forth in SEQ ID NO: 15, and a light chain variable region with the amino acid sequence set forth in SEQ ID NO: 16; or
(3) a heavy chain variable region with the amino acid sequence set forth in SEQ ID NO: 17, and a light chain variable region with the amino acid sequence set forth in SEQ ID NO: 18.

3. The antibody-drug conjugate according to claim 1, wherein the antibody is selected from a human antibody, a humanized antibody,
a chimeric antibody, a multispecific antibody, a monoclonal antibody, and a polyclonal antibody.

4. The antibody-drug conjugate according to claim 1, wherein the antigen-binding fragment is selected from a Fab, a Fab',
a F(ab')₂, an Fv, an scFv, a Fab'-SH, an sdAb, a VHH, a bispecific antibody, and a linear antibody.

5. The antibody-drug conjugate according to claim 1, wherein the antibody or the antigen-binding fragment thereof comprises an immunoglobulin constant region, the immunoglobulin constant region being a human IgG constant region, e.g., a human IgG1 constant region.

6. The antibody-drug conjugate according to claim 1, wherein the antibody or the antigen-binding fragment thereof comprises or consists of the following sequences:
(1) a heavy chain with the amino acid sequence set forth in SEQ ID NO: 19 or having at least 95%, 96%, 97%, 98%, or 99% sequence identity thereto, and a light chain with the amino acid sequence set forth in SEQ ID NO: 20 or having at least 95%, 96%, 97%, 98%, or 99% sequence identity thereto; or
(2) a heavy chain with the amino acid sequence set forth in SEQ ID NO: 21 or having at least 95%, 96%, 97%, 98%, or 99% sequence identity thereto, and a light chain with the amino acid sequence set forth in SEQ ID NO: 22 or having at least 95%, 96%, 97%, 98%, or 99% sequence identity thereto; or
(3) a heavy chain with the amino acid sequence set forth in SEQ ID NO: 23 or having at least 95%, 96%, 97%, 98%, or 99% sequence identity thereto, and a light chain with the amino acid sequence set forth in SEQ ID NO: 24 or having at least 95%, 96%, 97%, 98%, or 99% sequence identity thereto.

7. The antibody-drug conjugate according to any one of claims 1-6, wherein the glucocorticoid molecule comprises a structure represented by formula (I), and a tautomer, a mesomer, a racemate, an enantiomer, a diastereoisomer or a mixture thereof, or a pharmaceutically acceptable salt or a solvate thereof, 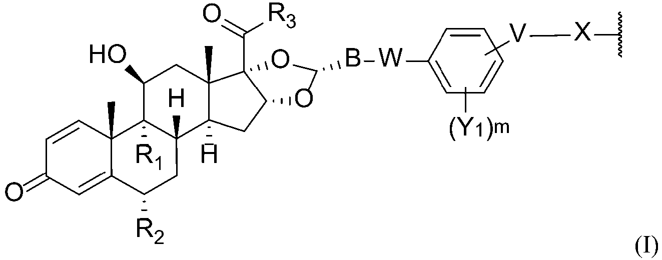 wherein
X is selected from: -O-, -S-, and -N(R^{1a})-, wherein R^{1a} is selected from H, C₁-C₆ alkyl, and -C₁-C₆ alkylhydroxy;
R₁ and R₂ are each independently selected from H, protium, deuterium, tritium, halogen, C₁-C₆ alkyl, and halogenated C₁-C₆ alkyl;
R₃ is selected from C₁-C₆ alkyl, halogenated C₁-C₆ alkyl, C₁-C₆ alkoxy, halogenated C₁-C₆ alkoxy, C₁-C₆ alkylthio, halogenated C₁-C₆ alkylthio, -C₁-C₆ alkyl-O-P(=O)(OC₁-C₆ alkyl)₂, and -C₁-C₆ alkyl-O-P(=O)(OH)₂, wherein the C₁-C₆ alkyl, C₁-C₆ alkoxy, and C₁-C₆ alkylthio are each independently optionally substituted with one or more substituents selected from halogen, CN, OH, or SH;
ring B is selected from phenyl or 5- to 6-membered heteroaryl, and ring B is optionally substituted with one or more substituents selected from halogen, OH, CN, NH₂, C₁-C₆ alkyl, C₁-C₆ alkoxy, and halogenated C₁-C₆ alkyl;
W is selected from a single bond, -O-, -S-, -C(O)-, -NH-, -C(O)NH-, -NHC(O)-, -C(O)-N(C₁-C₆ alkyl)-, -N(C₁-C₆ alkyl)-C(O)-, -C₁-C₆ alkylene-, -N(C₁-C₆ alkyl)-, -C₁₋₆ alkylene-NH-, -O-C₁-C₆ alkylene-, and -C₁-C₆ alkylene-O-; the C₁-C₆ alkyl and C₁-C₆ alkylene are optionally substituted with one or more substituents selected from halogen, C₁-C₆ alkyl, C₁-C₆ alkoxy, halogenated C₁-C₆ alkyl, C₃-C₆ cycloalkyl, oxo, OH, CN, or NH₂;
V is selected from a single bond or -(C(R^{2a})(R^{2b}))ₙ-, wherein R^{2a} and R^{2b} are each independently selected from H, halogen, OH, NH₂, -CN, C₁-C₆ alkyl, and halogenated C₁-C₆ alkyl, or R^{2a} and R^{2b}, together with the carbon atom to which they are linked, form carbonyl or C₃₋₆ cycloalkyl, and n is selected from 1, 2, 3, 4, 5, or 6;
Y₁ is selected from H, halogen, OH, nitro, NH₂, -NH(C₁-C₆ alkyl), -N(C₁-C₆ alkyl)₂, C₁-C₆ alkyl, -CN, C₁-C₆ alkoxy, -C₁-C₆ alkylhydroxy, and halogenated C₁-C₆ alkyl;
m is selected from 1, 2, or 3.

8. The antibody-drug conjugate according to claim 7, wherein X is selected from -O-, -S-, and -N(R^{1a})-, wherein R^{1a} is selected from H, -CH₃, and -CH₂CH₃.

9. The antibody-drug conjugate according to claim 8, wherein X is selected from -O- and - NH-.

10. The antibody-drug conjugate according to claim 7, wherein R₁ and R₂ are each independently selected from H, F, Cl, and -CH₃.

11. The antibody-drug conjugate according to claim 10, wherein R₁ and R₂ are each independently selected from H or F.

12. The antibody-drug conjugate according to claim 7, wherein R₃ is selected from -CH₂Cl, - CH₂SH, -CH₂OH, 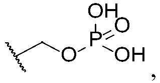 -OCH₃, -OCH₂F, -OCH₂Cl, -OCH₂CN, -OCH₂CH₃, -SCH₂F, -SCH₂Cl, -SCH₂CF₃ and -SCH₂CN.

13. The antibody-drug conjugate according to claim 12, wherein R₃ is selected from -CH₂OH, -SCH₂F, and

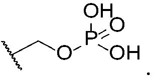

14. The antibody-drug conjugate according to claim 7, wherein ring B is selected from phenyl, pyridinyl, thienyl, pyrrolyl, furanyl, pyrazolyl, imidazolyl, and thiazolyl, and ring B is optionally substituted with one or more substituents selected from halogen, OH, CN, NH₂, C₁-C₆ alkyl, C₁-C₆ alkoxy, and halogenated C₁-C₆ alkyl.

15. The antibody-drug conjugate according to claim 14, wherein ring B is selected from phenyl and thienyl, and ring B is optionally substituted with one or more substituents selected from halogen, OH, CN, NH₂, -CH₃, -CH₂CH₃, or -OCH₃.

16. The antibody-drug conjugate according to claim 15, wherein ring B is selected from

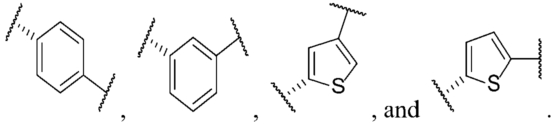

17. The antibody-drug conjugate according to claim 7, wherein W is selected from -C₁-C₆ alkylene-, wherein the C₁-C₆ alkylene is optionally substituted with one or more substituents selected from halogen, C₁-C₃ alkyl, C₁-C₃ alkoxy, halogenated C₁-C₃ alkyl, C₃-C₆ cycloalkyl, oxo, OH, CN, or NH₂.

18. The antibody-drug conjugate according to claim 17, wherein W is selected from

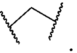

19. The antibody-drug conjugate according to claim 7, wherein V is selected from a single bond or -(C(R^{2a})(R^{2b}))ₙ-, wherein R^{2a} and R^{2b} are each independently selected from H, halogen, OH, NH₂, -CN, C₁-C₃ alkyl, and halogenated C₁-C₃ alkyl, and n is selected from 1, 2, or 3.

20. The antibody-drug conjugate according to claim 19, wherein V is selected from a single bond, -CH₂-, or -CH₂CH₂-.

21. The antibody-drug conjugate according to claim 7, wherein Y₁ is selected from H, halogen, OH, NH₂, C₁-C₃ alkyl, -CN, C₁-C₃ alkoxy, -C₁-C₃ alkylhydroxy, and halogenated C₁-C₃ alkyl.

22. The antibody-drug conjugate according to claim 21, wherein Y₁ is selected from H, halogen, OH, NH₂, -CH₃, -CH₂CH₃, -CN, -OCH₃, and -CH₂OH.

23. The antibody-drug conjugate according to any one of claims 7-22, wherein the structure of the glucocorticoid molecule is selected from the group consisting of:

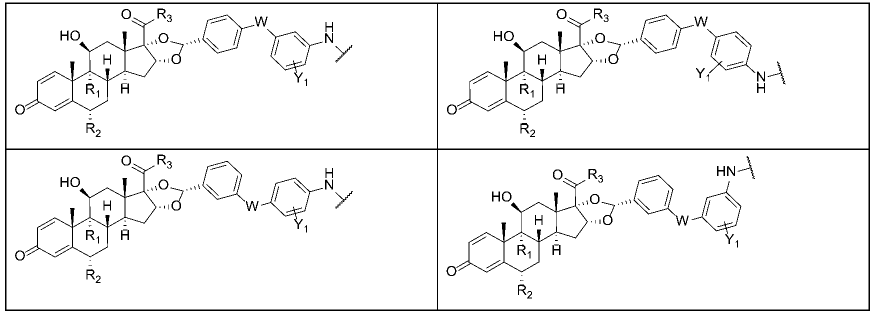

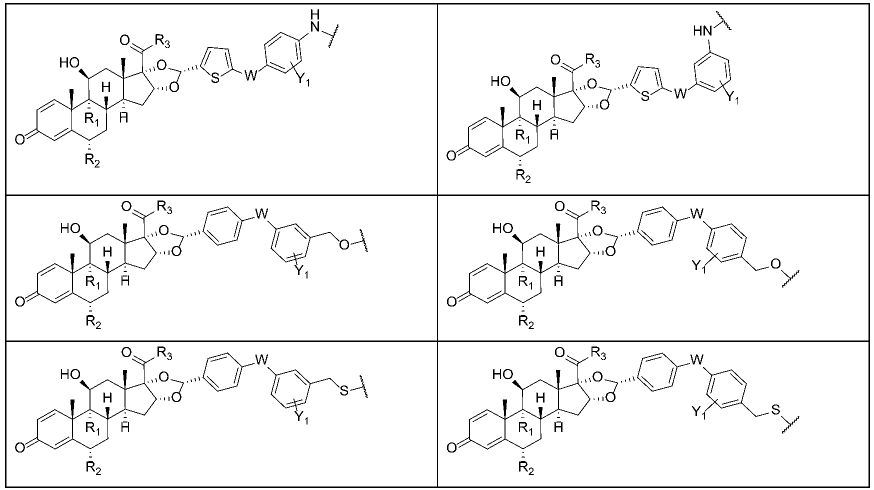

24. wherein
R₁ and R₂ are as defined in claim 7, 10, or 11;
R₃ is as defined in claim 7, 12, or 13;
W is as defined in claim 7, 17, or 18;
Y₁ is as defined in claim 7, 21, or 22.

25. The antibody-drug conjugate according to claim 23, wherein the structure of the glucocorticoid molecule is selected from the group consisting of:

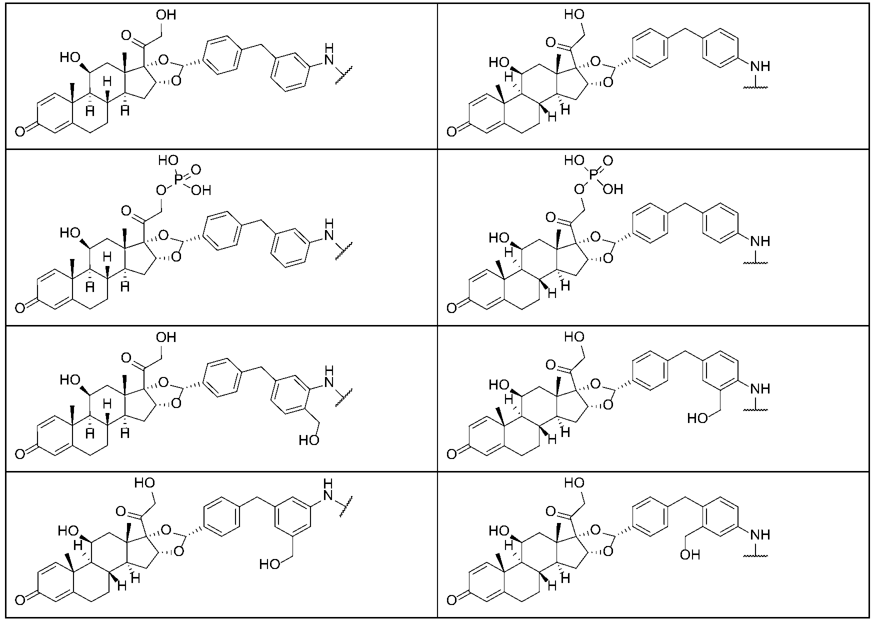

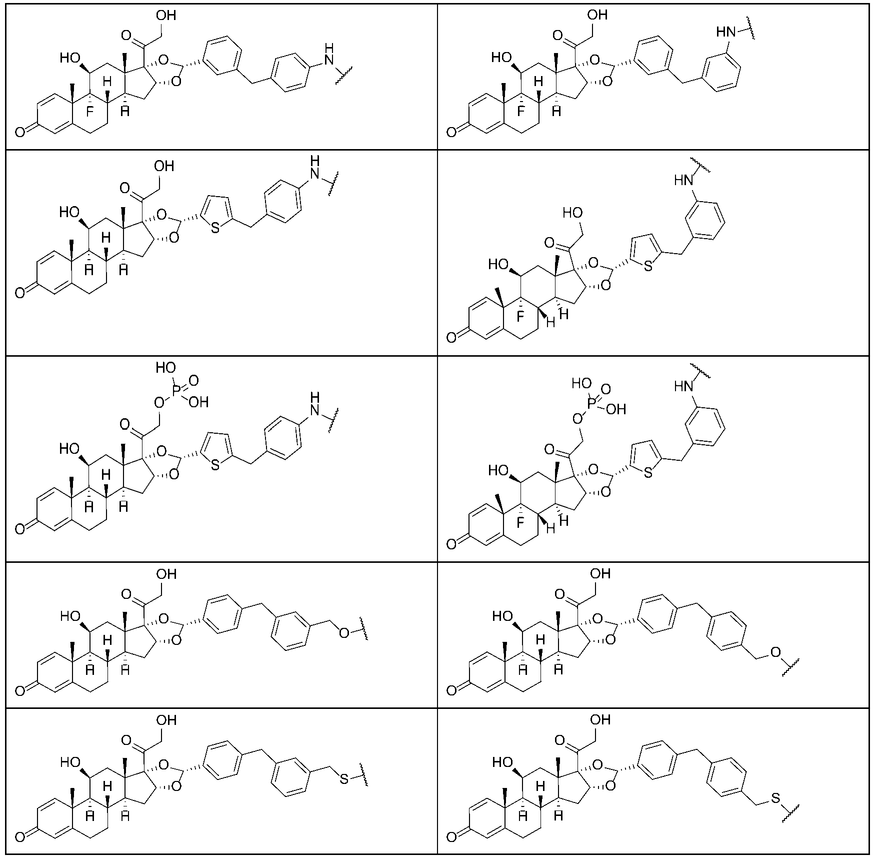

26. The antibody-drug conjugate according to any one of claims 1-24, wherein the linker fragment (L) comprises Tr, L₁, L₂, and L₃, and the antibody-drug conjugate comprises a structure represented by formula (II): 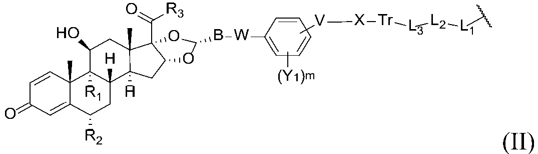
Tr is absent or Tr is any group;
L₃ is selected from a polypeptide fragment;
L₂ is absent or selected from a linker fragment;
L₁ is selected from a coupling unit.

27. The antibody-drug conjugate according to claim 25, wherein
Tr is absent or selected from: 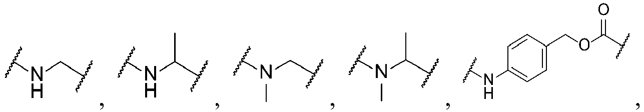 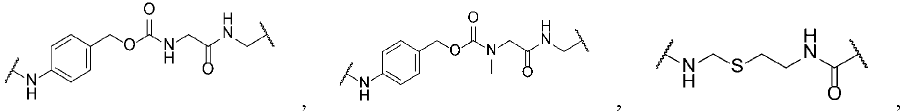 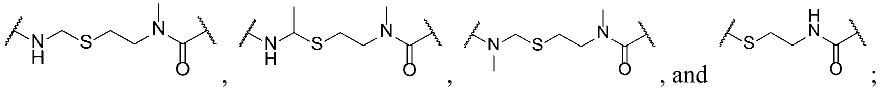
L₃ is selected from: glycine-glycine-phenylalanine-glycine (GGFG), alanine-alanine-alanine-glycine (AAAG), glycine-glycine-glycine-glycine (GGGG), valine-alanine-glycine (VAG), valine-citrulline-glycine (VCG), alanine-alanine-glycine (AAG), alanine-alanine-alanine (AAA), valine-alanine (VA), valine-citrulline (VC), alanine-alanine (AA), glutamic acid-alanine-glycine-glycine (EAGG), glycine-glutamic acid-alanine-glycine (GEAG), glycine-glutamic acid-glycine-glycine (GEGG), glutamic acid-glycine-glycine (EGG), glutamic acid-alanine-glycine (EAG), valine-lysine-glycine (VKG), glycine-glutamic acid-glycine (GEG), glutamic acid-alanine (EA), glutamic acid-glycine (EG), and glycine-glutamic acid (GE);
L₂ is absent or selected from: 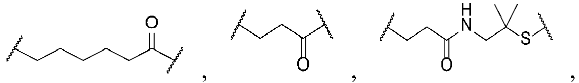 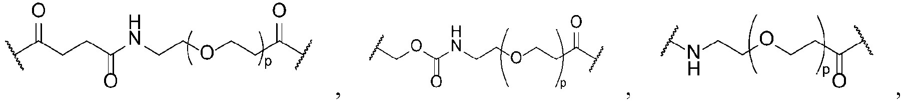 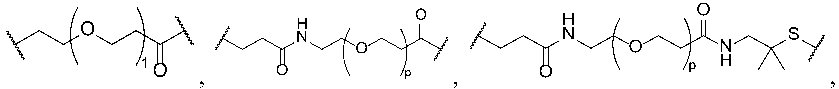 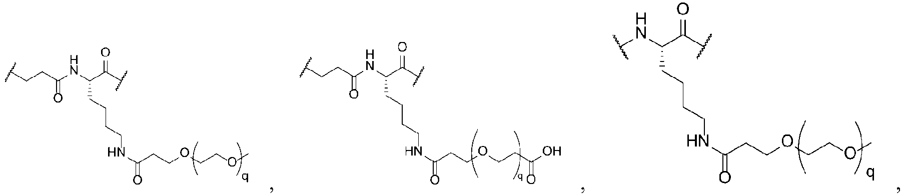 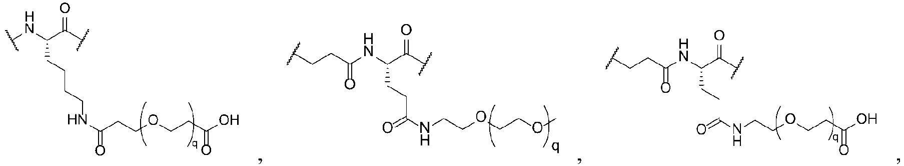 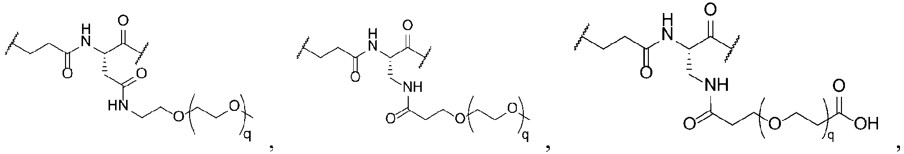 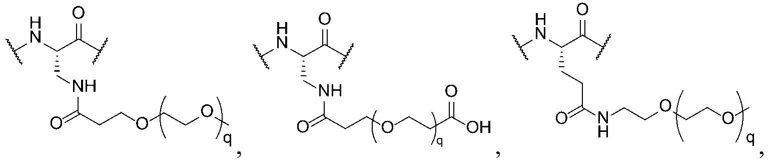 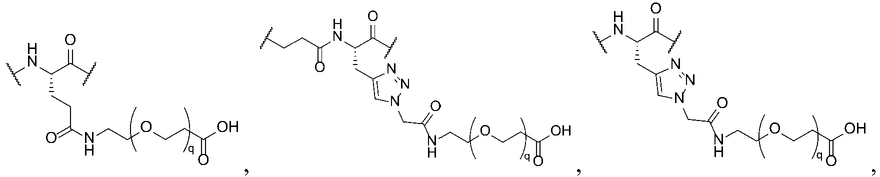 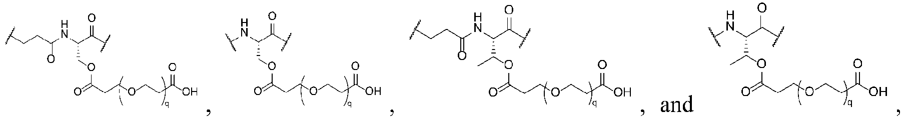
wherein q is selected from any integer from 1 to 30, and p is any integer from 1 to 20;
L₁ is selected from:
(1) when L₁ is coupled to Ab via sulfhydryl, L₁ is selected from the following structures: 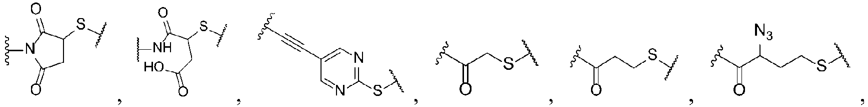 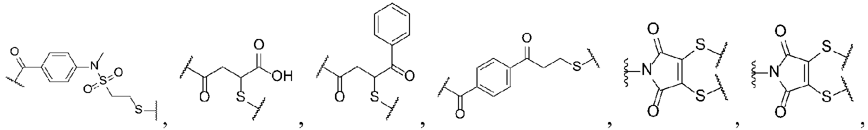 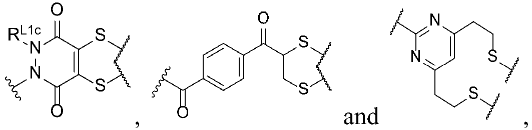 wherein R^{L1c} is selected from: hydrogen, optionally substituted alkyl, and optionally substituted aryl;
(2) when L₁ is coupled to Ab via amino, L₁ is selected from the following structures: 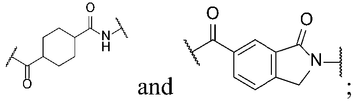
(3) when L₁ is coupled to Ab via click chemistry, L₁ is selected from the following structures:

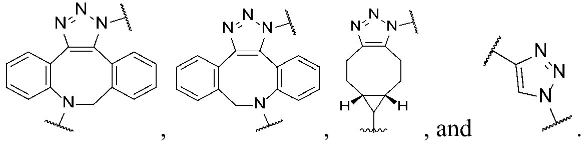

28. The antibody-drug conjugate according to claim 26, wherein the structural unit -Tr-L₃-L₂-L₁- is selected from: 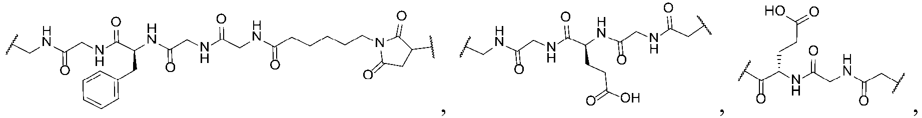 and

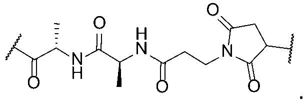

29. The antibody-drug conjugate according to any one of claims 1-27, wherein the antibody-drug conjugate is of the structure represented by formula (III-A): 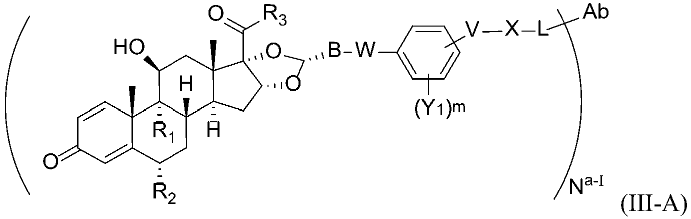 wherein
Ab is as defined in any one of claims 1-6, and N^{a-I} is any number from 1 to 10;
L is as defined in any one of claims 25, 26, or 27;
X is selected from: -O-, -S-, and -N(R^{1a})-, wherein R^{1a} is selected from H, C₁-C₆ alkyl, and C₁-C₆ alkylhydroxy;
R₁ and R₂ are each independently selected from H, protium, deuterium, tritium, halogen, C₁-C₆ alkyl, and halogenated C₁-C₆ alkyl;
R₃ is selected from halogenated C₁-C₆ alkyl, C₁-C₆ alkoxy, C₁-C₆ alkylthio, and -C₁-C₆ alkyl-OP(=O)(OH)₂, wherein the C₁-C₆ alkyl, C₁-C₆ alkoxy, and C₁-C₆ alkylthio are each independently optionally substituted with one or more substituents selected from halogen, CN, OH, or SH;
ring B is selected from phenyl or 5- to 6-membered heteroaryl, and ring B is optionally substituted with one or more substituents selected from halogen, OH, CN, NH₂, C₁-C₆ alkyl, C₁-C₆ alkoxy, and halogenated C₁-C₆ alkyl;
W is selected from a single bond, -O-, -S-, -C(O)-, -NH-, -C(O)NH-, -NHC(O)-, -C(O)-N(C₁-C₆ alkyl)-, -N(C₁-C₆ alkyl)-C(O)-, -C₁-C₆ alkylene-, -N(C₁-C₆ alkyl)-, -C₁₋₆ alkylene-NH-, -O-C₁-C₆ alkylene-, and -C₁-C₆ alkylene-O-; the C₁-C₆ alkyl and C₁-C₆ alkylene are optionally substituted with one or more substituents selected from halogen, C₁-C₆ alkyl, C₁-C₆ alkoxy, halogenated C₁-C₆ alkyl, C₃-C₆ cycloalkyl, oxo, OH, CN, or NH₂;
V is selected from a single bond or -(C(R^{2a})(R^{2b}))ₙ-, wherein R^{2a} and R^{2b} are each independently selected from H, halogen, OH, NH₂, -CN, C₁-C₆ alkyl, and halogenated C₁-C₆ alkyl, or R^{2a} and R^{2b}, together with the carbon atom to which they are linked, form carbonyl or C₃₋₆ cycloalkyl, and n is selected from 1, 2, 3, 4, 5, or 6;
Y₁ is selected from H, halogen, OH, NH₂, C₁-C₆ alkyl, -CN, C₁-C₆ alkoxy, -C₁-C₆ alkylhydroxy, and halogenated C₁-C₆ alkyl;
m is selected from 1, 2, or 3.

30. The antibody-drug conjugate according to claim 28, wherein the antibody-drug conjugate is of the structures represented by formulas (III-A1) to (III-A4): 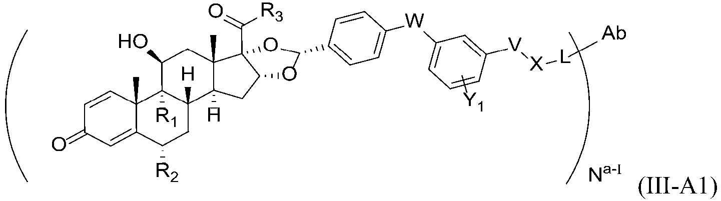 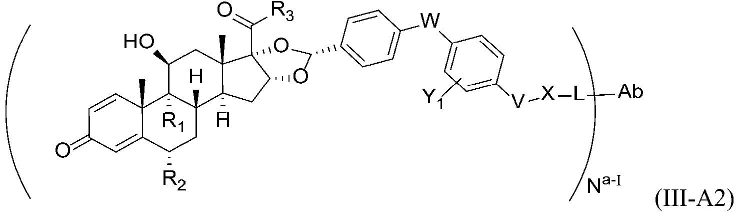 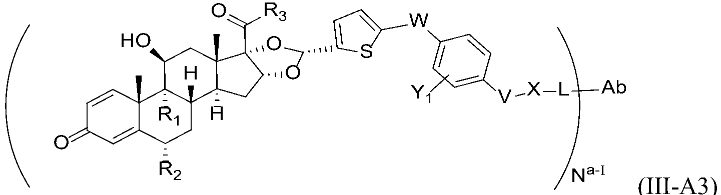 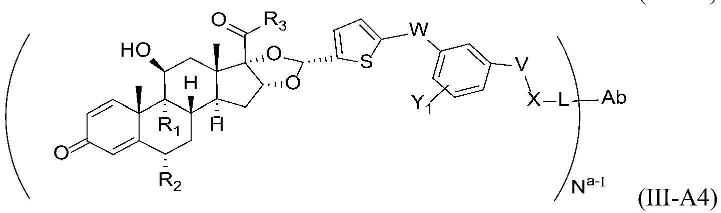 wherein
L is selected from

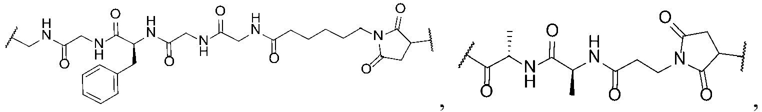

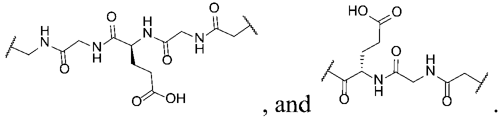

31. The antibody-drug conjugate according to any one of claims 1-29, having a structure selected from the following: 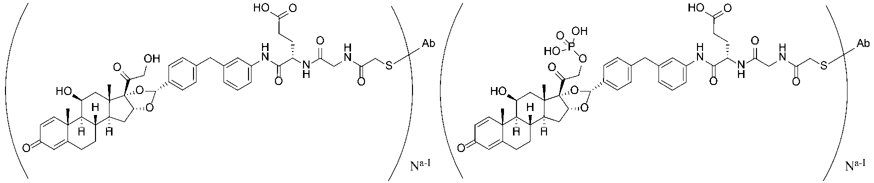 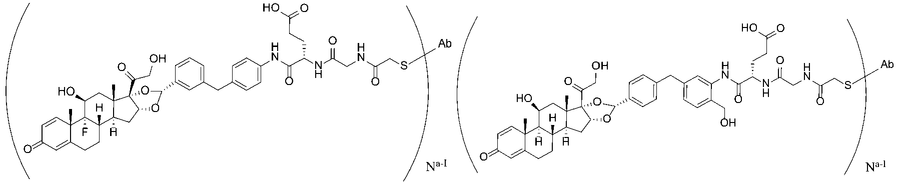 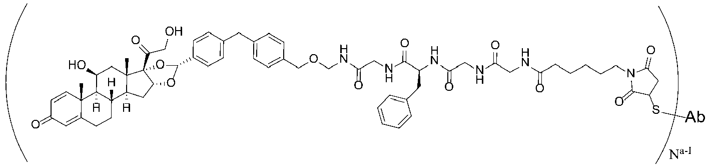 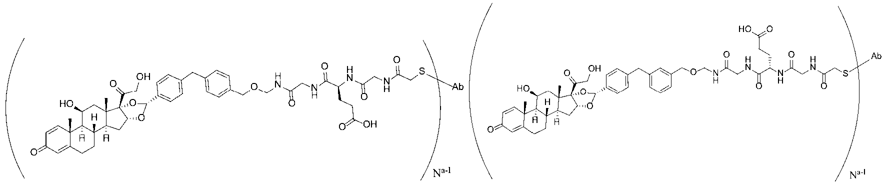 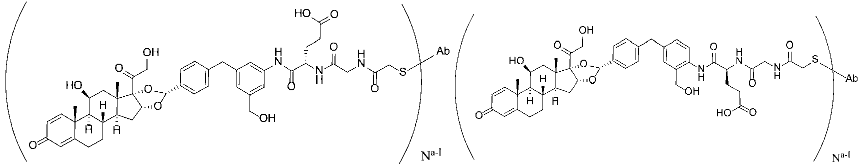 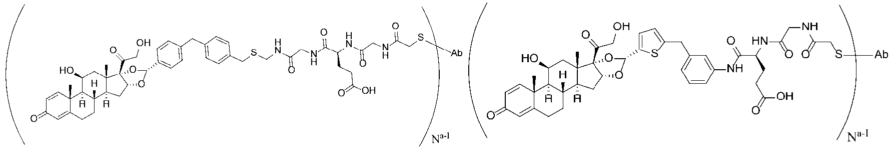 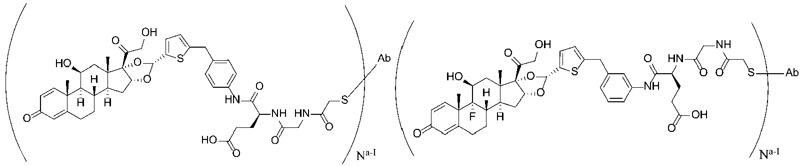 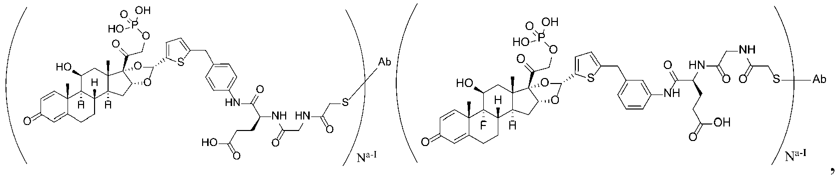 wherein each Ab is as defined in any one of claims 1-6, and N^{a-I} is any number from 1 to 10.

32. The antibody-drug conjugate according to claim 30, having a structure selected from the following: 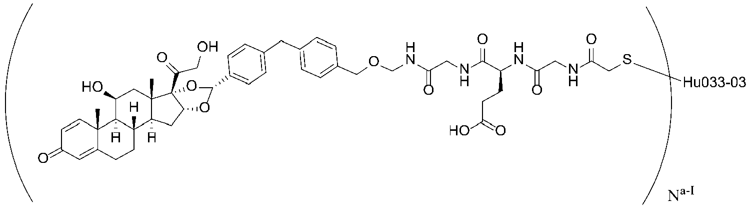 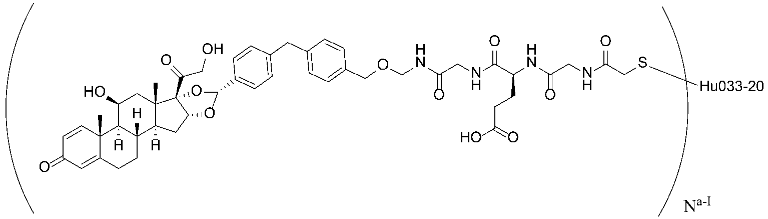 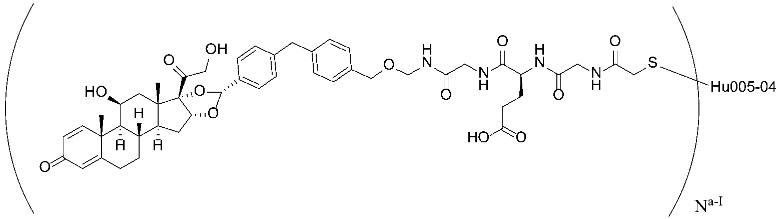 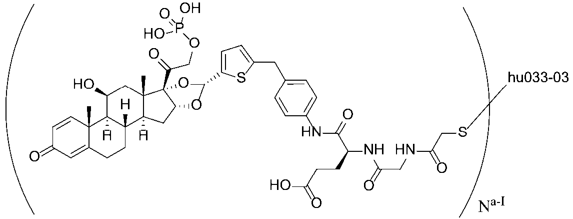 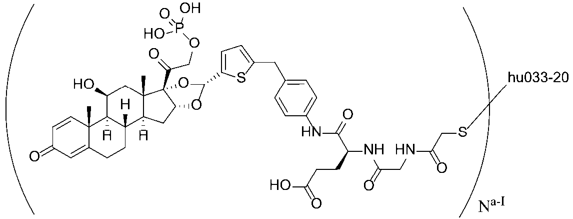 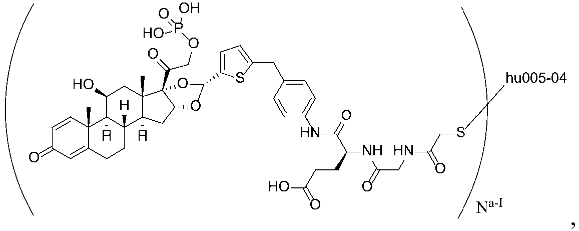 wherein N^{a-I} is any number from 1 to 10; preferably, N^{a-I} is an integer or a decimal from 2 to 8; preferably, N^{a-I} is an integer or a decimal from 3 to 8; preferably, N^{a-I} is an integer or a decimal from 3 to 4 or from 4 to 5; Hu033-03, Hu033-20, and Hu005-04 are anti-BDCA2 antibodies.

33. A pharmaceutical composition, comprising the antibody-drug conjugate according to any one of claims 1-31 and a pharmaceutically acceptable carrier or excipient.

34. Use of the antibody-drug conjugate according to any one of claims 1-31 or the pharmaceutical composition according to claim 32 in the preparation of a medicament for treating and/or preventing a disease or disorder mediated by BDCA2 and a plasmacytoid dendritic cell.

35. A method for treating and/or preventing a disease or disorder mediated by BDCA2 and a plasmacytoid dendritic cell, comprising administering to a subject in need thereof the antibody-drug conjugate according to any one of claims 1-31 or the pharmaceutical composition according to claim 32.

36. The use according to claim 33 or the method according to claim 34, wherein the disease and/or disorder is selected from systemic lupus erythematosus, discoid lupus, lupus nephritis, epidermal lupus erythematosus, rheumatoid arthritis, inflammatory bowel disease, systemic sclerosis (scleroderma), psoriasis, type I diabetes, dermatomyositis, and polymyositis.
